# EUROPEAN PATENT SPECIFICATION

(11) **EP 2 209 789 B1**
(45) Date of publication and mention of the grant of the patent: **16.11.2011**
(21) Application number: 07874300.2
(22) Date of filing: 20.11.2007
(51) Int. Cl.: C07D 487/08, A61K 31/55, A61P 31/12

(54) **CYCLOPROPYL FUSED INDOLOBENZAZEPINE HCV NS5B INHIBITORS**
CYCLOPROPYLKONDENSIERTE INDOLOBENZAZEPINE ALS HCV-NS5B-INHIBITOREN
INHIBITEURS DE LA POLYMÉRASE NS5B DU VHC À BASE D'INDOLOBENZAZÉPINE FUSIONNÉE AU CYCLOPROPYLE

(43) Date of publication of application: 28.07.2010
(73) Proprietor: Bristol-Myers Squibb Company, Princeton, NJ 05843-4000 (US)
(72) Inventor: BENDER, John A., Wallingford, Connecticut 06492 (US); DING, Min, Wallingford, Connecticut 06492 (US); GENTLES, Robert G., Wallingford, Connecticut 06492 (US); HEWAWASAM, Piyasena, Wallingford, Connecticut 06492 (US)
(74) Representative: Reitstötter - Kinzebach
(86) International application number: PCT/US2007/085183
(87) International publication number: WO 2009/067108

(56) References cited:
- WO-A-2007/136982
- WO-A-2008/112473
- US-A1- 2007 060 565
- US-A1- 2008 146 537

## Description

### BACKGROUND OF THE INVENTION

Hepatitis C virus (HCV) is a major human pathogen, infecting an estimated 170 million persons worldwide - roughly five times the number infected by human immunodeficiency virus type 1. A substantial fraction of these HCV infected individuals develop serious progressive liver disease, including cirrhosis and hepatocellular carcinoma (Lauer, G. M.; Walker, B. D. N. Engl. J Med. 2001, 345, 41-52).

HCV is a positive-stranded RNA virus. Based on a comparison of the deduced amino acid sequence and the extensive similarity in the 5'-untranslated region, HCV has been classified as a separate genus in the Flaviviridae family. All members of the Flaviviridae family have enveloped virions that contain a positive stranded RNA genome encoding all known virus-specific proteins via translation of a single, uninterrupted, open reading frame.

Considerable heterogeneity is found within the nucleotide and encoded amino acid sequence throughout the HCV genome. At least six major genotypes have been characterized, and more than 50 subtypes have been described. The major genotypes of HCV differ in their distribution worldwide, and the clinical significance of the genetic heterogeneity of HCV remains elusive despite numerous studies of the possible effect of genotypes on pathogenesis and therapy.

The single strand HCV RNA genome is approximately 9500 nucleotides in length and has a single open reading frame (ORF) encoding a single large polyprotein of about 3000 amino acids. In infected cells, this polyprotein is cleaved at multiple sites by cellular and viral proteases to produce the structural and non-structural (NS) proteins. In the case of HCV, the generation of mature non-structural proteins (NS2, NS3, NS4A, NS4B, NS5A, and NS5B) is effected by two viral proteases. The first one is believed to be a metalloprotease and cleaves at the NS2-NS3 junction; the second one is a serine protease contained within the N-terminal region of NS3 (also referred to as NS3 protease) and mediates all the subsequent cleavages downstream of NS3, both in cis, at the NS3-NS4A cleavage site, and in trans, for the remaining NS4A-NS4B, NS4B-NS5A, NS5A-NS5B sites. The NS4A protein appears to serve multiple functions, acting as a cofactor for the NS3 protease and possibly assisting in the membrane localization of NS3 and other viral replicase components. The complex formation of the NS3 protein with NS4A seems necessary to the processing events, enhancing the proteolytic efficiency at all of the sites. The NS3 protein also exhibits nucleoside triphosphatase and RNA helicase activities. NS5B (also referred to as HCV polymerase) is a RNA-dependent RNA polymerase that is involved in the replication of HCV. The HCV NS5B protein is described in "Structural Analysis of the Hepatitis C Virus RNA Polymerase in Complex with Ribonucleotides (Bressanelli; S. et al., Journal of Virology 2002, 3482-3492; and Defrancesco and Rice, Clinics in Liver Disease 2003, 7, 211-242.

US 2008/0146537, WO 2007/136982, WO 2008/112473 and US 2007/0060565 disclose cyclopropyl fused indolobenzazepine compounds which activity against hepatitis C virus and are therefore useful in treating HCV infections.

Currently, the most effective HCV therapy employs a combination of alpha-interferon and ribavirin, leading to sustained efficacy in 40% of patients (Poynard, T. et al. Lancet 1998, 352, 1426-1432). Recent clinical results demonstrate that pegylated alpha-interferon is superior to unmodified alpha-interferon as monotherapy (Zeuzem, S. et al. N. Engl. J Med. 2000, 343, 1666-1672). However, even with experimental therapeutic regimens involving combinations of pegylated alpha-interferon and ribavirin, a substantial fraction of patients do not have a sustained reduction in viral load. Thus, there is a clear and important need to develop effective therapeutics for treatment of HCV infection.

### DESCRIPTION OF THE INVENTION

One aspect of the invention is a compound of formula I 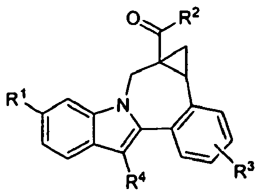 where R¹, R², R³ and R⁴ are so as to give the following compounds: 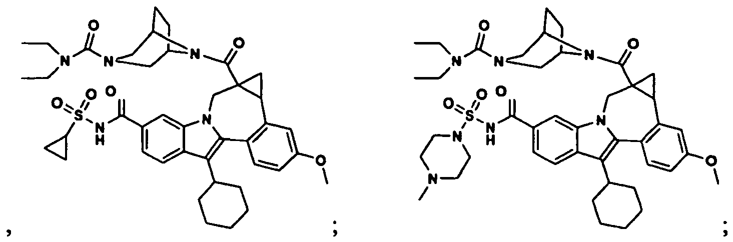 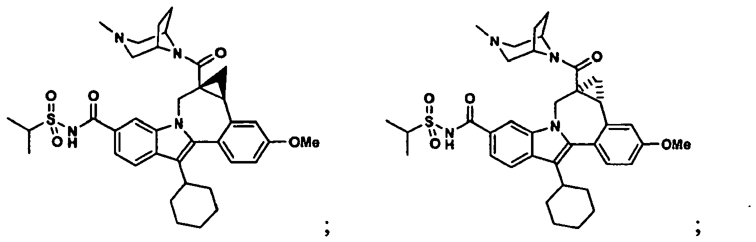 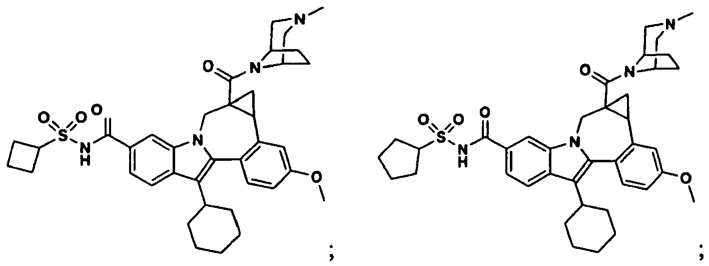 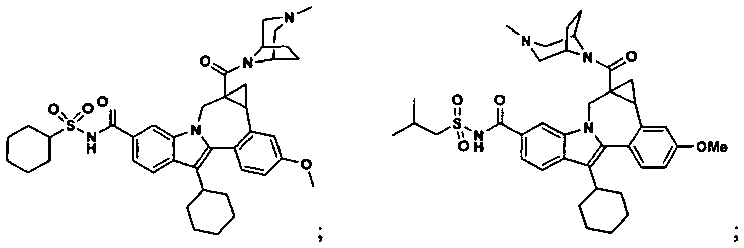 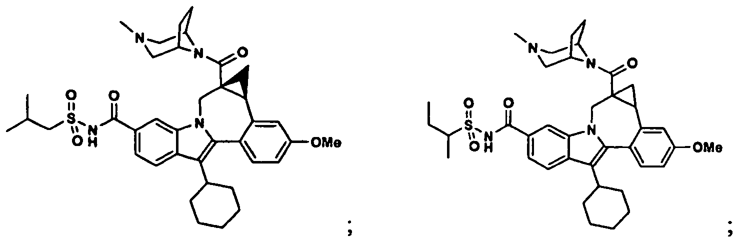 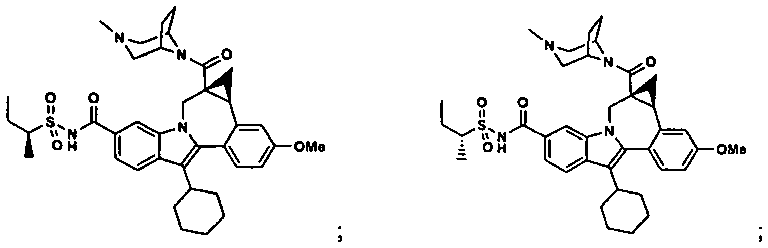 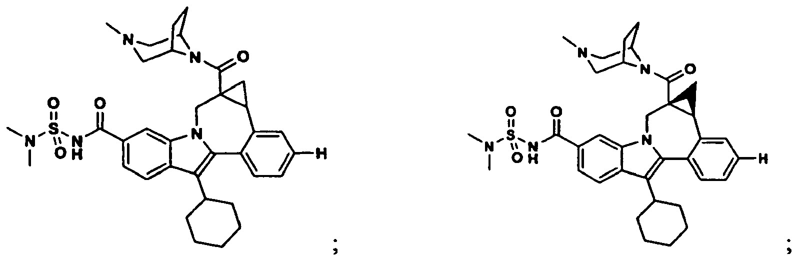 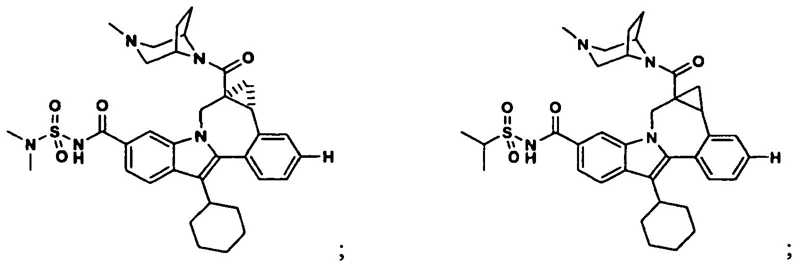 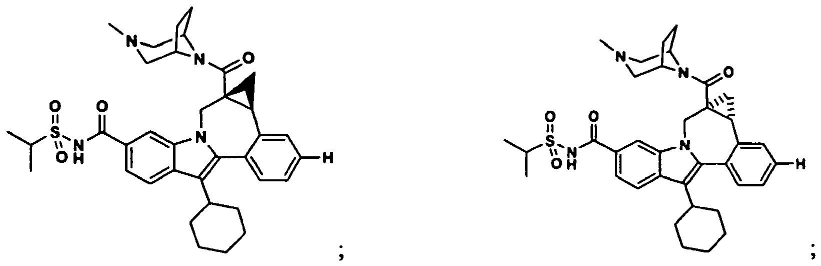 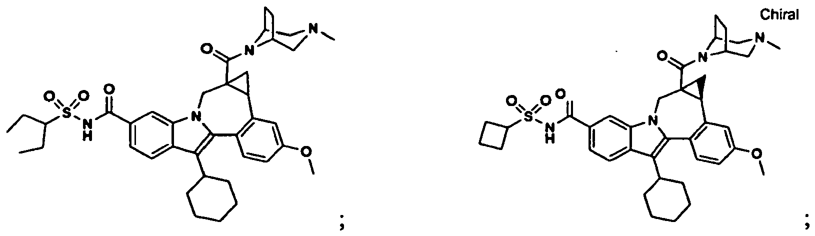 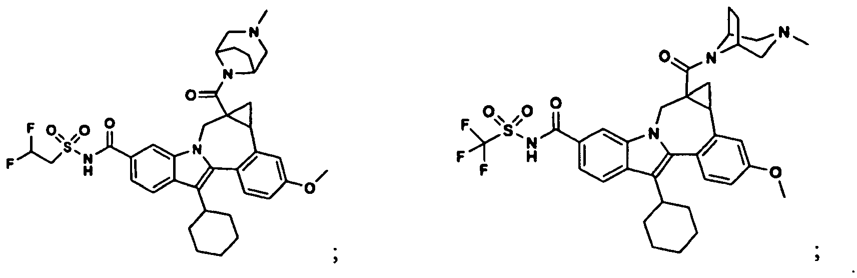 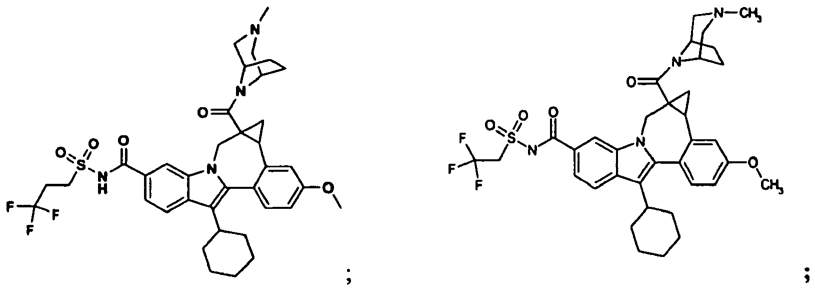 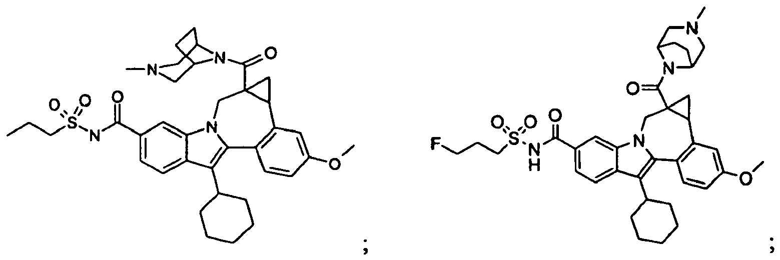 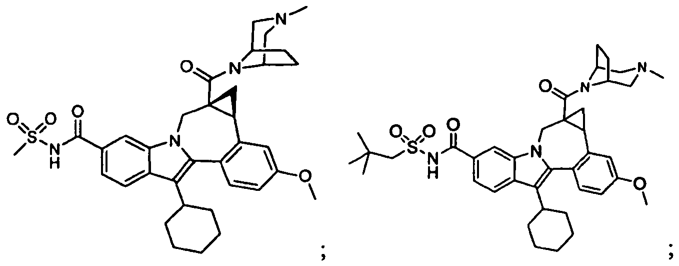 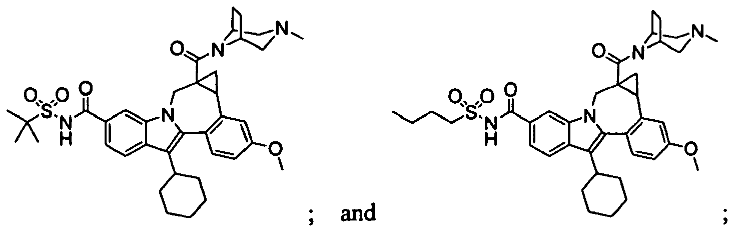 or a pharmaceutically acceptable salt thereof.

An embodiment of the invention are the compounds of the following formulae: 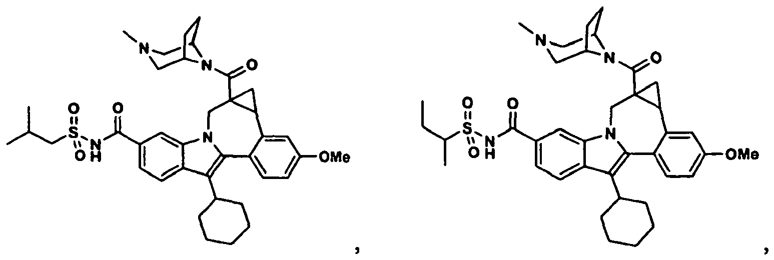 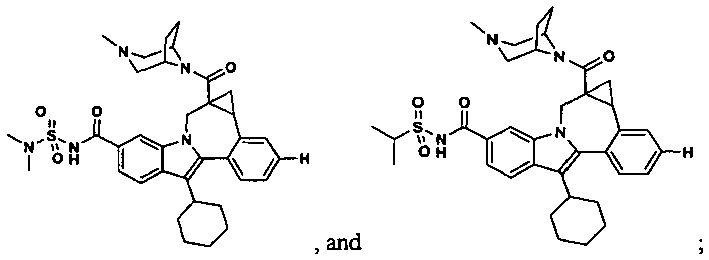 or a pharmaceutically acceptable salt thereof.

A further embodiment of the invention are the compounds of the following formulae: 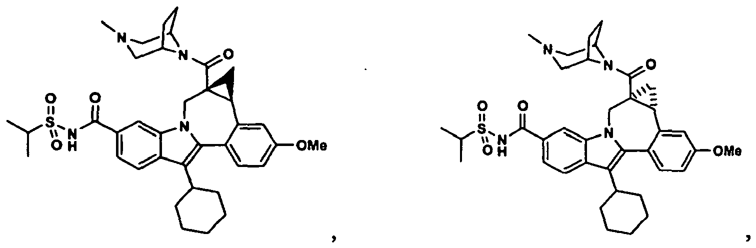 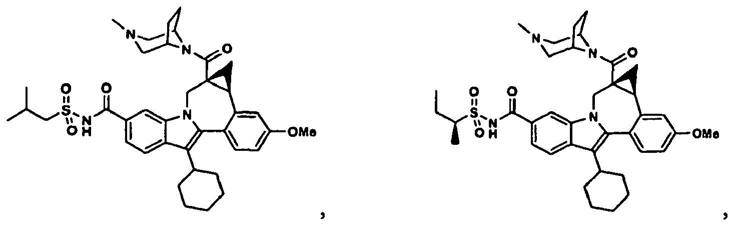 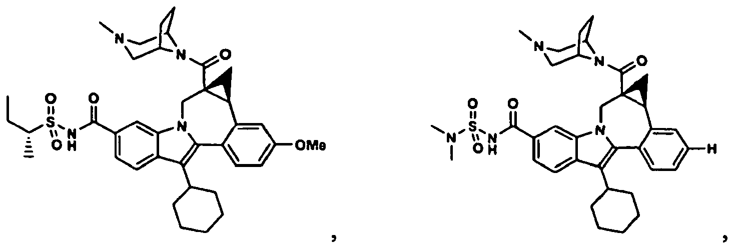 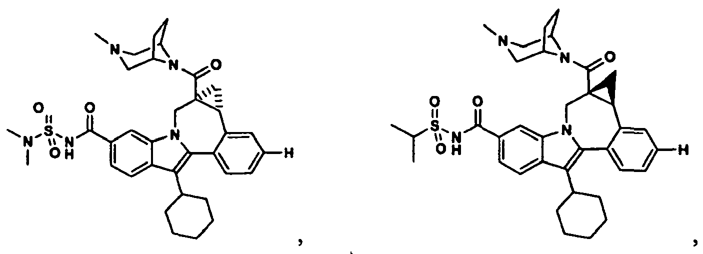 and 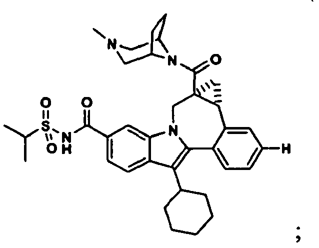 or a pharmaceutically acceptable salt thereof.

Another aspect of the invention is a compound of formula I according to the following stereochemistry.

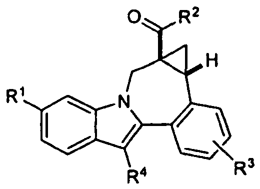

Another aspect of the invention is a compound of formula I according to the following stereochemistry.

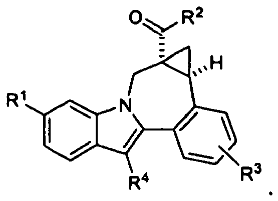

Unless specified otherwise, these terms have the following meanings. "Alkyl" means a straight or branched alkyl group composed of 1 to 6 carbons. "Alkenyl" means a straight or branched alkyl group composed of 2 to 6 carbons with at least one double bond. "Cycloalkyl" means a monocyclic ring system composed of 3 to 7 carbons. "Hydroxyalkyl," "alkoxy" and other terms with a substituted alkyl moiety include straight and branched isomers composed of I to 6 carbon atoms for the alkyl moiety. "Haloalkyl" and "haloalkoxy" include all halogenated isomers from monohalo substituted alkyl to perhalo substituted alkyl. "Aryl" includes carbocyclic and heterocyclic aromatic substituents. Parenthetic and multiparenthetic terms are intended to clarify bonding relationships to those skilled in the art. For example, a term such as ((R)alkyl) means an alkyl substituent further substituted with the substituent R.

The invention includes all pharmaceutically acceptable salt forms of the compounds. Pharmaceutically acceptable salts are those in which the counter ions do not contribute significantly to the physiological activity or toxicity of the compounds and as such function as pharmacological equivalents. These salts can be made according to common organic techniques employing commercially available reagents. Some anionic salt forms include acetate, acistrate, besylate, bromide, camsylate, chloride, citrate, fumarate, glucouronate, hydrobromide, hydrochloride, hydroiodide, iodide, lactate, maleate, mesylate, nitrate, pamoate, phosphate, succinate, sulfate, tartrate, tosylate, and xinofoate. Some cationic salt forms include ammonium, aluminum, benzathine, bismuth, calcium, choline, diethylamine, diethanolamine, lithium, magnesium, meglumine, 4-phenylcyclohexylamine, piperazine, potassium, sodium, tromethamine, and zinc.

Some of the compounds of the invention possess asymmetric carbon atoms (see, for example, the structures below). The invention includes all stereoisomeric forms, including enantiomers and diastereomers as well as mixtures of stereoisomers such as racemates. Some stereoisomers can be made using methods known in the art. Stereoisomeric mixtures of the compounds and related intermediates can be separated into individual isomers according to methods commonly known in the art. The use of wedges or hashes in the depictions of molecular structures in the following schemes and tables is intended only to indicate relative stereochemistry, and should not be interpreted as implying absolute stereochemical assignments.

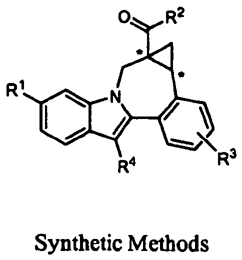

The compounds may be made by methods known in the art including those described below. Some reagents and intermediates are known in the art. Other reagents and intermediates can be made by methods known in the art using readily available materials. The variables (e.g. numbered "R" substituents) used to describe the synthesis of the compounds arc intended only to illustrate how to make and arc not to be confused with variables used in the claims or in other sections of the specification. Abbreviations used within the schemes generally follow conventions used in the art.

Methyl 2-bromo-3-cyclohexyl-1H-indole-6-carboxylate can be hydrolyzed to 2-bromo-3-cyclohexyl-1H-indole-6-carboxylic acid (See Scheme 1). This compound can be condensed with a variety of sulfonyl ureas, using for example, 1,1'-carbonyldiimidazole in combination with 1,8-diazabicyclo[5.4.0]undec-7-ene in anhydrous THF. The resultant acyl sulfamides can be subjected to known coupling reactions with a diversity of 2-formyl boronic acids or esters, using for example, Suzuki coupling conditions, to provide cyclic hemiaminal intermediates of the type depicted. These compounds can be converted to indolobenzazepines derivatives by treatment with methyl 2-(dimethoxyphosphoryl)acrylate under the influence of cesium carbonate in DMF via consecutive Michael and Horner Emmons reactions.

Related fused cyclopropyl ester derivatives can be generated by methods known in the art, including treatment of the indolobenzazepine esters with trimethyl sulfoxonium iodide under strongly basic conditions in DMSO. The residual aliphatic ester moiety in the resultant fused cyclopropanes can be hydrolyzed and the product acids can be condensed with a variety of alkyl-bridged piperazines. For example, O-(1H-benzotriazol-1-yl)-N,N, N',N'-tetramethyluronium tetrafluoroborate and diisopropyl ethyl amine in DMSO can give alkyl bridged piperazine carboxamides.

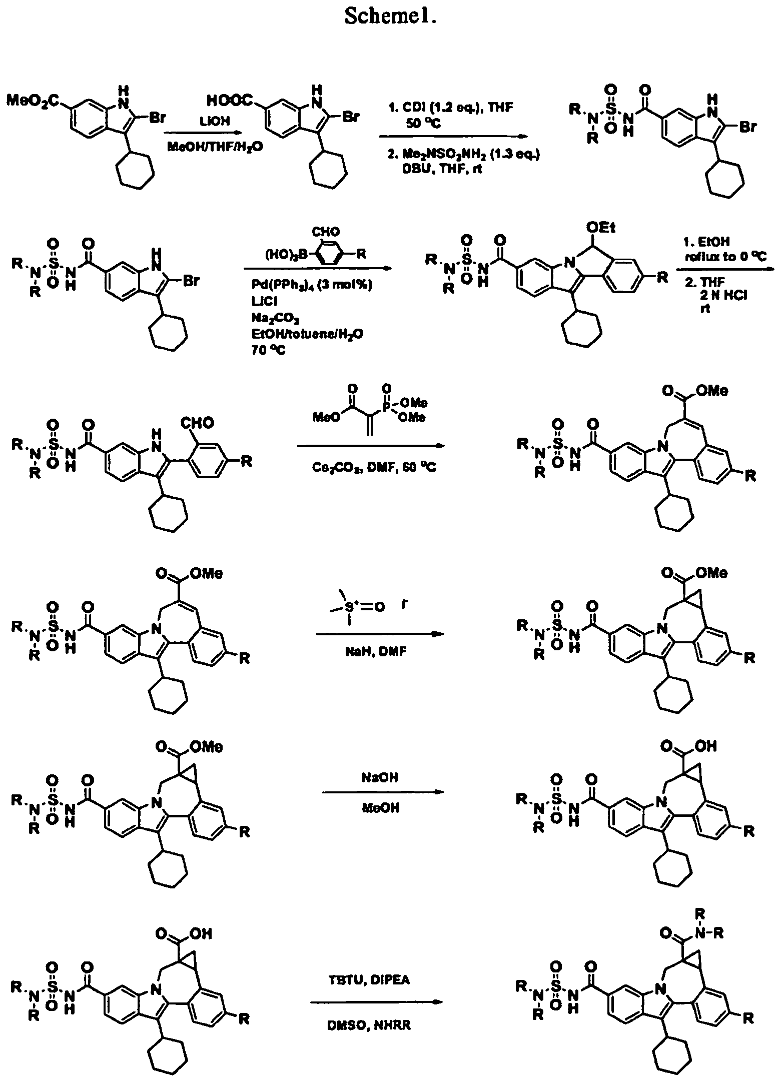

N-protected piperazines can also be coupled to the intermediate indolobenzazepine acids and the resultant piperazine carboxamides can be deprotected using methods known in the art and derivatized using a variety of synthetic protocols, some illustrative examples of which are shown below (See Scheme 2).

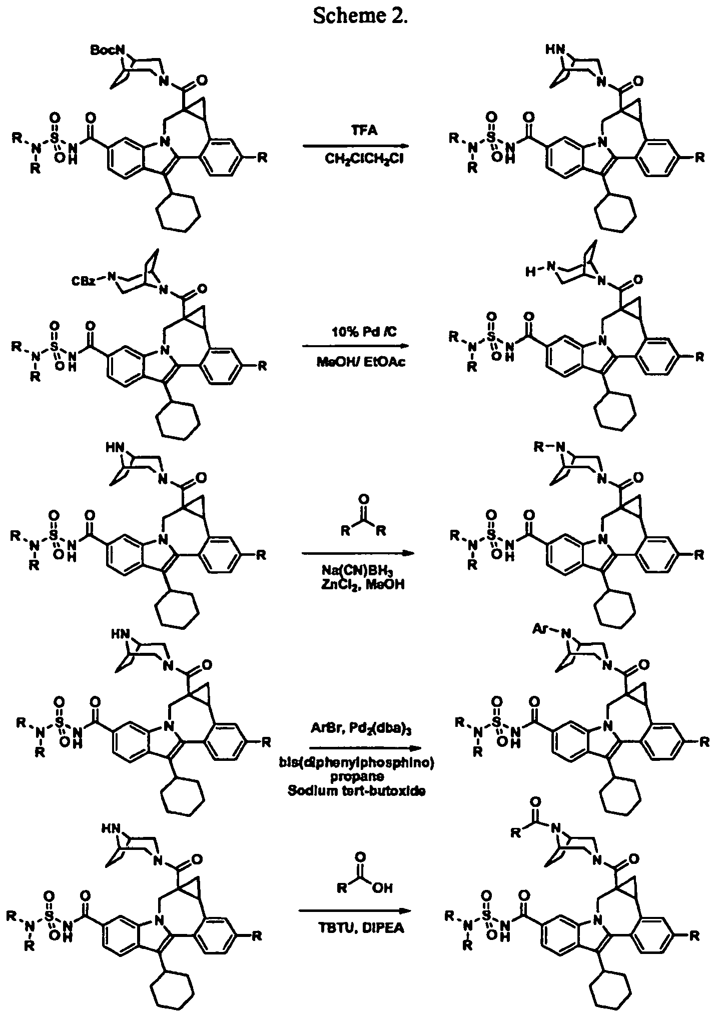

An intermediate useful for the synthesis of some compounds of the invention involves the preparation of the tert-butyl ester indolobenzazepine shown in Scheme 3.

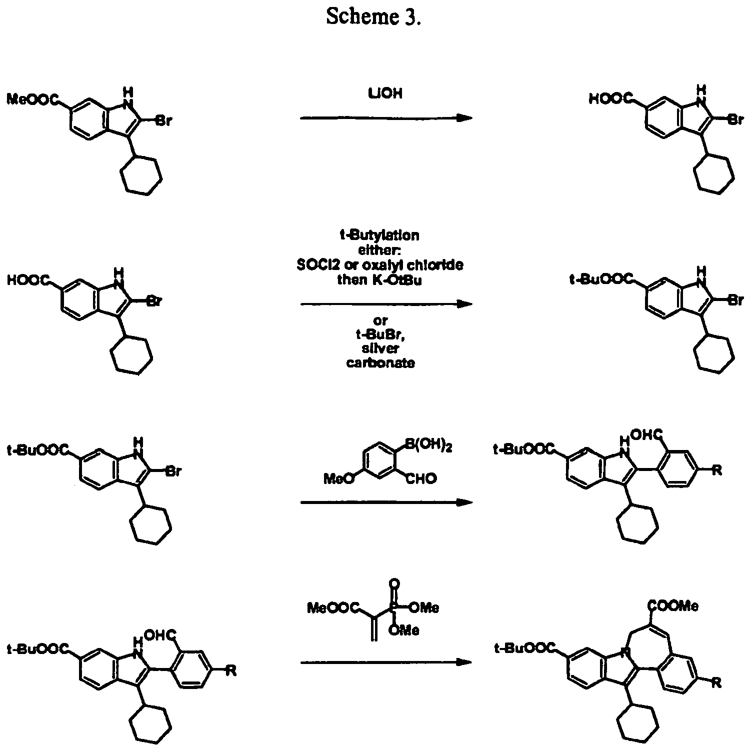

This methodology involves base catalyzed hydrolysis of the indole methyl ester shown, followed by its reaction with either thionyl chloride and potassium tertiary butoxide, or alkylation with silver carbonate and tertiary butyl bromides. The resultant compound can be transformed using chemistry analogous to that outlined previously to provide the mixed ester indolobenzazepines shown above.

These intermediates are useful in an alternative procedure that can be employed for the preparation of acylsulfamide and acylsulfonamide alkyl-bridged piperazines, as shown in Scheme 4. Cyclopropanation of an intermediate t-butyl ester indolobenzazepine and subsequent cleavage of the t-butyl ester group can generate the acid which can be coupled to a diversity of sulfonamides and sulfonylureas. Subsequent hydrolysis affords the related aliphatic acid, which can be coupled with a diversity of alkyl-bridged piperazines. For example, O-(1H-benzotriazol-1-yl)-N,N, N',N'-tetramethyluronium tetrafluoroborate and diisopropyl ethyl amine in DMSO can give the alkyl bridged piperazine carboxamides.

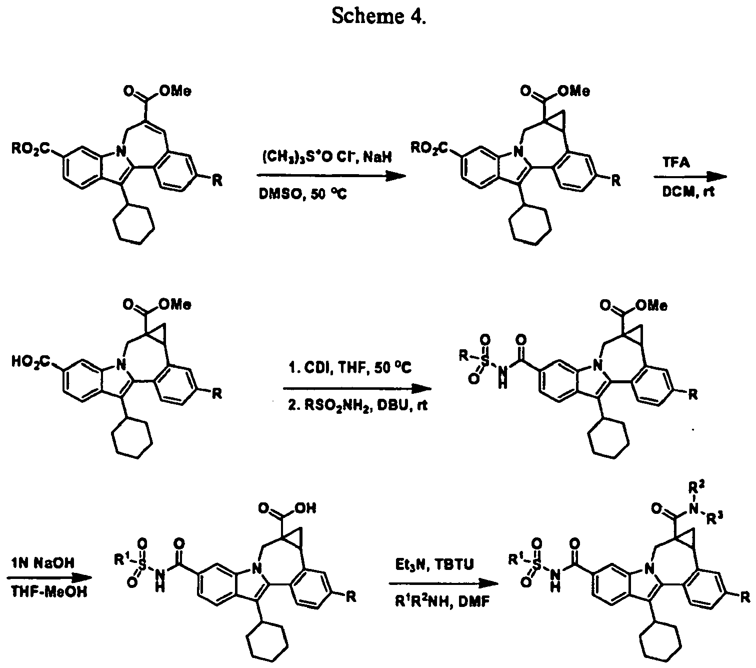

Some examples exist as stereoisomeric mixtures. The invention encompasses all stereoisomers of the compounds. Methods of fractionating stereoisomeric mixtures are well known in the art, and include but are not limited to; preparative chiral supercritical fluid chromatography (SFC) and chiral high performance liquid chromatography (HPLC). An example using this approach is shown in scheme 5.

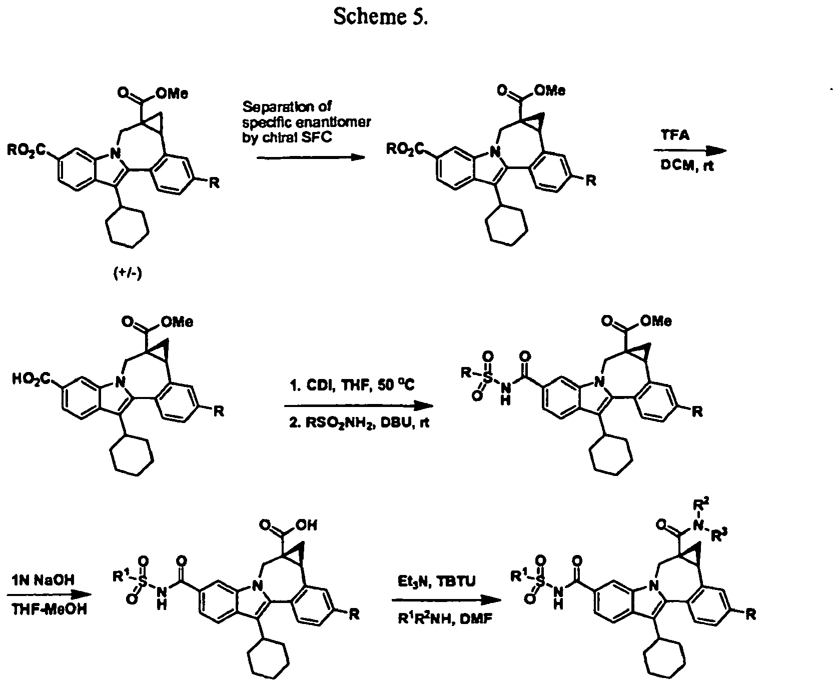

An additional method to achieve such separations involves the preparation of mixtures of diastereomers which can be separated using a variety of methods known in the art. One example of this approach is shown below (Scheme 6).

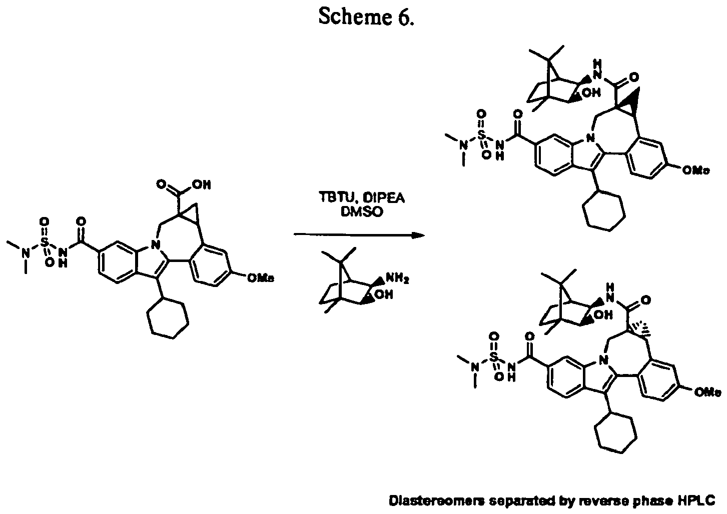

Some diastereomeric amides can be separated using reverse phase HPLC. After hydroysis, the resultant optically active acids can be coupled with bridged piperazine derivatives (Scheme 6). For example, O-(1H-benzotriazol-1-yl)-N,N, N',N'-tetramethyluronium tetrafluoroborate and diisopropyl ethyl amine in DMSO can be used to give the alkyl bridged piperazine carboxamides. Other standard acid amine coupling methods can also be used to give optically active carboxamides.

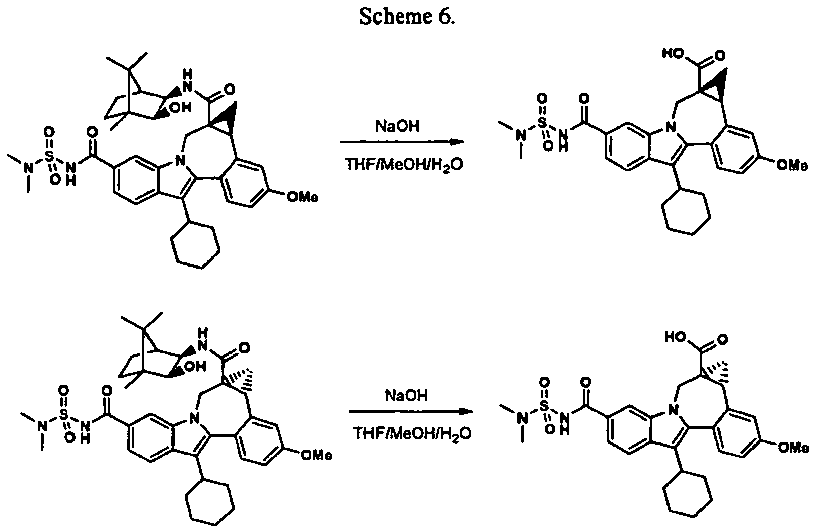

Schemes 7-9 illustrate other methods of making intermediates and compounds.

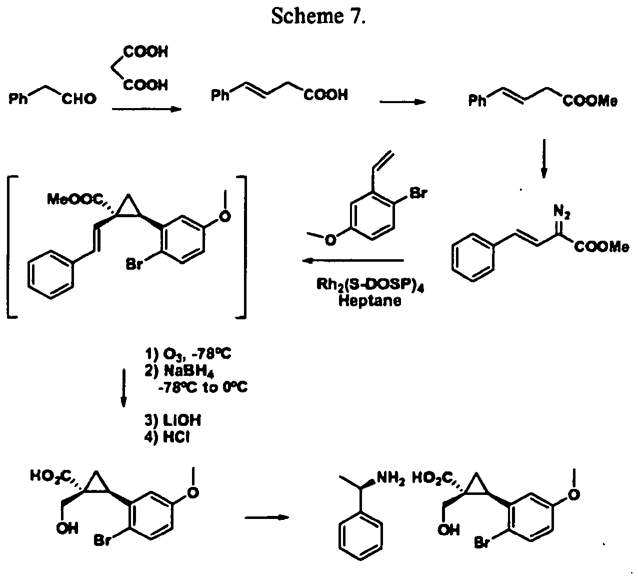

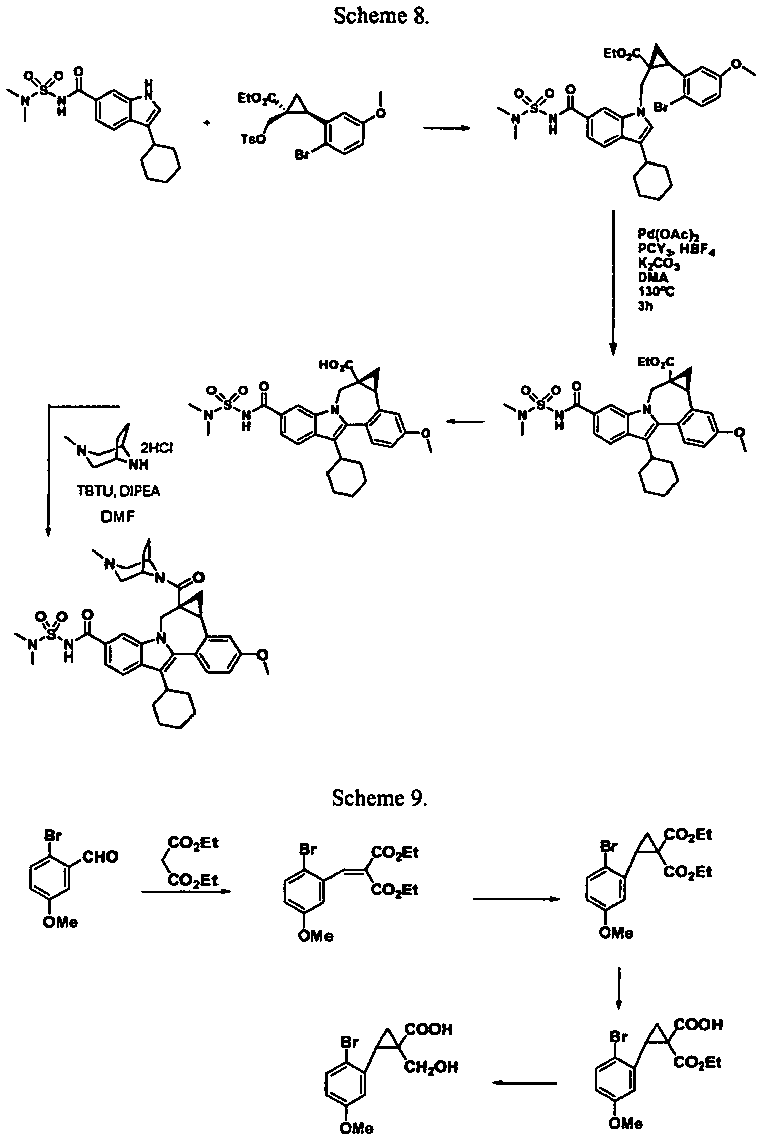

### Biological Methods

The compounds demonstrated activity against HCV NS5B as determined in the following HCV RdRp assays.

*HCV NS5B RdRp cloning, expression, and purification.* The cDNA encoding the NS5B protein of HCV, genotype 1b, was cloned into the pET21a expression vector. The protein was expressed with an 18 amino acid C-terminal truncation to enhance the solubility. The E. coli competent cell line BL21(DE3) was used for expression of the protein. Cultures were grown at 37 °C for ~ 4 hours until the cultures reached an optical density of 2.0 at 600 nm. The cultures were cooled to 20 °C and induced with 1 mM IPTG. Fresh ampicillin was added to a final concentration of 50 µg/ml and the cells were grown overnight at 20 °C.

Cell pellets (3L) were lysed for purification to yield 15-24 mgs of purified NS5B. The lysis buffer consisted of 20 mM Tris-HCl, pH 7.4, 500 mM NaCl, 0.5% triton X-100, 1 mM DTT, 1 mM EDTA, 20% glycerol, 0.5 mg/ml lysozyme, 10 mM MgCl2, 15 ug/ml deoxyribonuclease I, and Complete TM protease inhibitor tablets (Roche). After addition of the lysis buffer, frozen cell pellets were resuspended using a tissue homogenizer. To reduce the viscosity of the sample, aliquots of the lysate were sonicated on ice using a microtip attached to a Branson sonicator. The sonicated lysate was centrifuged at 100,000 x g for Ihr at 4 °C and filtered through a 0.2 µm filter unit (Coming).

The protein was purified using two sequential chromatography steps: Heparin sepharose CL-6B and polyU sepharose 4B (Pharmacia). The chromatography buffers were identical to the lysis buffer but contained no lysozyme, deoxyribonuclease I, MgCl2 or protease inhibitor and the NaCl concentration of the buffer was adjusted according to the requirements for charging the protein onto the column. Each column was eluted with a NaCl gradient which varied in length from 5-50 column volumes depending on the column type. After the final chromatography step, the resulting purity of the enzyme is >90% based on SDS-PAGE analysis. The enzyme was aliquoted and stored at -80 °C.

*Standard HCV NSSB RdRp enzyme assay.* HCV RdRp genotype 1b assays were run in a final volume of 60 µl in 96 well plates (Coming 3600). The assay buffer is composed of 20 mM Hepes, pH 7.5, 2.5 mM KCl, 2.5 mM MgCl2, 1 mM DTT, 1.6 U RNAse inhibitor (Promega N2515), 0.01 mg/ml BSA (Sigma B6917), and 2 % glycerol. All compounds were serially diluted (3-fold) in DMSO and diluted further in water such that the final concentration of DMSO in the assay was 2%. HCV RdRp genotype 1b enzyme was used at a final concentration of 28 nM. A polyA template was used at 6 nM, and a biotinylated oligo-dT12 primer was used at 180 nM final concentration. Template was obtained commercially (Amersham 27-4110). Biotinylated primer was prepared by Sigma Genosys. 3H-UTP was used at 0.6 µCi (0.29 µM total UTP). Reactions were initiated by the addition of enzyme, incubated at 30 °C for 60 min, and stopped by adding 25 µl of 50 mM EDTA containing SPA beads (4 µg/µl, Amersham RPNQ 0007). Plates were read on a Packard Top Count NXT after >1hr incubation at room temperature.

*Modified HCV NS5B RdRp enzyme assay.* A modified enzyme assay was performed essentially as described for the standard enzyme assay except for the following: The biotinylated oligo dT12 primer was precaptured on streptavidin-coated SPA beads by mixing primer and beads in assay buffer and incubating at room temperature for one hour. Unbound primer was removed after centrifugation. The primer-bound beads were resuspended in 20 mM Hepes buffer, pH 7.5 and used in the assay at final concentrations of 20 nM primer and 0.67 µg/µl beads. Order of addition in the assay: enzyme (1.75 nM) was added to diluted compound followed by the addition of a mixture of template (0.36 nM), 3H-UTP (0.6 µCi, 0.29 µM), and primer-bound beads, to initiate the reaction; concentrations given are final. Reactions were allowed to proceed for 4 hours at 30° C.

IC₅₀ values for compounds were determined using seven different [I]. IC₅₀ values were calculated from the inhibition using the formula y = A+((B-A)/(1+((C/x)^D))).

*FRET Assay Preparation.* The HCV FRET screening assay was performed in 96-well cell culture plates. The FRET peptide (Anaspec, Inc.) (Taliani et al., Anal. Biochem. 1996, 240, 60-67) contains a fluorescence donor, EDANS, near one end of the peptide and an acceptor, DABCYL, near the other end. The fluorescence of the peptide is quenched by intermolecular resonance energy transfer (RET) between the donor and the acceptor, but as the NS3 protease cleaves the peptide the products are released from RET quenching and the fluorescence of the donor becomes apparent. The assay reagent was made as follows: 5X cell Luciferase cell culture lysis reagent from Promega (#E153A) diluted to 1X with dH₂O, NaCl added to 150 mM final, the FRET peptide diluted to 20 µM final from a 2 mM stock.

To prepare plates, HCV replicon cells, with or without a Renilla luciferase reporter gene, were trypsinized and plated in a 96-well plate with titrated test compounds added in columns 3 through 12; columns 1 and 2 contained a control compound (HCV control inhibitor), and the bottom row contained cells with DMSO only. The plates were then placed in a CO₂ incubator at 37 °C.

*Assays.* Subsequent to addition of the test compounds described above (FRET Assay Preparation), at various times the plate was removed and Alamar blue solution (Trek Diagnostics, #00-100) was added to measure cellular toxicity. After reading in a Cytoflour 4000 instrument (PE Biosystems), plates were rinsed with PBS and then used for FRET assay by the addition of 30 ul of the FRET peptide assay reagent described above (FRET Assay Preparation) per well. The plate was then placed into the Cytoflour 4000 instrument which had been set to 340 excite/490 emission, automatic mode for up to 20 cycles and the plate read in a kinetic mode. Typically, the signal to noise using an endpoint analysis after the reads was at least three-fold. Alternatively, after Alamar blue reading, plates were rinsed with PBS, then used for luciferase assay using the Promega Dual-Glo Luciferase Assay System or the Promega EnduRen Live Cell Substrate assay.

Compound analysis was performed by quantification of the relative HCV replicon inhibition and the relative cytotoxicity values. To calculate cytoxicity values, the average Alamar Blue fluorescence signals from the control wells were set as 100% non-toxic. The individual signals in each of the compound test wells were then divided by the average control signal and multiplied by 100% to determine percent cytotoxicity. To calculate the HCV replicon inhibition values, an average background value was obtained from the two wells containing the highest amount of HCV control inhibitor at the end of the assay period. These numbers were similar to those obtained from naïve Huh-7 cells. The background numbers were then subtracted from the average signal obtained from the control wells and this number was used as 100% activity. The individual signals in each of the compound test wells were then divided by the averaged control values after background subtraction and multiplied by 100% to determine percent activity. EC₅₀ values were calculated as the concentration which caused a 50% reduction in FRET or luciferase activity. The two numbers generated for the compound plate, percent cytoxicity and percent activity, were used to determine compounds of interest for further analysis.

Representative data for compounds are reported in Table 1.

**Table 1.**

| Structure | IC₅₀ | EC₅₀ |
|---|---|---|
| 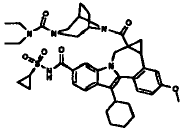 | B | B |
| 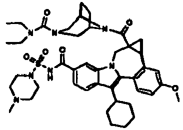 | B | B |
| 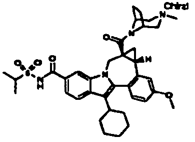 | B | B |
| 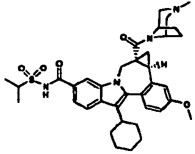 | B | B |
| 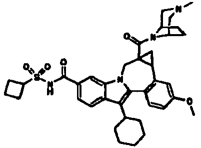 | B | B |
| 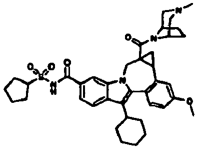 | B | B |
| 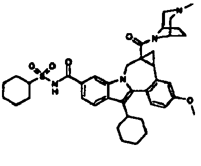 | B | B |
| 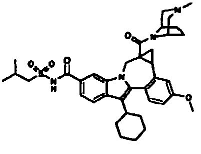 | B | B |
| 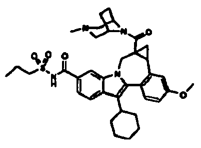 | B | B |
| 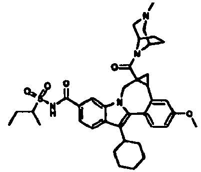 | B | B |
| 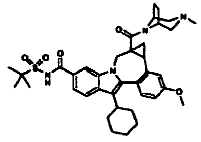 | B | B |
| 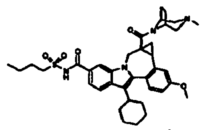 | B | B |
| 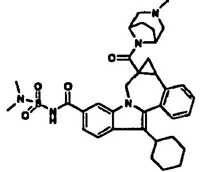 | B | B |
| 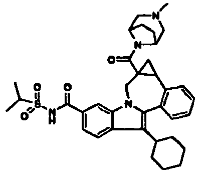 | B | B |
| 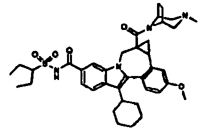 | B | E |
| 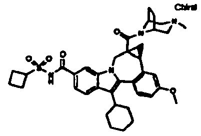 | B | B |
| 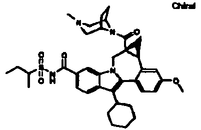 | B | B |
| 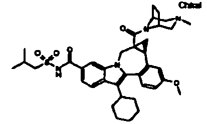 | B | B |
| 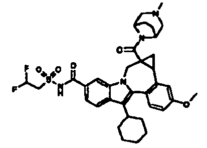 | B | B |
| 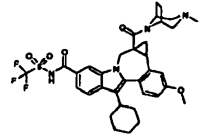 | B | B |
| 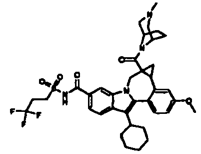 | B | B |
| 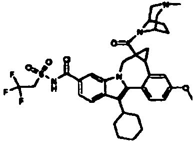 | B | B |

| | | |
|---|---|---|
| A>0.5 µM; B 0.001 µM - 0.5 µM; C <0.02 µM but an exact value was not determined; D>0.04 µM; but an exact value was not determined, D>0.11 µM; but an exact value was not determined,; | | |

### Pharmaceutical Compositions and Methods of Treatment

The compounds demonstrate activity against HCV NS5B and can be useful in treating HCV and HCV infection. Therefore, another aspect of the invention is a composition comprising a compound, or a pharmaceutically acceptable salt thereof, and a pharmaceutically acceptable carrier.

Another aspect of the invention is a composition further comprising a compound having anti-HCV activity.

Another aspect of the invention is a composition where the compound having anti-HCV activity is an interferon. Another aspect of the invention is where the interferon is selected from interferon alpha 2B, pegylated interferon alpha, consensus interferon, interferon alpha 2A, and lymphoblastoid interferon tau.

Another aspect of the invention is a composition where the compound having anti-HCV activity is a cyclosporin. Another aspect of the invention is where the cyclosporin is cyclosporin A.

Another aspect of the invention is a composition where the compound having anti-HCV activity is selected from the group consisting of interleukin 2, interleukin 6, interleukin 12, a compound that enhances the development of a type 1 helper T cell response, interfering RNA, anti-sense RNA, Imiqimod, ribavirin, an inosine 5'-monophospate dehydrogenase inhibitor, amantadine, and rimantadine.

Another aspect of the invention is a composition where the compound having anti-HCV activity is effective to inhibit the function of a target selected from HCV metalloprotease, HCV serine protease, HCV polymerase, HCV helicase, HCV NS4B protein, HCV entry, HCV assembly, HCV egress, HCV NS5A protein, IMPDH, and a nucleoside analog for the treatment of an HCV infection.

Another aspect of the invention is a composition comprising a compound, or a pharmaceutically acceptable salt thereof, a pharmaceutically acceptable carrier, an interferon and ribavirin.

Another aspect of the invention is a compound of the invention for use in a method of inhibiting the function of the HCV replicon comprising contacting the HCV replicon with a compound or a pharmaceutically acceptable salt thereof.

Another aspect of the invention is a compound of the invention for use in a method of inhibiting the function of the HCV NS5B protein comprising contacting the HCV NS5B protein with a compound or a pharmaceutically acceptable salt thereof.

Another aspect of the invention is a compound of the invention for use in a method of treating an HCV infection in a patient comprising administering to the patient a therapeutically effective amount of a compound or a pharmaceutically acceptable salt thereof. In another embodiment the compound is effective to inhibit the function of the HCV replicon. In another embodiment the compound is effective to inhibit the function of the HCV NS5B protein.

Another aspect of the invention is a compound of the invention for use in a method of treating an HCV infection in a patient comprising administering to the patient a therapeutically effective amount of a compound, or a pharmaceutically acceptable salt thereof, in conjunction with (prior to, after, or concurrently) another compound having anti-HCV activity.

Another aspect of the invention is the method where the other compound having anti-HCV activity is an interferon.

Another aspect of the invention is the method where the interferon is selected from interferon alpha 2B, pcgylatcd interferon alpha, consensus interferon, interferon alpha 2A, and lymphoblastoid interferon tau.

Another aspect of the invention is the method where the other compound having anti-HCV activity is a cyclosporin.

Another aspect of the invention is the method where the cyclosporin is cyclosporin A.

Another aspect of the invention is the method where the other compound having anti-HCV activity is selected from interleukin 2, interleukin 6, interleukin 12, a compound that enhances the development of a type 1 helper T cell response, interfering RNA, anti-sense RNA, Imiqimod, ribavirin, an inosine 5'-monophospate dehydrogenase inhibitor, amantadine, and rimantadine.

Another aspect of the invention is the method where the other compound having anti-HCV activity is effective to inhibit the function of a target selected from the group consisting of HCV metalloprotease, HCV serine protease, HCV polymerase, HCV helicase, HCV NS4B protein, HCV entry, HCV assembly, HCV egress, HCV NS5A protein, IMPDH, and a nucleoside analog for the treatment of an HCV infection.

Another aspect of the invention is the method where the other compound having anti-HCV activity is effective to inhibit the function of target in the HCV life cycle other than the HCV NS5B protein.

"Therapeutically effective" means the amount of agent required to provide a meaningful patient benefit as understood by practitioners in the field of hepatitis and HCV infection.

"Patient" means a person infected with the HCV virus and suitable for therapy as understood by practitioners in the field of hepatitis and HCV infection.

"Treatment," "therapy," "regimen," "HCV infection," and related terms are used as understood by practitioners in the field of hepatitis and HCV infection.

The compounds of this invention are generally given as pharmaceutical compositions comprised of a therapeutically effective amount of a compound or its pharmaceutically acceptable salt and a pharmaceutically acceptable carrier and may contain conventional excipients. A therapeutically effective amount is that which is needed to provide a meaningful patient benefit. Pharmaceutically acceptable carriers are those conventionally known carriers having acceptable safety profiles. Compositions encompass all common solid and liquid forms including capsules, tablets, losenges, and powders as well as liquid suspensions, syrups, elixers, and solutions. Compositions are made using common formulation techniques, and conventional excipients (such as binding and wetting agents) and vehicles (such as water and alcohols) are generally used for compositions.

Solid compositions are normally formulated in dosage units and compositions providing from about 1 to 1000 mg of the active ingredient per dose are preferred. Some examples of dosages are 1 mg, 10 mg, 100 mg, 250 mg, 500 mg, and 1000 mg. Generally, other agents will be present in a unit range similar to agents of that class used clinically. Typically, this is 0.25-1000 mg/unit.

Liquid compositions are usually in dosage unit ranges. Generally, the liquid composition will be in a unit dosage range of 1-100 mg/mL. Some examples of dosages are 1 mg/mL, 10 mg/mL, 25 mg/mL, 50 mg/mL, and 100 mg/mL. Generally, other agents will be present in a unit range similar to agents of that class used clinically. Typically, this is 1-100 mg/mL.

The invention encompasses all conventional modes of administration; oral and parenteral methods are preferred. Generally, the dosing regimen will be similar to other agents used clinically. Typically, the daily dose will be 1-100 mg/kg body weight daily. Generally, more compound is required orally and less parenterally. The specific dosing regime, however, will be determined by a physician using sound medical judgement.

The invention also encompasses methods where the compound is given in combination therapy. That is, the compound can be used in conjunction with, but separately from, other agents useful in treating hepatitis and HCV infection. In these combination methods, the compound will generally be given in a daily dose of 1-100 mg/kg body weight daily in conjunction with other agents. The other agents generally will be given in the amounts used therapeutically. The specific dosing regime, however, will be determined by a physician using sound medical judgement.

Some examples of compounds suitable for compositions and methods are listed in Table 2.

**Table 2.**

| Brand Name | Type of Inhibitor or Target | Source Company |
|---|---|---|
| Omega IFN | IFN-ω | Intarcia Therapeutics |
| BILN-2061 | serine protease inhibitor | Boehringer Ingelheim Pharma KG, Ingelheim, Germany |
| Summetrel | antiviral | Endo Pharmaceuticals Holdings Inc., Chadds Ford, PA |
| Roferon A | IFN-α2a | F. Hoffmann-La Roche LTD, Basel, Switzerland |
| Pegasys | PEGylated IFN-α2a | F. Hoffmann-La Roche LTD, Basel, Switzerland |
| Pegasys and Ribavirin | PEGylated IFN-α2a/ribavirin | F. Hoffmann-La Roche LTD, Basel, Switzerland |
| CellCept | HCV IgG immunosuppressant | F. Hoffmann-La Roche LTD, Basel, Switzerland |
| Wellferon | lymphoblastoid IFN-αn1 | GlaxoSmithKline plc, Uxbridge, UK |
| Albuferon - α | albumin IFN-α2b | Human Genome Sciences Inc., Rockville, MD |
| Levovirin | ribavirin | ICN Pharmaceuticals, Costa Mesa, CA |
| IDN-6556 | caspase inhibitor | Idun Pharmaceuticals Inc., San Diego, CA |
| IP-501 | antifibrotic | Indevus Pharmaceuticals Inc., Lexington, MA |
| Actimmune | INF-γ | InterMune Inc., Brisbane, CA |
| Infergen A | IFN alfacon-1 | InterMune Pharmaceuticals Inc., Brisbane, CA |
| ISIS 14803 | antisense | ISIS Pharmaceuticals Inc, Carlsbad, CA/Elan Phamaceuticals Inc., New York, NY |
| JTK-003 | RdRp inhibitor | Japan Tobacco Inc., Tokyo, Japan |
| Pegasys and Ceplene | PEGylated IFN-α2a/ immune modulator | Maxim Pharmaceuticals Inc., San Diego, CA |
| Ceplene | immune modulator | Maxim Pharmaceuticals Inc., San Diego, CA |
| Civacir | HCV IgG immunosuppressant | Nabi Biopharmaceuticals Inc., Boca Raton, FL |
| Intron A and Zadaxin | IFN-α2b/α1-thymosin | RegeneRx Biopharmiceuticals Inc., Bethesda, MD/ SciClone Pharmaceuticals Inc, San Mateo, CA |
| Levovirin | IMPDH inhibitor | Ribapharm Inc., Costa Mesa, CA |
| Viramidine | Ribavirin Prodrug | Ribapharm Inc., Costa Mesa, CA |
| Heptazyme | ribozyme | Ribozyme Pharmaceuticals Inc., Boulder, CO |
| Intron A | IFN-α2b | Schering-Plough Corporation, Kenilworth, NJ |
| PEG-Intron | PEGylated IFN-α2b | Schering-Plough Corporation, Kenilworth, NJ |
| Rebetron | IFN-α2b/ribavirin | Schering-Plough Corporation, Kenilworth, NJ |
| Ribavirin | ribavirin | Schering-Plough Corporation, Kenilworth, NJ |
| PEG-Intron / Ribavirin | PEGylated IFN-α2b/ribavirin | Schering-Plough Corporation, Kenilworth, NJ |
| Zadazim | Immune modulator | SciClone Pharmaceuticals Inc., San Mateo, CA |
| Rebif | IFN-β1a | Serono, Geneva, Switzerland |
| IFN-β and EMZ701 | IFN-β and EMZ701 | Transition Therapeutics Inc., Ontario, Canada |
| Batabulin (T67) | β-tubulin inhibitor | Tularik Inc., South San Francisco, CA |
| Merimepodib (VX-497) | IMPDH inhibitor | Vertex Pharmaceuticals Inc., Cambridge, MA |
| Telaprevir (VX-950, LY-5703 10) | NS3 serine protease inhibitor | Vertex Pharmaceuticals Inc., Cambridge, MA/ Eli Lilly and Co. Inc., Indianapolis, IN |
| Omniferon | natural IFN-α | Viragen Inc., Plantation, FL |
| XTL-6865 (XTL-002) | monoclonal antibody | XTL Biopharmaceuticals Ltd., Rehovot, Isreal |
| HCV-796 | NS5B Replicase Inhibitor | Wyeth / Viropharma |
| NM-283 | NS5B Replicase Inhibitor | Idenix / Novartis |
| GL-59728 | NS5B Replicase Inhibitor | Gene Labs / Novartis |
| GL-60667 | NS5B Replicase Inhibitor | Gene Labs / Novartis |
| 2'C MeA | NS5B Replicase Inhibitor | Gilead |
| PSI 6130 | NS5B Replicase Inhibitor | Roche |
| R1626 | NS5B Replicase Inhibitor | Roche |
| SCH 503034 | serine protease inhibitor | Schering Plough |
| NIM811 | Cyclophilin Inhibitor | Novartis |
| Suvus | Methylene blue | I Bioenvision |
| Multiferon | Long lasting IFN | Viragen/Valentis |
| Actilon (CPG10101) | TLR9 agonist | Coley |
| Interferon-β | Interferon-β-1a | Serono |
| Zadaxin | Immunomodulator | Sciclone |
| Pyrazolopyrimidine compounds and salts From WO-2005047288 26 May 2005 | HCV Inhibitors | Arrow Therapeutics Ltd. |
| 2'C Methyl adenosine | NS5B Replicase Inhibitor | Merck |
| GS-9132 (ACH-806) | HCV Inhibitor | Achillion / Gilead |

### DESCRIPTION OF SPECIFIC EMBODIMENTS

Unless otherwise specified, analytical LCMS data on the following intermediates and examples were acquired using the following columns and conditions. Stop time: Gradient time + I minute; Starting conc: 0% B unless otherwise noted; Eluent A: 5% CH₃CN / 95% H₂O with 10 mM NH₄OAc (for columns A, D and E); 10 % MeOH / 90 % H₂O with 0.1% TFA (for columns B and C); Eluent B: 95% CH₃CN / 5% H₂O with 10 mM NH₄OAc (for columns A, D and E); 90 % MeOH / 10 % H₂O with 0.1% TFA (for columns B and C); Column A: Phenomenex 10µ 4.6 x 50 mm C 18; Column B: Phenomenex C18 10µ 3.0 x 50 mm; Column C: Phenomenex 4.6 x 50 mm C18 10µ; Column D: Phenomenex Lina C18 5µ 3.0 x 50 mm; Column E: Phenomenex 5µ 4.6 x 50 mm C18.

As an artifact of the graphics software, some structures have missing hydrogen atoms.

### Intermediate 1

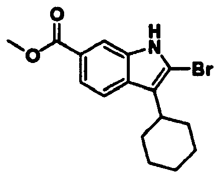

*1H-Indole-6-carboxylic acid, 2-bromo-3-cyclohexyl-, methyl ester.* Freshly recrystallized pyridinium tribromide (recrystallization from hot AcOH (5 mL per 1 g), rinsed with cold AcOH and dried under high vacuum over KOH) was added in portions (over 10 min.) to a stirring solution of methyl 3-cyclohexyl-1H-indole-6-carboxylate (60 g, 233 mmol) (prepared using procedures describe in W02004/065367) in CHCl₃/THF (1:1, 1.25 L) at 2o C. The reaction solution was stirred at 0-5 °C for 2.5h, and washed with sat. aq. NaHSO₃ (1 L), 1 N HCl (1 L) and brine (1 L). The organic layer was dried (MgSO₄) and concentrated. The resulting red oil was diluted with Et₂O and concentrated. The resulting pink solid was dissolved into Et₂O (200 mL) treated with hexanes (300 mL) and partially concentrated. The solids were collected by filtration and rinsed with hexanes. The mother liquor was concentrated to dryness and the procedure repeated. The solids were combined to yield 1H-indole-6-carboxylic acid, 2-bromo-3-cyclohexyl-, methyl ester (64 g, 190 mmol, 82%) as a fluffy pink solid, which was used without further purification. 1HNMR (300 MHz), CDCl₃) δ 8.47 (br s, 1H), 8.03 (d, J = 1.4 Hz, 1H), 7.74 (dd, J = 1.4, 8.8 Hz, 1H), 7.69 (d, J = 8.8 Hz, 1H), 3.92 (s, 3H), 2.82 (tt, J = 3.7, 11.7 Hz, 1H), 1.98 - 1.72 (m, 7H), 1.50 - 1.27 (m, 3H). 13CNMR (75 MHz, CDCl3) δ 168.2, 135.6, 130.2, 123.1, 120.8, 120.3, 118.7, 112.8, 110.7, 52.1, 37.0, 32.2(2), 27.0(2), 26.1. LCMS: m/e 334 (M-H)⁻, ret time 3.34 min, column A, 4 minute gradient.

### Intermediate 2

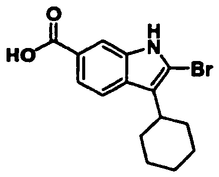

*1H-Indole-6-carboxylic acid, 2-bromo-3-cyclohexyl-.* A solution of methyl 2-bromo-3-cyclohexyl-1*H*-indole-6-carboxylate (20 g, 60 mmol) and LiOH (3.8 g, 160 mmol) in MeOH/THF/H₂O (1:1:1, 300 mL) was heated at 90 °C for 2h. The reaction mixture was cooled in an ice/H₂O bath, neutralized with 1M HCl (~160 mL) diluted with H₂O (250 mL) and stirred for 1h at rt. The precipitates were collected by filtration rinse with H₂O and dried to yield 1H-indole-6-carboxylic acid, 2-bromo-3-cyclohexyl- (quant.) which was used without further purification.

An alternative procedure that can by used to provide 1H-indole-6-carboxylic acid, 2-bromo-3-cyclohexyl- is described below:

A solution of methyl 2-bromo-3-cyclohexyl-1H-indole-6-carboxylate (117 g, 349 mmol) and LiOH.H₂O (26.4 g, 629 mmol) in MeOH/THF/H2O (1:1:1, 1.8 L) was heated at reflux for 3h. The reaction mixture was cooled in an ice/H2O bath to ~2 °C, neutralized with 1M HCl (~650 mL) (added at such a rate that temperature did not exceed 5 °C), diluted with H2O (1 L) and stirred while warming to ambient temperature. The precipitates were collected by filtration rinsed with H₂O and dried to yield the mono THF solvate of 1H-indole-6-carboxylic acid, 2-bromo-3-cyclohexyl- (135.5 g, 345 mmol, 99%) as a yellow solid, which was used without further purification. 1HNMR (300 MHz, CDCl₃) δ 11.01 (br s, 1H), 8.77 (s, 1H), 8.07 (d, J = 1.5 Hz, 1H), 7.82 (dd, J = 1.5, 8.8 Hz, 1H), 7.72 (d, J = 8.8 Hz, 1H), 3.84 - 3.74 (m, 4H), 2.89 (m, 1H), 1.98 - 1.72 (m, 11H), 1.50 - 1.24 (m, 3H). 13CNMR (75 MHz, CDCl3) δ 172.7, 135.5, 130.7, 122.3, 120.9(2), 118.8, 113.3, 111.1, 67.9(2), 37.0, 32.2(2), 27.0(2), 26.1, 25.5(2). LCMS: m/e 320 (M-H)⁻, ret time 2.21 min, column A, 4 minute gradient.

### Intermediate 3

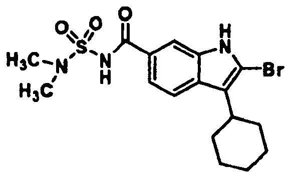

*1H-Indole-6-carboxamide, 2-bromo-3-cyclohetyl-N-[(dimethylamino)sulfonyl]-.* 1,1'-Carbonyldiimidazole (1.17 g, 7.2 mmol) was added to a stirred solution of 2-bromo-3-cyclohexyl-1H-indole-6-carboxylic acid (2.03 g, 6.3 mmol) in THF (6 mL) at 22 °C. The evolution of CO₂ was instantaneous and when it slowed the solution was heated at 50°C for 1 hr and then cooled to 22°C. N,N-Dimethylsulfamide (0.94 g, 7.56 mmol) was added followed by the dropwise addition of a solution of DBU (1.34 g ,8.8 mmol) in THF (4 mL). Stirring was continued for 24 hr. The mixture was partitioned between ethyl acetate and dilute HCl. The ethyl acetate layer was washed with water followed by brine and dried over Na₂SO₄. The extract was concentrated to dryness to leave the title product as a pale yellow friable foam, (2.0 g, 74 %, >90 % purity, estimated from NMR). ¹H NMR (300 MHz, DMSO-D6) δ ppm 1.28 - 1.49 (m, 3 H) 1.59 - 2.04 (m, 7 H) 2.74 - 2.82 (m, 1 H) 2.88 (s, 6 H) 7.57 (dd, *J*=8.42, 1.46 Hz, 1 H) 7.74 (d, *J*=8.78 Hz, 1 H) 7.91 (s, 1 H) 11.71 (s, 1 H) 12.08 (s, 1 H).

An alternative method for the preparation of 1H-indole-6-carboxamide, 2-bromo-3-cyclohexyl-N-[(dimethylamino)sulfonyl]- is described below.

To a 1 L four necked round bottom flask equipped with a mechanical stirrer, a temperature controller, a N2 inlet, and a condenser, under N2, was added 2-bromo-3-cyclohexyl-1*H*-indole-6-carboxylic acid (102.0 g, 0.259 mol) and dry THF (300 mL). After stirring for 10 min, CDI (50.3 g, 0.31 mol) was added portion wise. The reaction mixture was then heated to 50 oC for 2 h. After cooling to 30 oC, *N,N-*dimethylaminosulfonamide (41.7 g, 0.336 mol) was added in one portion followed by addition of DBU (54.1 mL, 0.362 mol) drop wise over a period of 1 h. The reaction mixture was then stirred at rt for 20 h. The solvent was removed in vacuo and the residue was partitioned between EtOAc and 1 N HCl (1 : 1, 2 L). The organic layer was separated and the aqueous layer was extracted with EtOAc (500 mL). The combined organic layers were washed with brine (1.5 L) and dried over MgSO4. The solution was filtered and concentrated in vacuo to give the crude product (111.0 g). The crude product was suspended in EtOAc (400 mL) at 60 oC. To the suspension was added heptane (2 L) slowly. The resulting suspension was stirred and cooled to 0 oC. It was then filtered. The filter cake was rinsed with small amount of heptane and house vacuum air dried for 2 days. The product was collected as a white solid (92.0 g, 83%). ¹H NMR (MeOD, 300 MHz) δ 7.89 (s, H), 7.77 (d, *J* = 8.4 Hz, 1H), 7.55 (dd, *J* = 8.4 and 1.8 Hz, 1H), 3.01 (s, 6H), 2.73-2.95 (m, 1H), 1.81-2.05 (m, 8H), 1.39-1.50 (m, 2H); m/z 429 (M +H)+.

### Intermediate 4

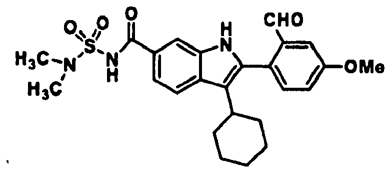

*1H-Indole-6-carboxamide, 3-cyclohexyl-N-[(dimethylamino)sulfonyl]-2-(2-formyl-4-methoxyphenyl)-.* A mixture of the 2-Bromo-3-cyclohexyl- N-[(dimethylamino)sulfonyl]-1H-indole-6-carboxamide (4.28g, 0.01 mol), 4-methoxy-2-formylphenyl boronic acid (2.7g, 0.015 mol), 2-dicyclohexylphosphino-2',6'-dimethoxy-biphenyl (41 mg, 0.0001 mol), palladium acetate (11.2 mg), and finely ground potassium carbonate (4.24g, 0.02 mol) in toluene (30 mL) was stirred under reflux and under nitrogen for 30 min, at which time LC/MS analysis showed the reaction to be complete. The reaction mixture was then diluted with ethyl acetate and water, and then acidified with an excess of dilute HCl. The ethyl acetate layer was then collected and washed with dilute HCl, water and brine. The organic solution was then dried (magnesium sulfate), filtered and concentrated to give a gum. The gum was diluted with hexanes (250 ml) and ethyl acetate (25 mL), and the mixture was stirred for 20 hr at 22° C during which time the product was transformed into a bright yellow granular solid (4.8 g) which was used directly without further purification.

An alternative procedure for the preparation of 1H-indole-6-carboxamide, 3-cyclohexyl-N-[(dimethylamino)sulfonyl]-2-(2-formyl-4-methoxyphenyl)- is provided below:

To a slurried solution of 2-bromo-3-cyclohexyl-N-[(dimethylamino)sulfonyl]-indole-6-carboxamide (54.0 g, 126 mmol), 4-methoxy-2-formylphenylboronic acid (29.5 g, 164 mmol) and LiCl (13.3 g, 315 mmol) in EtOH/toluene (1:1, 1 L) was added a solution of Na₂CO₃ (40.1 g, 379 mmol) in water (380 mL). The reaction mixture was stirred 10 min. and then Pd(PPh3)4 (11.3 g, 10.0 mmol) was added. The reaction solution was flushed with nitrogen and heated at 70 °C (internal monitoring) overnight and then cooled to rt. The reaction was diluted with EtOAc (1 L) and EtOH (100 mL), washed carefully with 1N aqueous HCl (1 L) and brine (500 mL), dried (MgSO4), filtered and concentrated. The residual solids were stirred with Et2O (600 mL) for 1h and collected by filtration to yield 1H-indole-6-carboxamide, 3-cyclohexyl-N-[(dimethylamino)sulfonyl]-2-(2-formyl-4-methoxyphenyl)- (52.8g, 109 mmol, 87%) as a yellow powder which was used without further purification. 1HNMR (300 MHz, d6-DMSO) δ 11.66 (s, 1H), 8.17 (s, 1H), 7.75 (d, J = 8.4 Hz, 1H), 7.74 (d, J = 8.4 Hz, 1H), 7.59 (dd, J = 1.4, 8.4 Hz, 1H), 7.23 - 7.16 (m, 2H), 7.08 (dd, J = 2.6, 8.4 Hz, 1H), 6.54 (d, J = 8.8 Hz, 1H), 3.86 (s, 3H), 3.22 - 3.08 (m, 1H), 2.91 (s, 6H), 2.00 - 1.74 (m, 7H), 1.60 - 1.38 (m, 3H). 13CNMR (75 MHz), CDCl3) δ 165.7, 158.8, 147.2, 139.1, 134.3, 132.0, 123.4, 122.0, 119.2, 118.2, 114.8, 112.3, 110.4, 109.8, 79.6, 45.9, 37.2(2), 34.7, 32.0(2), 25.9(2), 24.9. LCMS: m/e 482 (M-H)⁻, ret time 2.56 min, column A, 4 minute gradient.

### Intermediate 5

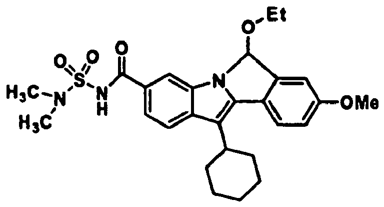

*6H-Isoindolo[2,1-a]indole-3-carboxamide, 11-cyclohexyl-N-[(dimethylamino)sulfonyl]-6-ethoxy-8-methoxy-.* To a 5 L four necked round bottom flask equipped with a temperature controller, a condenser, a N2 inlet and a mechanical stirrer, was charged toluene (900 mL), EtOH (900 mL), 2-bromo-3-cyclohexyl-*N*-(*N*,*N*-dimethylsulfamoyl)-1*H*-indole-6-carboxamide (90 g, 0.21 mol), 2-formyl-4-methoxyphenylboronic acid (49.2 g, 0.273 mol) and LiCl (22.1 g, 0.525 mol). The resulting solution was bubbled with N₂ for 15 mins. A solution of Na₂CO₃ (66.8 g, 0.63 mol) in H₂O (675 mL) was added and the reaction mixture was bubbled with N₂ for another (10 mins). Pd(PPh₃)₄ (7.0 g, 6.3 mmol) was added and the reaction mixture was heated to 70 °C for 20 h. After cooling to 35 °C, a solution of 1 N HCl (1.5 L) was added slowly. The resulting mixture was transferred to a 6 L separatory funnel and extracted with EtOAc (2 X 1.5 L). The combined organic extracts were washed with brine (2 L), dried over MgSO4, filtered and concentrated in vacuo to give a yellow solid, which was triturated with 20% EtOAc in hexane (450 mL, 50 °C to 0 °C) to give 3-cyclohexyl-N-(N,N-dimethylsulfamoyl)-2-(2-formyl-4-methoxyphenyl)-1H-indole-6-carboxamide(65.9 g) as a yellow solid. HPLC purity, 98%.

The mother liquid from the trituration was concentrated in vacuo. The residue was refluxed with EtOH (50 mL) for 3 h. The solution was then cooled to 0 °C. The precipitates were filtered and washed with cooled TBME (5 °C) (20 mL). The filter cake was house vacuum air dried to give a further quantity of the title compound as a white solid (16.0 g). HPLC purity, 99%. ¹H NMR (CDCl3, 300 MHz) δ 8.75 (s, 1H), 7.96 (s, 1H), 7.73 (d, *J* = 8.4 Hz, 1H), 7.67 (d, *J* = 8.4 Hz, 1H), 7.45 (dd, *J* = 8.4 and 1.4 Hz, 1H), 7.09 (d, *J* = 2.2 Hz, 1H), 6.98 (dd, *J* = 8.4 and 2.2 Hz, 1H), 6.50 (s, 1H), 3.86 (s, 3H), 3.05 (s, 6H), 2.92-3.13 (m, 3H), 1.85-1.93 (m, 7 H), 1.40-1.42 (m, 3H), 1.05 (t, *J* = 7.1 Hz, 3H). m/z 512 (M + H)+.

### Intermediate 6

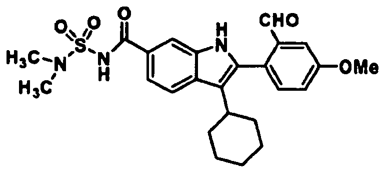

*1H-indole-6-carboxamide, 3-cyclohexyl-N-[(dimethylamino)sulfonyl]-2-(2-formyl-4-methoxyphenyl)-.* 11-cyclohexyl-N-(N,N-dimethylsulfamoyl)-6-ethoxy-8-methoxy-6H-isoindolo[2,1-a]indole-3-carboxamide was dissolved in THF (75 mL). To the solution was added a solution of 2 N HCl (300 mL). The mixture was vigorously stirred under N2 at rt for 16 h. The resulting suspension was filtered and washed with cooled TBME (2 X 30 mL). the filer cake was vacuum air dried overnight to give the title compound as a yellow solid. HPLC purity, 99% ¹H NMR (DMSO-d6, 300 MHz) δ 11.65 (s, 1H), 8.16 (s, 1H), 7.76 (d, *J* = 5.9 Hz, 1H), 7.73 (d, *J* = 5.9 Hz, 1H), 7.58 (dd, *J* = 8.5 and 1.5 Hz, 1H), 7.17-7.20 (m, 2H), 7.08 (dd, *J* = 8.5 and 1.4 Hz, 1H), 6.55 (d, *J* = 8.6 Hz, 1H), 3.86 (s, 3H), 3.14-3.18 (m, 1H), 2.91 (s, 6H), 1.75-1.99 (m, 7H), 1.48-1.60 (m, 3H); m/z 484 (M + H)+.

### Intermediate 7

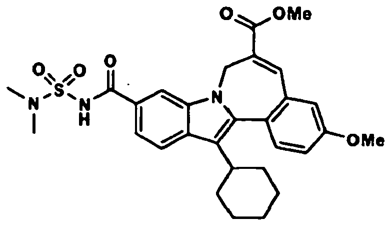

*7H-Indolo[2,1-a][2]benzazepine-6-carboxylic acid, 13-cyclohexyl-10-[[[(dimethylamino)sulfonyl]amino]carbonyl]-3-methoxy-, methyl ester.* A mixture of the 3-cyclohexyl-N-(N,N-dimethylsulfamoyl)-2-(2-formyl-4-methoxyphenyl)-1H-indole-6-carboxamide (4.8g, 0.01 mol), methyl 2-(dimethoxyphosphoryl)acrylate (9.7 g, 0.02 mol) and cesium carbonate (7.1 g, 0.02 mol) in DMF (28mL) was stirred for 20 hr at an oil bath temperature of 55°C. The mixture was poured into ice-water and acidified with dilute HCl to precipitate the crude product. The solid was collected, dried and flash chromatographed on SiO₂ (110g) using an ethyl acetate and methylene chloride (1:10) solution containing 2% acetic acid. Homogeneous fractions were combined and evaporated to afford the title compound as a pale yellow solid (3.9g, 71 % yield). MS: 552 (M=H+).

An alternate procedure for the preparation of 7H-indolo[2,1-a][2]benzazepine-6-carboxylic acid, 13-cyclohexyl-10-[[[(dimethylamino)sulfonyl]amino]carbonyl]-3-methoxy-, methyl ester is provided below.

A solution of 11-cyclohexyl-N-[(dimethylamino)sulfonyl]-6-hydroxy-8-methoxy-6H-isoindolo[2,1-a]indole-3-carboxamide (cyclic hemiaminal) (63.0 g, 130 mmol), methyl 2-(dimethoxyphosphoryl)acrylate (60 g, 261 mmol), cesium carbonate (106 g, 326 mmol) in DMF (400 mL) was heated at 60°C (bath temp) for 4.5h. Additional methyl 2-(dimethoxyphosphoryl)acrylate (15 g, 65 mmol) and cesium carbonate (21.2 g, 65 mmol) were added and the reaction was heated at 60°C overnight then and cooled to rt. The stirring reaction mixture was diluted with H₂O (1 L), slowly neutralized with 1N aqueous HCl (800 mL), stirred 3h, and then the precipitates were collected by filtration. The solids were triturated with Et2O (800 mL) and dried to yield methyl 7H-indolo[2,1-a][2]benzazepine-6-carboxylic acid, 13-cyclohexyl-10-[[[(dimethylamino)sulfonyl]amino]carbonyl]-3-methoxy-, methyl ester (70.2 g, 127 mmol, 98%) as a yellow solid which was used without further purification. 1HNMR (300 MHz, CDCl3) δ 8.67 (s, 1H), 8.09 (s, 1H), 7.86 (d, J = 8.4 Hz, 1H), 7.80 (s, 1H), 7.50 (d, J = 8.4 Hz, 1H), 7.42 (d, J = 8.8 Hz, 1H), 7.08 (dd, J = 2.6, 8.8 Hz, 1H), 6.98 (d, J = 2.6 Hz, 1H), 5.75 - 5.51 (m, 1H), 4.29 - 4.01 (m, 1H), 3.89 (s, 3H), 3.82 (s, 3H), 3.05 (s, 6H), 2.87 - 2.73 (m, 1H), 2.11 - 1.12 (m, 1OH). LCMS: m/e 550 (M-H)-, ret time 3.21 min, column A, 4 minute gradient.

### Intermediate 8

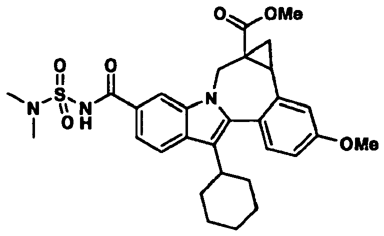

*Cycloprop[d]indolo[2,1-a][2]benzazepine-1a(2H)-carboxylic acid, 8-cyclohexyl-5-[[[(dimethylamino)sulfonyl]amino]carbonyl]-1,12b-dihydro-1-methoxy-, methyl ester, (*+/-*)*-. DMSO (5 mL) was added to a mixture of trimethylsulfoxonium iodide (199 mg, 0.906 mmol) and NaH (38 mg in 60% oil dispersion, 0.953 mmol) in a round-bottomed flask The reaction mixture was stirred at rt for 0.5 hr. 7H-Indolo[2,1-a][2]benzazepine-6-carboxylic acid, 13-cyclohexyl-10-[[[(dimethylamino)sulfonyl]amino]carbonyl]-3-(methoxy)-, methyl ester (125 mg, 0.227 mmol) was then added and the reaction mixture was stirred at rt. for 3 hr., and then at 50°C for a further 3 hr. The reaction was then quenched with water and acidified with 1N HCl solution. The crude product then precipitated as a light yellow solid which was collected by filtration and air dried, (106 mg, 83% yield). 6 mg of this material was then purified by Prep. HPLC to afford the title compound as a light yellow solid (1.8 mg). MS m/z 566(MH⁺), Retention time: 3.850 min.1H NMR (500 MHz, MeOD) δ ppm 0.28 (m, 0.36 H) 1.19 - 2.20 (m, 11.64 H) 2.70 - 3.02 (m, 2 H) 3.03 (s, 2.16 H) 3.05 (s, 3.84 H) 3.49 (d, J= 15.26 Hz, 0.64 H) 3.54 (s, 1.92 H) 3.83 (s, 1.08 H) 3.91 (s, 3 H) 4.08 (d, J=15.26 Hz, 0.36 H) 5.29 (d, J=15.26 Hz, 0.36 H) 5.50 (d, J=14.95 Hz, 0.64 H) 6.98 - 7.06 (m, 1 H) 7.16 (d, J=2.44 Hz, 0.36 H) 7.23 (d, J=2.44 Hz, 0.64 H) 7.30 (d, J=8.55 Hz, 0.64 H) 7.34 (d, J=8.55 Hz, 0.36 H) 7.56 (dd, J=8.55, 1.53 Hz, 0.64 H) 7.63 (dd, J=8.55, 1.53 Hz, 0.36 H) 7.88 (d, J=8.55 Hz, 0.64 H) 7.91 (d, J=8.55 Hz, 0.36 H) 8.12 (s, 0.36 H) 8.33 (d, J=1.53 Hz, 0.64 H).

An alternative procedure for the preparation of the title compounds is provided below.

To a flame dried, four necked, 1 L round bottom flask equipped with a mechanical stirrer, N2 inlet and a thermometer, under N2, was charged sodium hydride (95%) (3.09 g, 129.2 mmol) and dry DMF (200 mL). With vigorous stirring, trimethylsulfoxonium iodide (32.5 g, 147.3 mmol) portion wise during which time the temperature rose to 30 °C. After stirring for 30 mins, a solution of 7H-Indolo[2,1-a][2]benzazepine-6-carboxylic acid, 13-cyclohexyl-10-[[[(dimethylamino)sulfonyl]amino]carbonyl]-3-(methoxy)-, methyl ester (33.8 g, 61.3 mmol) in dry DMF (70 mL) was added quickly. The reaction mixture was stirred below 30°C for 30 mins and then poured into an ice cold solution of 1 N HCl (130 mL) in H2O (2 L) portion wise. After the resulting suspension was mechanically stirred for I h, the precipitates were filtered and the filter cake was washed with H2O (100 mL). The filter cake was partitioned between EtOAc and 0.5 N HCl (1:1, 4 L). The organic phase was separated, washed with H2O (1 L) and brine (1 L), dried over MgSO₄, filtered and concentrated in vacuo. The residue was dissolved in EtOAc (150 mL), and the solution was filtered through a silica gel pad (300 g in hexane) and rinsed with 50% EtOAc in hexane (5 L). The filtrate was concentrated in vacuo to give a slightly yellow solid which was triturated with 10% EtOAc in TBME (220 mL) from 50°C to 0 °C to to give cycloprop[d]indolo[2,1-a][2]benzazepine-1a(2H)-carboxylic acid, 8-cyclohcxyl-5-[[[(dimethylamino)sulfonyl]amino]carbonyl]-1,12b-dihydro-11-methoxy-, methyl ester, (+/-)- as a white solid (26.1 g, 75% yield). HPLC purity, 100%. ¹H NMR (DMSO-d₆, 300 MHz) δ 11.61 (s, 1H), 8.47 (s, 0.5H), 8.25 (s, 0.5H), 7.81-7.88 (m, 1H), 7.57-7.63 (m, 1H), 7.23-7.29 (m, 2H), 7.01-7.07 (m, 1H), 5.43 (d, J = 15.0 Hz, 0.5H), 5.22 (d, J = 15 Hz, 0.5H), 4.04 (dd, J = 15.4 and 6.6 Hz, 0.5H), 3.83 (s, 3H), 3.75 (s, 1H), 3.08-3.47 (m, 0.5H), 3.29 (s, 3H), 2.73-2.92 (m, 8H), 1.11-1.99 (m, 10.5Hz), 0.20 (m, 0.5H); m/z 566 (M + H)⁺.

### Intermediate 9

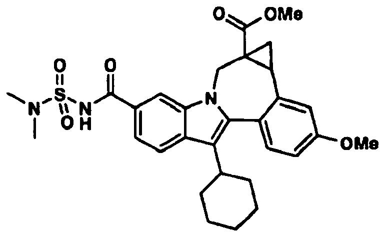

*Cycloprop[d]indolo[2,1-a][2]benzazepine-1a(2H)-carboxylic acid, 8-cyclohexyl-5-[[[(dimethylamino)sulfonyl]amino]carbonyl]-1,12b-dihydro-11-methoxy-, methyl ester, (-)-*. A sample of (+/-) cycloprop[d]indolo[2,1-a][2]benzazepine-1a(2H)-carboxylic acid, 8-cyclohexyl-5-[[[(dimethylamino)sulfonyl]amino]carbonyl]-1,12b-dihydro-11-methoxy-methyl ester was dissolved in EtOH/CH₃CN 1/1 + 0.5% DEA at a concentration of 50 mg/ml. [The addition of DEA ensures the compound remains in solution during the injection process]. This solution was then injected onto a Thar SFC-350 preparative SFC under the conditions shown below.

*Preparative conditions on Thar SFC-350*: Column: Chiralcel OJ-H 5x25 cm; mobile phase: 25% MeOH/CH3CN (1/1) in CO2; pressure (bar): 100; flow rate (ml/ min): 240; solution concentration (mg/ml): 50; injection amount (ml): 4.5-5; Cycle time (min/inj): 6.5-7; Temperature (°C): 45; throughput (g/ hr): ∼2; Detector wavelength (nm): 254.

From 371.4 g of racemic starting material, a total of 177.3g of the desired second eluting (-) isomer was obtained, containing ∼1 Meq of diethylamine. This material was purified using the following procedure. The mixture (24.7 g) dissolved in dichloromethane (800 mL)) was washed sequentially with; 0.5 N HCl (1 x 400 mL, 1 x 240 mL), H₂O (2 x 240 mL), and brine (2 x 240 mL). The organic layer was then dried (Anhy. Na₂SO₄), filtered and evaporated to give 22.33 g of (cycloprop[d]indolo[2,1-a][2]benzazepine-1a(2H)-carboxylic acid, 8-cyclohexyl-5-[[[(dimethylamino)sulfonyl]amino]carbonyl]-1,12b-dihydro-11-methoxy-, methyl ester, (-)- as a yellow solid (92% recovery). HPLC¹ > 99% (Rt 2.38 min); LC/MS (ES⁺) 566.51 (M+H, 100); [α]_{D}^{25C}- 194.64 °(c 1.03, MeOH). Anal. Calcd for C₃₀H₃₅N₃O₆S•0.33H₂O: C, 63.04; H, 6.29; N, 7.35; S, 5.61; H₂O, 1.04. Found: C, 63.07; H, 6.01; N, 7.24; S, 5.58; H₂O, 1.03. The NMR shows the absence of Et₂NH. The EE of this material was determined to be > 99% using the following analytical HPLC procedure.

*Analytical conditions of ee determination on Thar analytical SFC.* Analytical Column: Chiralcel OJ (.46x25cm, 10µl); BPR pressure: 100 bars; Temperature: 35°C; Flow rate: 3.0 ml/min; Mobile Phase: 15% MeOH/CH₃CN (1/1) in CO₂; Detector Wavelength: 254 nm; Retention time (min): 4, 6.5.

### Intermediate 10

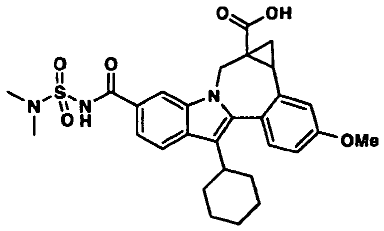

*Cycloprop[d]indolo[2,1-a][2]benzazepine-1a(2H)-carboxylic acid, 8-cyclohexyl-5-[[[(dimethylamino)sulfonyl]amino]carbony*/*]-1,12b-dihydro-11-methoxy-, (-)-*. To a solution of (-) cycloprop[d]indolo[2,1-a][2]benzazepine-1a(2H)-carboxylic acid, 8-cyclohexyl-5-[[[(dimethylamino)sulfonyl]amino]carbonyl]-1,12b-dihydro-11-methoxy-, methyl ester (22.33 g, 39.5 mmol) in MeOH (300 mL) was added 1 N NaOH (120 mL) slowly over 20 min., while maintaining the reaction temperature < 30°C. The mixture was stirred at rt under N₂ for 18 h. The HPLC indicated the reaction was complete. To the reaction solution was added 1 N HCl (130 mL). After addition was complete, the pH of the reaction mixture was about 2. The methanol in the reaction mixture was evaporated. Water (300 mL) was added to the mixture which was then extracted with CH₂Cl₂ (1 x 600 mL, 1 x 200 mL). The combined extracts were washed with H₂O (2 x 300 mL), brine (2 x 300 mL), dried (Na₂SO₄) and evaporated to give 20.82 g (96% yield) of the title compound as a yellow solid. HPLC conditions column: Phenomenoex Synergi Polar-RP 4 um 4.6 x 50 mm; UV: 220 nm; gradient time: 4 min; flow rate: 4 mL/min, 75 - 100% B; solvent A: 10% MeOH/90% H₂O with 0.2% H₃PO₄, solvent B: 90% MeOH/10% H₂O with 0.2% H₃PO₄. HPLC > 99% (Rt 1.80 min.) LC/MS (ES⁺) 552.25 (M+H, 100); [α]_{D}^{25C} - 166.99 °(c 1.00, MeOH). GC analysis: CH₂Cl₂ 4.94%; Anal. Calcd for C₂₉H₃₃N₃O₆S•0.16 H₂O•0.35 CH₂Cl₂: C, 60.37; H, 5.87; N, 7.20; S, 5.49; H₂O, 0.49; CH₂Cl₂, 5.02. Found: C, 59.95; H, 5.89; N, 7.03; S, 5.38; H₂O, 0.47; CH₂Cl₂, 4.94.

### Intermediate 11

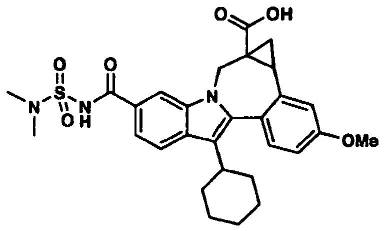

*Cycloprop[d]indolo[2,1-a][2]benzazepine-1a(2H)-carboxylic acid, 8-cyclohexyl-5-[[[(dimethylamino)sulfonyl]amino]carbonyl]-1,12b-dihydro-11-methoxy-,* (+/-)-. To a solution of (+/-) cycloprop[d]indolo[2,1-a][2]benzazepine-1a(2H)-carboxylic acid, 8-cyclohexyl-5-[[[(dimethylamino)sulfonyl]amino]carbonyl]-1,12b-dihydro-11-methoxy-, methyl ester (100 mg, 0.177 mmol) in THF/Methanol mixture (2.0 mL/2.0 mL), 2N NaOH solution (1.0 mL) was added. The reaction mixture was heated at 90°C under microwave conditions for 5 min. It was then concentrated, acidified with 1N HCl solution and extracted with ethyl acetate (2X20 mL). The organic layers were combined, dried (MgSO₄), filtered and concentrated. The residue was purified by preparative HPLC to afford the desired product as a light yellow solid, (59 mg, 60% yield). MS m/z 552(MH⁺), Retention time: 3.850 min.1H NMR (300 MHz, MeOD) δ ppm 0.25 (m, 0.38 H) 1.14 - 2.22 (m, 11.62 H) 2.69 - 2.98 (m, 2 H) 3.02 (s, 2.28 H) 3.02 (s, 3.72 H) 3.41 (d, J=15.00 Hz, 0.62 H) 3.88 (s, 3 H) 4.01 (d, J=15.00 Hz, 0.38 H) 5.26 (d, J=15.00 Hz, 0.38 H) 5.45 (d, J=14.64 Hz, 0.62 H) 6.94 - 7.02 (m, 1 H) 7.13 (d, J=2.56 Hz, 0.38 H) 7.21 (d, J=2.20 Hz, 0.62 H) 7.26 (d, J=8.42 Hz, 0.62 H) 7.30 (d, J=8.78 Hz, 0.38 H) 7.53 (dd, J=8.42, 1.46 Hz, 0.62 H) 7.61 (dd, J=8.60, 1.65 Hz, 0.38 H) 7.85 (d, J=8.42 Hz, 0.62 H) 7.89 (d, J=8.42 Hz, 0.38 H) 8.10 (s, 0.38 H) 8.28 (d, J=1.46 Hz, 0.62 H).

### Intermediate 12

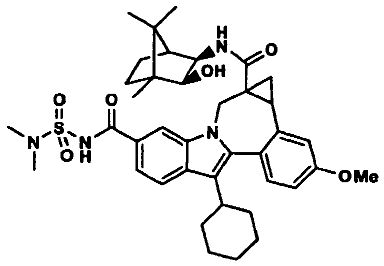

*Cycloprop[d]indolo[2,1-a][2]benzazepine-1a.5(2H)-dicarboxamide, 8-cyclohexyl-N⁵-[(dimethylamino)sulfonyl]-1,12b-dihydro-N^{1a}-[(2R,3S)-3-hydroxy-4,7,7trimethylbicyclo[2.2.1]hept-2-yl]-11-methoxy-, (1aR)-[partial]-.* TBTU (437 mg, 1.36 mmol) and DIPEA (0.95 mL, 5.436 mmol) were added to a solution of (+/-) cycloprop[d]indolo[2,1-a][2]benzazepine-1a(2H)-carboxylic acid, 8-cyclohexyl-5-[[[(dimethylamino)sulfonyl]amino]carbonyl]-1,12b-dihydro-11-methoxy-(500 mg, 0.906 mmol) in DMSO (20.0 mL). The reaction mixture was stirred at rt for 15 min. (2S,3R)-3-Amino-1,7,7-trimethylbicyclo[2.2.1]heptan-2-ol (280 mg, 1.36mmol) was then added and the reaction mixture was stirred at rt overnight. The reaction mixture was quenched with water and acidified with 1N HCl solution. A brown solid separated which was collected by filtration. This material was then fractionated by Preparative HPLC under the following conditions. Column: Waters Sunfire 19mm x 100mm; Solvent A: 10% CH3CN-90% H2O-0.1% TFA; Solvent B: 90% CH3CN-10% H2O-0.1% TFA; Program: Start with 65% solvent B, initial hold time for 5 min, then gradually increase to 90% solvent B in 30 min with flow rate 25 mL/min. Load: 50-60 mg/run.

Cycloprop[d]indolo[2,1-a][2]benzazepine-1a,5(2H)-dicarboxamide, 8-cyclohexyl-N⁵-[(dimethylamino)sulfonyl]-1,12b-dihydro-N^{1a}-[(2R,3S)-3-hydroxy-4,7,7-trimethylbicyclo[2.2.1]hept-2-yl]-11-methoxy-, (1aR)- [partial*]*-elutes before Cycloprop[d]indolo[2,1-a][2]benzazepine-1a,5(2H)-dicarboxamide, 8-cyclohexyl-N⁵-[(dimethylamino)sulfonyl]-1,12b-dihydro-N^{1a}-[(2R,3S)-3-hydroxy-4,7,7-trimethylbicyclo[2.2.1]hept-2-yl]-11-methoxy-, (1aS)- *[partial]-*under the HPLC conditions described above. Product obtained as a light yellow solid, 230 mg, 36% yield). MS m/ 703(MH⁺), Retention time: 3.936 min. 1H NMR (500 MHz, MeOD) δ ppm 0.14 - 0.24 (m, 2.64 H) 0.51 (s, 2.46 H) 0.72 - 2.21 (m, 20.9 H) 2.49 (m, 0.18 H) 2.62 (m, 0.82 H) 2.85 (m, 0.18 H) 2.96 (m, 0.82 H) 3.03 (s, 6 H) 3.39 (m, 0.82 H) 3.49 - 3.58 (m, 1.64 H) 3.71 - 3.80 (m, 0.36 H) 3.90 (s, 3 H) 4.17 (d, *J*=14.65 Hz, 0.18 H) 5.06 (d, *J*=14.65 Hz, 0.18 H) 5.37 (d, *J*=14.95 Hz, 0.82 H) 6.73 (d, *J*=5.49 Hz, 0.82 H) 6.98 - 7.05 (m, 1 H) 7.08 (d, *J*=4.58 Hz, 0.18 H) 7.10 (d, *J*=2.44 Hz, 0.18 H) 7.21 (d, *J*=2.44 Hz, 0.82 H) 7.31 (d, *J*=8.55 Hz, 0.82 H) 7.34 (d, *J*=8.55 Hz, 0.18 H) 7.59 - 7.64 (m, 1 H) 7.87 - 7.93 (m, 1 H) 7.99 (s, 0.18 H) 8.09 (d, *J*=1.22 Hz, 0.82 H).

### Intermediate 13

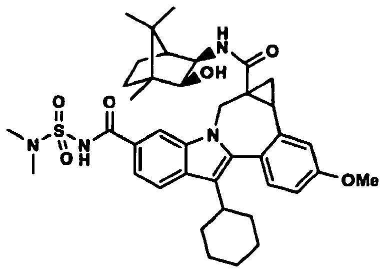

*Cycloprop[d]indolo[2,1-a][2]benzazepine-1a,5(2H)-dicarboxamide, 8-cyclohexyl-N⁵-[(dimethylamino)sulfonyl]-1,12b-dihydro-N^{1a}-[(2R,3S)-3-hydroxy-4,7,7-trimethylbicyclo[2.2.1]hept-2-yl]-11-methoxy-, (1aS)- [partial]-.* TBTU (437 mg, 1.36 mmol) and DIPEA (0.95 mL, 5.436 mmol) were added to a solution of (+/-) cycloprop[d]indolo[2,1-a][2]benzazepine-1a(2H)-carboxylic acid, 8-cyclohexyl-5-[[[(dimethylamino)sulfonyl]amino]carbonyl]-1,12b-dihydro-11-methoxy-(500 mg, 0.906 mmol) in DMSO (20.0 mL). The reaction mixture was stirred at rt for 15 min. Then (2S,3R)-3-amino-1,7,7-trimethylbicyclo[2.2.1]heptan-2-ol (280 mg, 1.36mmol) was added, and the reaction mixture was stirred at rt overnight. The reaction mixture was quenched with water and then acidified with 1N HCl solution. A brown colored solid separated that was collected by filtration. This material was then fractionated by preparative HPLC under the following conditions. Column: Waters Sunfire 19mm x 100mm; Solvent A: 10% CH3CN-90% H2O-0.1% TFA; Solvent B: 90% CH3CN-10% H2O-0.1% TFA; Program: Start with 65% solvent B, initial hold time for 5 min, then gradually increase to 90% solvent B in 30 min with flow rate 25 mL/min. Load: 50-60 mg/run.

Cycloprop[d]indolo[2,1-a][2]benzazepine-1a,5(2H)-dicarboxamide, 8-cyclohexyl-N⁵-[(dimethylamino)sulfonyl]-1,12b-dihydro-N^{1a}-[(2R,3S)-3-hydroxy-4,7,7-trimethylbicyclo[2.2.1]hept-2-yl]-11-methoxy-, (1aS)- [partial]- elutes after cycloprop[d]indolo[2,1-a][2]benzazepine-1a,5(2H)-dicarboxamide, 8-cyclohexyl-N⁵-[(dimethylamino)sulfonyl]-1,12b-dihydro-N^{1a}-[(2R,3S)-3-hydroxy-4,7,7-trimethylbicyclo[2.2.1]hept-2-yl]-11-methoxy-, (1aR)- [partial]- under the HPLC conditions described above. Product obtained as a light yellow solid, 215 mg, 34% yield). MS m/ 703(MH⁺), Retention time: 4.038 min. 1H NMR (500 MHz, MeOD) δ ppm 0.20 (m, 0.38 H) 0.75 (s, 1.86 H) 0.76 (s, 1.86 H) 0.84 (s, 1.86 H) 0.85 (s, 1.14 H) 0.89 - 2.18 (m, 18.9 H) 2.52 (m, 0.38 H) 2.70 (m, 0.62 H) 2.85 (m, 0.38 H) 2.97 (m, 0.62 H) 3.03 (s, 2.28 H) 3.04 (s, 3.72 H) 3.33 - 3.39 (m, 0.62 H) 3.43 - 3.51 (m, 1.24 H) 3.73 - 3.77 (m, 0.38 H) 3.78 - 3.84 (m, 0.38 H) 3.90 (s, 1.86 H) 3.90 (s, 1.14 H) 4.14 (d, J=14.65 Hz, 0.38 H) 5.11 (d, J=14.65 Hz, 0.38 H) 5.44 (d, J=15.26 Hz, 0.62 H) 6.68 (d, J=4.88 Hz, 0.62 H) 6.96 - 7.03 (m, 1 H) 7.07 (d, J=5.19 Hz, 0.38 H) 7.12 (d, J=2.44 Hz, 0.38 H) 7.23 (d, J=2.14 Hz, 0.62 H) 7.27 (d, J=8.54 Hz, 0.62 H) 7.33 (d, J=8.54 Hz, 0.38 H) 7.55 (dd, J=8.39, 1.68 Hz, 0.62 H) 7.62 (dd, J=8.55, 1.53 Hz, 0.38 H) 7.87 (d, J=8.54 Hz, 0.62 H) 7.91 (d, J=8.55 Hz, 0.38 H) 8.08 (d, J=1.22 Hz, 0.38 H) 8.10 (d, J=1.22 Hz, 0.62 H).

### Intermediate 14

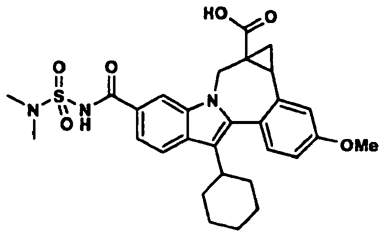

*Cycloprop[d]indolo[2,1-a][2]benzazepine-1a(2H)-carboxylic acid, 8-cyclohexyl-5-[[[(dimethylamino)sulfonyl]amino]carbonyl]-1,12b-dihydro-11-methoxy-, (-)-.* 10 N NaOH (2.0 mL, 20 mmol) solution and 4 mL ofwater were added to a solution of cycloprop[d]indolo[2,1-a][2]benzazepine-1a,5(2H)-dicarboxamide, 8-cyclohexyl-N⁵-[(dimethylamino)sulfonyl]-1,12b-dihydro-N^{1a}-[(2R,3S)-3-hydroxy-4,7,7-trimethylbicyclo[2.2.1]hept-2-yl]-11-methoxy-, (1aR)-[partial]- (160 mg, 0.228 mmol) in THF/MeOH (7 mL/7 mL). The reaction mixture was heated at 120°C under microwave conditions for 1 hr. It was then concentrated, acidified with conc. HCl solution and extracted with ethyl acetate twice (2X30 mL). The organic layers were combined, dried (MgSO₄) filtered and concentrated in vacuo to an orange oil. The crude product was then purified by Prep. HPLC column to afford the product a light yellow solid, (80 mg, 64% yield). Average specific rotation -130.85°; Solvent MeOH; Wavelength 589 nm; 50 cm cell. MS m/ 552(MH⁺), Retention time: 3.760 min. 1H NMR (500 MHz, MeOD) δ ppm 0.27 (m, 0.38 H) 1.14 - 2.22 (m, 11.62 H) 2.76 (m, 0.38 H) 2.80 - 2.92 (m, 1 H) 2.92 - 3.09 (m, 6.62 H) 3.45 (d, J=14.95 Hz, 0.62 H) 3.90 (s, 1.86 H) 3.91 (s, 1.14 H) 4.04 (d, J=15.26 Hz, 0.38 H) 5.28 (d, J=15.26 Hz, 0.38 H) 5.47 (d, J=15.26 Hz, 0.62 H) 6.95 - 7.05 (m, 1 H) 7.15 (d, J=2.75 Hz, 0.38 H) 7.23 (d, J=1.83 Hz, 0.62 H) 7.28 (d, J=8.55 Hz, 0.62 H) 7.33 (d, J=8.54 Hz, 0.38 H) 7.54 (dd, J=8.39, 1.68 Hz, 0.62 H) 7.63 (dd, J=8.55, 1.53 Hz, 0.38 H) 7.86 (d, J=8.55 Hz, 0.62 H) 7.91 (d, J=8.55 Hz, 0.38 H) 8.11 (d, J=1.22 Hz, 0.62 H) 8.29 (d, J=1.22 Hz, 0.38 H).

### Intermediate 15

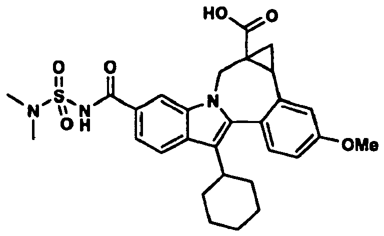

*Cycloprop[d]indolo[2,1-a][2]benzazepine-1a(2H)-carboxylic acid, 8-cyclohexy*/*-5-[[[(dimethylamino)sulfony*/*]amino]carbonyl]-1,12b-dihydro-11-methoxy-, (+)-*. 10 N NaOH (1.8 mL, 18 mmol) solution and 4 mL of water were added to a solution of cycloprop[d]indolo[2,1-a][2]benzazepine-1a,5(2H)-dicarboxamide, 8-cyclohexyl-N⁵-[(dimethylamino)sulfonyl]-1,12b-dihydro-N^{1a}-[(2R,3S)-3-hydroxy-4,7,7-trimethylbicyclo[2.2.1]hept-2-yl]-11-methoxy-, (1aS)-[partial]- (130 mg, 0.185 mmol) in bTHF/MeOH (7 mL/7 mL). The reaction mixture was heated at 120°C under microwave conditions for 1 hr. It was concentrated, acidified with conc. HCl solution and extracted with ethyl acetate twice (2X30 mL). The organic layers were combined, dried (MgSO₄) filtered and concentrated in vacuo to give an orange oil. The crude product was then purified by Prep. HPLC column to afford the product as a light yellow solid, (68 mg, 67% yield). Average specific rotation + 174.73°; Solvent MeOH; Wavelength 589 nm; 50 cm cell MS m/ 552(MH⁺), Retention time: 3.773 min. 1H NMR (500 MHz, MeOD) δ ppm 0.27 (m, 0.38 H) 1.14 - 2.22 (m, 11.62 H) 2.76 (m, 0.38 H) 2.80 - 2.92 (m, 1H) 2.92 - 3.09 (m, 6.62 H) 3.45 (d, J=14.95 Hz, 0.62 H) 3.90 (s, 1.86 H) 3.91 (s, 1.14 H) 4.04 (d, J=15.26 Hz, 0.38 H) 5.28 (d, J=15.26 Hz, 0.38 H) 5.47 (d, J=15.26 Hz, 0.62 H) 6.95 - 7.05 (m, 1 H) 7.15 (d, J=2.75 Hz, 0.38 H) 7.23 (d, J=1.83 Hz, 0.62 H) 7.28 (d, J=8.55 Hz, 0.62 H) 7.33 (d, J=8.54 Hz, 0.38 H) 7.54 (dd, J=8.39, 1.68 Hz, 0.62 H) 7.63 (dd, J=8.55, 1.53 Hz, 0.38 H) 7.86 (d, J=8.55 Hz, 0.62 H) 7.91 (d, J=8.55 Hz, 0.38 H) 8.11 (d, J=1.22 Hz, 0.62 H) 8.29 (d, J=1.22 Hz, 0.38 H).

### Intermediate 16

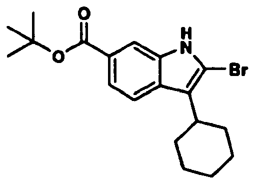

*1H-Indole-6-carboxylic acid, 2-bromo-3-cyclohexyl-, 1,1-dimethylethyl ester.* To a mechanically stirred solution of 2-bromo-3-cyclohexyl-1H-indole-6-carboxylic acid (80 g, 0.24 m) in dry methylene dichloride(1.2 L) and THF (100 mL) were added activated molecular sieves (4A, 80 g) and silver carbonate (275 g, 0.99 m). The reaction mixture was cooled to 0°C and t-Butyl bromide (142 g, 1.04 m) was added drop wise. The mixture was stirred overnight at rt and monitored by TLC (Hexane-Ethyl acetate 80:20, R_{f} (Product) = 0.7). If any bromo acid was left unconverted a further 10% of silver carbonate was added and stirring was continued for an addition 2 - 4 h. On completion, the reaction mixture was filtered through a thin bed of celite. The filtrand was washed with methylene dichloride (500 mL). The combined filtrates were concentrated in-vacuo, and the crude product thus obtained was purified by silica gel chromatography: (230 - 400 mesh, clutcd with a gradient of ethyl acetate in pet ether 0 - 2%). Homogeneous fractions were combined and evaporated under reduced pressure to give 80 g (85%) of the title compound. HPLC : 90.1% (RT = 6.56 min), Column : C18 BDS, (50X4.6mm), Mobile Phase : Gradient of 0.1% TFA in water : ACN (30 → 100 → 30), Flow rate 0.8 mL / min. LCMS : 99.8% (RT = 4.44 min), Column : Geneis, C18 50X4.6 mm Mobile Phase : Gradient of 0.1% Formic acid in water : ACN (70 → 95 → 70), Flow rate : 0.8 mL / min; M - 1 = 376.5; ¹H NMR CDCl₃) (400 MHz) δ 1.37 - 1.40 (m, 3H, cyc.Hexyl), 1.62 (s, 9H, t-Bu), 1.80 -1.94 (two sets of m, 3H, & 4H respectively, cyc.Hexyl part), 2.81 (m, 1H, CH of cyc.Hexyl - benzylic), 7.70 - 7.75 (m, 2H, Indole-H_{4&5}), 8.04 (s, 1H, Indole-H₇), 8.52 (s, 1H, Indole-NH).

### Intermediate 17

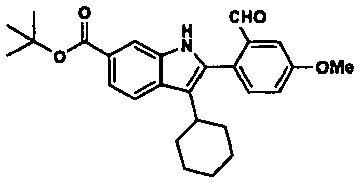

*1H-Indole-6-carboxylic acid 3-cyclohexyl-2-(2-formyl-4-methoxyphenyl)-, 1,1-dimethylethyl ester.* tert-Butyl 2-bromo-3-cyclohexyl-1H-indole-6-carboxylate (72 g, 0.19 m) was dissolved in a 1:1 mixture of toluene and ethanol (720 mL) and degasified. LiCl (23.9 g, 0.51 m) was then added, followed by sodium carbonate (720 mL, 1.0 M solution degasified separately,) and Pd-tetrakis (13.1 g, 0.011 m). After stirring for 0.25 h, 2-formyl-4-methoxyphenylboronic acid (41.1 g, 0.22 m) was added and the reaction mixture was heated to 85°C for 4 h. The reaction was then monitored by TLC, (Hexane-Ethyl acetate 80:20, R_{f} (Product) = 0.55). On completion, the reaction mixture was cooled to rt and water (1.0 L) was added followed by ethyl acetate (1.0 L). The organic layer was washed with brine, and dried and concentrated under vacuum to afford the title compound as a yellow solid. Yield 75 g (74%). HPLC : 99.7% (RT = 6.30 min), Column : C18 BDS (4.6 X 50 mm), SC-307, Mobile Phase : Gradient of 0.1% TFA in water : ACN (30 → 100 → 30), Flow rate 0.8 mL / min. LCMS : 98.0% (RT = 5.28 min), Column : Gcncis, C 18 (50X4.6 mm), Mobile Phase : Gradient of 0.1% Formic acid in water : ACN (70 → 95 → 70), Flow rate : 0.8 mL / min; M - 1 = 432.2; ¹H NMR (DMSO -d₆) (400 MHz) δ 1.40 - 1.48 (m, 3H, cyc.Hexyl), 1.57 (s, 9H, t-Bu), 1.84 - 1.90 (m, 7H, cyc.Hexyl part), 3.09 (m, 1H, CH of cyc.Hexyl - benzylic), 3.84 (s, 3H, OCH₃), 6.55 (d, J = 4 Hz, 1H, aryl H_{2'}), 7.06 (d, 1H, aryl H_{3'}), 7.08 (s, 1H, aryl H_{6'}), 7.23 (d, 1H, Indole-H₅), 7.53 (d, J = 8 Hz, 1H, Indole-H₄), 7.70 - 7.75 (m, 2H, NH + Indole-H₇), 8.06 (s, 1H, CHO).

### Intermediate 18

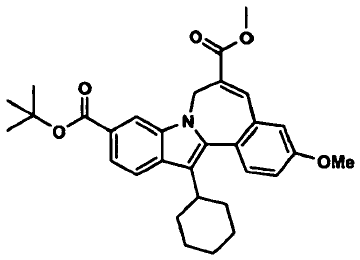

*7H-Indolo[2,1-a][2]benzazepine-6,10-dicarboxylic acid, 13-cyclohexyl-, 10-(1,1-dimethylethyl) 6-methyl ester.* tert-Butyl 3-cyclohexyl-2-(2-formyl-4-methoxyphenyl)-1H-indole-6-carboxylate (62.5 g, 0.144 m) was dissolved in dry DMF (1.2 L) and stirred mechanically. Cesium carbonate (84 g, 0.17 m) and methyl 2-(dimethoxyphosphoryl)acrylate (65 - 70% GC pure, 56.2 g, 0.18 m) were then added and the reaction mixture was heated to 65°C for 4h, and the reaction was monitored by TLC (Hexane-Ethyl acetate 80:20, R_{f} (Product) = 0.7). On completion, the mixture was cooled to rt, then quenched with water (1.0 L). A yellow solid precipitated, which was collected by filtration and air dried. This material was then slurried in methanol, filtered, and dried under vacuum to give the product as a yellow powder, (70 g, 90%). HPLC : 99.1% (RT = 6.45 min), Column : C18 BDS (4.6 X 50 mm), Mobile Phase : Gradient of 0.1% TFA in water : ACN (30 → 100 → 30), Flow rate 0.8 mL / min. LCMS : 100% (RT = 7.00 min), Column : Geneis, C18 (50X4.6 mm), Mobile Phase : Gradient of 0.1% Formic acid in water : ACN (70 → 95 → 70), Flow rate: 0.8 mL / min; M + 1 = 502.2; ¹H NMR (CDCl₃) (400 MHz) δ 1.10 -1.30 (m, 3H, cyc.Hexyl), 1.64 (s, 9H, t-Bu), 1.77 - 2.07 (m, 7H, cyc.Hexyl part), 2.80 (m, 1H, CH of cyc.Hexyl - benzylic), 3.84 (s, 3H, OCH₃), 3.93 (s, 3H, COOCH₃), 4.15 & 5.65 (two br. peak., 1H each, allylic CH₂), 6.95 (s, 1H, aryl H_{6'}), 7.01 (d, 1H, aryl H_{2'}), 7.53 (d, J = 8 Hz, 1H, aryl H_{3'}), 7.70 (d, J = 4 Hz, 1H, Indole-H₅), 7.84 (s + d, 2H, olefinic H + Indole-H₄), 8.24 (s, 1H, indole - H₇); ¹³C NMR (CDCl₃) (100.0 MHz) δ 166.92, 165.71, 158.96, 142.28, 136.47, 13.50, 134.61, 132.43, 132.01, 129.73, 124.78, 124.68, 120.33, 119.39, 119.04, 115.62, 115.05, 111.27, 80.27, 55.49, 52.50, 39.09, 36.81, 33.40, 28.38, 27.15, 26.28.

### Intermediate 19

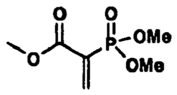

*2-Propenoic acid, 2-(dimethoxyphosphinyl)-, methyl ester.* To a 5 L four necked round bottom flask equipped with a mechanical stirrer, a condenser, a temperature controller and a N2 inlet , was charged paraformaldehyde (40.5 g, 1.35 mol), MeOH (2 L) and piperidine (2 mL). The reaction mixture was heated to reflux under N2 for 3 h. After cooling to 50°C, 2-(dimethoxyphosphoryl)acetate (150 g, 0.824 mol) was added in one portion. The reaction mixture was continued to reflux for 18 h. After cooling to rt, the reaction solution was concentrated in vacuo to give a clear colorless oil. The above oil was dissolved in dry toluene (1 L) in a 3 L four necked round bottom flask equipped a temperature controller, a N₂ inlet, a magnetic stirrer and a Dean-Stark apparatus. To the solution was added TsOH.H₂O (5.2 g). The reaction mixture was then refluxed azeotropically to remove methanol for 18 h. After cooling to rt, the solution was concentrated in vacuo to give a yellow oil which was vacuum distilled at 150-155°C/0.2 mmHg to afford the product as a colorless oil (135.0 g). Purity, 90% based on 1H NMR ¹H NMR (CDCl3, 300 MHz) 8 7.0 (dd, *J=* 42.4 and 1.5 Hz, 1H), 6.73 (dd, *J =* 20.5 and 1.8 Hz, 1H), 3.80 (s, 6H), 3.76 (s, 3H).

### Intermediate 20

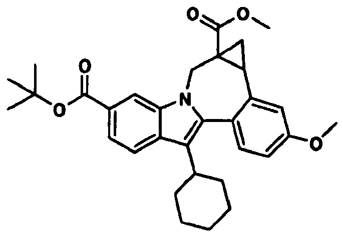

*Cycloprop[d]indolo[2,1-a][2]benzazepine-1a,5(2H)-dicarboxylic acid, 8-cyclohexyl-1,12b-dihydro-11-methoxy-, 5-(1,1-dimethylethyl) 1a-methyl ester, (+*/*-)*. Sodium hydride (96 mg, 4 mmol) was added to a stirred suspension of trimethylsulfoxonium chloride (567 mg, 4.4 mmol) in anhydrous DMSO (10 mL) under nitrogen. The resultant mixture was stirred at rt for 30-45 min and then neat 7H-indolo[2,1-a][2]benzazepine-6,10-dicarboxylic acid, 13-cyclohexyl-3-methoxy-, 10-(1,1-dimethylethyl) 6-methyl ester (1.0, 2 mmol) was added in small portions. The suspension was diluted with DMSO (5 mL) and heated at 50 °C for 3-4 h. The reaction mixture was allowed to cool to rt and water was added. A solid separated, which was collected by filtration and washed with water and then air dried overnight to afford 1.15 g of crude product. This material was purified by flash column chromatography (silica gel, 3% MeOH in DCM) to provide pure title compound (0.96 g): LC/MS: Retention time 3.816 min; m/e 516 (MH⁺). ¹H NMR (400 MHz, CDCl₃): The product was observed to exist as inter-converting rotamers, as evidenced from the compound's NMR spectrum.

The following procedure is an example of a method to effect the resolution of racemic cycloprop[d]indolo[2,1-a][2]benzazepine-1a,5(2H)-dicarboxylic acid, 8-cyclohexyl-1,12b-dihydro-11-methoxy-, 5-(1,1-dimethylethyl) la-methyl ester, (+/-). A sample of cycloprop[d]indolo[2,1-a][2]benzazepine-1a,5(2H)-dicarboxylic acid, 8-cyclohexyl-1,12b-dihydro-11-methoxy-, 5-(1,1-dimethylethyl) 1a-methyl ester, (+/-)-was dissolved in a mixture of isopropanol and acetonitrile (8:2) to give a final concentration of 20mg/mL. This mixture was injected on a preparative chiral SFC chromatography system using the following conditions: Chiralcel OJ-H column, 4.6 x 250mm, 5µm; Mobile Phase: 8% MeOH in CO₂; Temp: 35 °C; Flow rate: 2 mL/min for 16 min; UV monitored @ 260nm; Injection: 5µL of ∼20.0mg/mL in IPA:ACN (8:2).

### Intermediate 21

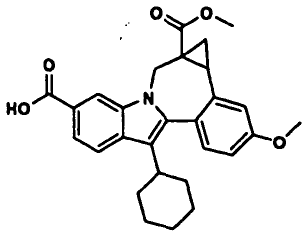

*Cycloprop[d]indolo[2,1-a][2]benzazepine-1a,5(2H)-dicarboxylic acid, 8-cyclohexyl-1,12b-dihydro-11-methoxy-, 1a-methyl ester, (+*/*-)-*. TFA (5 mL) was added to a solution of (+/-) 8-Cyclohexyl-1,1a,2,12b-tetrahydro-11-methoxy-1a-(methoxycarbonyl)-cycloprop[d]indolo[2,1-a][2]benzazepine-5-carboxylic acid, tert-butyl ester (515 mg, 1 mmol) in anhydrous DCM (10 mL). The resultant solution was stirred at rt for approximately 8 to 12 hr. The reaction was then evaporated to dryness to afford the title compound (0.47g, 100%). LC/MS: Retention time 2.245 min; m/e 460 (MH⁺). ¹H NMR (400 MHz, CDCl₃): From the compounds NMR spectrum, the product was observed to exist as a mixture of interconverting rotamers.

### Intermediate 22

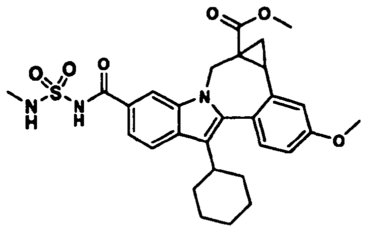

*Cycloprop[d]indolo[2,1-a][2]benzazepine-1a(2H)-carboxylic acid, 8-cyclohexyl-1,12b-dihydro-11-methoxy-5-[[[(methylamino)sulfonyl]amino]carbonyl]-, methyl ester.* A solution of 8-Cyclohexyl-1,1a,2,12b-tetrahydro-11-methoxy-1a-(methoxycarbonyl)-cycloprop[d]indolo[2,1-a][2]benzazepine-5-carboxylic acid (140 mg, 0.31 mmol) and CDI (64 mg, 0.40 mmol) in THF (3 mL) was stirred for 1 hr at 60 °C. N-methylsulfamide (68 mg, 0.62 mmol) and DBU (71.6 mg, 0.47 mmol) were added and the mixture was stirred at 60 °C overnight. The reaction was then poured into cold water, acidified with dilute hydrochloric acid and extracted into ethyl acetate. The extracts were washed sequentially with dilute hydrochloric acid (0.1 N), and brine, and then dried (anhy. sodium sulfate), filtered and evaporated to provide the title compound as a brown solid. ESI-MS m/e 552 (MH⁺). This material was used without further purification.

### Intermediate 23

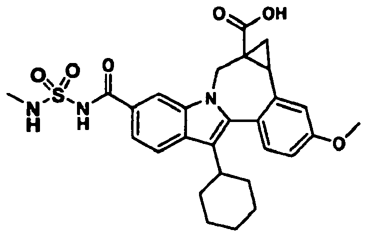

*Cycloprop[d]indolo[2,1-a][2]benzazepine-1a(2H)-carboxylic acid, 8-cyclohexyl-1,12b-dihydro-11-methoxy-5-[[[(methylamino)sulfonyl]amino]carbonyl]-.* Cycloprop[d]indolo[2,1-a][2]benzazepine-1a(2H)-carboxylic acid, 8-cyclohexyl-5-[[[(methylamino)sulfonyl]amino]carbonyl]-1,12b-dihydro-11-methoxy-, methyl ester was dissolved in THF, MeOH mixture (2 mL,2 mL). 2.5 M NaOH (aq.) (1.2 mL, 3 mmol) was then added and the reaction was shaken at 22°C for 2 hr. The solution was then neutralized with 1M HCl (aq.) (3 mL) and concentrated to remove the organic solvents. The residue was slurried with H₂O and the solids were collected by filtration, washed with H₂O and dried to yield compound the title compound (160 mg, 0.30 mmol). ESI-MS m/e 538 (MH⁺). This material was used without further purification.

### Intermediate 24

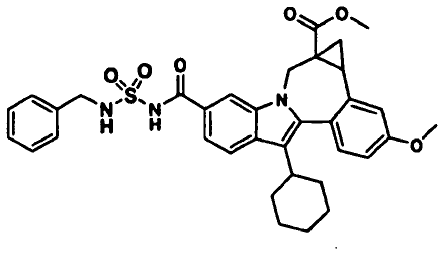

*Cycloprop[d]indolo[2,1-a][2]benzazepine-1a(2H)-carboxylic acid, 8-cyclohexyl-5-[[[(benzylamino)sulfonyl]amino]carbonyl]-1,12b-dihydro-11-(methoxy)-12-(methoxy)-, methyl ester, (*+/-*)*-. A solution of (+/-) 8-cyclohexyl-1,1a,2,12b-tetrahydro-11-methoxy-1a-(methoxycarbonyl)-cycloprop[d]indolo[2,1-a][2]benzazepine-5-carboxytic acid (200 mg, 0.44 mmol) and CDI (92 mg, 0.57 mmol) in THF (5 mL) was stirred for 1 hr at 60 °C. N-benzylsulfamide (164 mg, 0.88 mmol) and DBU (100 mg, 0.66 mmol) were then added and the resultant mixture was stirred at 60 °C overnight. The reaction was then poured into cold water, acidified with dilute hydrochloric acid and extracted into ethyl acetate. The organic phase was washed hydrochloric acid (0.1 N), brine and dried (sodium sulfate) and evaporated in vacuo to provide the title compound as a brown solid. ESI-MS m/e 628 (MH⁺).

### Intermediate 25

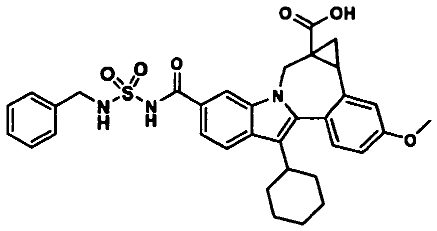

*Cycloprop[d]indolo[2,1-a][2]benzazepine-1a(2H)-carboxylic acid, 8-cyclohexyl-1,12b-dihydro-11-methoxy-5-[[[[(pheny*/*methy*/*)amino]su*/*fony*/*]amino]carbony*/*]-, (+*/*-)-*. The title compound was prepared using a similar procedure to that described for cycloprop[d]indolo[2,1-a][2]benzazepine-1a(2H)-carboxylic acid, 8-cyclohexyl-5-[[[(methylamino)sulfonyl]amino]carbonyl]-1,12b-dihydro-11-methoxy-cycloprop[d]indolo[2,1-a][2]benzazepine-1a(2H)-carboxylic acid starting from (+/-) 8-cyclohexyl-1,1a,2,12b-tetrahydro-11-methoxy-1a-(methoxycarbonyl)-cycloprop[d]indolo[2,1-a][2]benzazepine-5-carboxylic acid. ESI-MS m/e 613 (MH+), 1H NMR (500 MHz, MeOD) δ ppm 1.22 - 2.20 (m, 13H) 3.27 - 3.31 (m, 1 H) 3.47 (d, J=14.95 Hz, 0.6 H) 3.92 (d, J=2.44 Hz, 3 H) 4.04 (d, 0.4 H) 4.31 (d, J=2.75 Hz, 2 H) 5.24 (d, 0.4 H) 5.48 (d, 0.6 H) 7.02 (d, 1 H) 7.17 (d, J=2.75 Hz, 1 H) 7.19 - 7.35 (m, 5 H) 7.39 (t, J=7.48 Hz, 2 H) 7.45 - 7.52 (m, 1 H) 7.80 (d, J=1.53 Hz, 0.4 H) 7.85 (dd, J=8.39, 6.87 Hz, 1 H) 8.22 (d, J=1.53 Hz, 0.6 H).
product was observed to exist as inter-converting rotamers, as evidenced from the compound's NMR spectrum.

### Intermediate 27

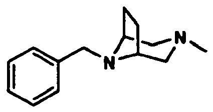

*3,8-Diazabicyclo[3.2.1]octane, 3-methyl-8-(phenylmethyl)-.* Cis-1-Benzyl-2,5-bis(chloromethyl)pyrrolidine hydrochloride (37.5 g, 0.13 mol) (Prepared as described in Published PCT patent application WO200232902) was suspended in CH₃CN (900 mL) in a 3-neck 5 L round bottom flask fitted with mechanical stirrer, reflux condenser, and thermometer. The stirred suspension was warmed to 50°C, NaHCO₃ (97 g, 1.1 mol) was added, and the suspension was warmed to 70°C. NaI (50 g, 0.33 mol) was added and stirred at 70 °C for 5 min, at which point an addition funnel was affixed atop the condenser. To the addition funnel was added 48 mL of 40% aqueous MeNH₂ (0.55 mol) in 850 mL of CH₃CN, and this solution was added dropwise (rate of addition maintained between 10-15 ml/min). The addition was complete after 75 min, at which point the reaction was cooled to rt., the solids filtered off, and the solvent concentrated to ∼800 mL. The reaction was poured into EtOAc (800 mL) and washed with 1 N NaOH (2 x 100 mL). The aqueous phase was re-extracted with EtOAc (2 x 100 mL), the combined organic phases were dried over Na₂SO₄ and concentrated. The resulting residue was introduced on to silica gel (620g) and eluted with 2.8% MeOH/0.4% conc. NH₄OH in CHCl₃ (6 L total). Pure fractions were collected from 2 L to 4 L. Concentration yielded 8.76 g (32% yield) of the title compound as a brown oil. 1H NMR (400 MHz, CDCl3) δ ppm 1.79 - 1.87 (m, 2 H) 1.92 - 1.99 (m, 2 H) 2.23 (s, 3 H) 2.27 - 2.37 (m, 2 H) 2.54 - 2.63 (m, 2 H) 3.10 (s, 2 H) 3.52 (s, 2 H) 7.20 - 7.26 (m, 1 H) 7.30 (t, J=7.30 Hz, 2 H) 7.36 - 7.42 (m, 2 H). LC method: Solvent A = 10% MeOH / 90% H2O / 0.1% TFA, Solvent B = 90% MeOH / 10% H2O / 0.1 % TFA, Start %B = 0%, Final %B = 100, Flow Rate = 4 ml/min, Gradient time = 2 min, Run time = 3 min, Column: Phenomenex-Luna 10 µm C18 50 mm x 3.0 mm, Rt = 0.23 min; MS: (ES+) m/z (M+H)+ = 217.3. An additional 6.1 g of mixed fractions were obtained from the column (>80% pure by 1H NMR integration).

### Intermediate 28

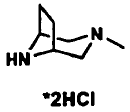

*3,8-Diazabicyclo[3.2.1]octane, 3-methyl-, dihydrochloride.* N-methyl-N-benzylbicyclodiamine, (14.22g, 65.7mMol) was dissolved in 650ml of methanol and 17ml of 12M aqueous hydrochloric acid was added. The solution was placed in a 2L Parr bottle under nitrogen and 3.66g of 20% palladium hydroxide on carbon added to the reaction. The mixture was placed on a Parr shaker under 60psig of hydrogen for 17hours. The reaction was judged complete by TLC analysis (Silica Gel plate eluted with a 10 parts by volume solution of 2M ammonia in methanol dissolved in 90 parts by volume of chloroform). The reaction was filtered through a plug of ceilite, which was then rinsed sequentially with water and methanol. The combined filtrates were concentrated in vacuuo and methanol and benzene added until a homogenous solution was obtained. 75mL of 2.0M hydrochloric acid in diethyl ether was then added. Volatiles were removed from the product solution in vacuuo. A pale yellow solid was eventually obtained by repeated azetroping of water from the product solution using a methanol / benzene mixture. The solid product, 3-methyl-3,8-diazabicyclo[3.2.1]octane was dried in vacuuo overnight to obtain 11.98g (91%) of a hygroscopic solid. The product was removed from the flask and bottled in a glove bag under nitrogen due to its hygroscopic nature. ¹H NMR (500 MHz, DMSO-D6) δ ppm 1.96 - 2.14 (m, 2 H) 2.34 (d, J=8.24 Hz, 2 H) 2.66 (s, 3 H) 3.46 (d, J=11.90 Hz, 2 H) 3.58 (s, 3 H, contains H2O) 4.17 (s, 2 H) 9.92 (s, 1 H) 10.21 (s, 1 H) 11.39 (s, 1H); ¹³C NMR (126 MHz, DMSO-D6) δ ppm 24.04 (s, 1 C) 43.49 (s, 1 C) 52.50 (s, 1 C) 54.47 (s, 1 C).

### Intermediates 29 and 30

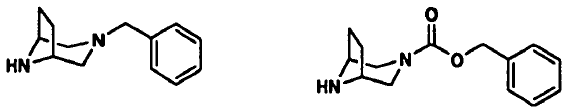

*3,8-diazabicyclo[3.2.1]octane-3-carboxylic acid, phenylmethyl ester and 3-(phenylmethyl)-3,8-diazabicyclo[3.2.1]octane*. Triethylamine (1.44 mL, 10.363 mmol) was added to a solution of 8-boc-3,8-diaza-bicyclo[3.2.1]ocatane (2.0 g, 9.421 mmol) in CH₂Cl₂ (20 mL), Benzyl chloroformate (1.46 mL, 10.363 mmol) was added dropwise at 0°C and the reaction mixture was stirred at 0°C for 0.5 hr, then allowed to warm to rt. and stirring was continued for 3 days. The reaction mixture was then quenched with water and acidified with 1N HCl solution. The organic layer was separated, washed with brine, dried (MgSO₄) and concentrated to give a colorless thick oil as the crude product. 70 mg of this material was then dissolved in 1,2-dichloroethane (2mL) and TFA (0.5 mL) was added. The reaction mixture was stirred at rt. for 2 hr. The solvent and TFA were then evaporated to give a mixture of the two title compounds as a colorless thick oil.

### Intermediate 31

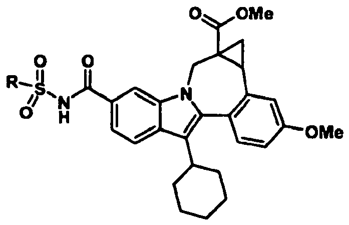

General procedure for making sulfonamides. A mixture of acid (1 equiv) and carbonyldiimidazole (1.5 equiv) in an. THF was heated at 50 °C for 30 min and allowed to cool to rt. Then 1 equiv of either sulfamide (R = NR₂) or sulfonamide (R = alkyl or aryl) and DBU (2 equiv) were added consecutively. The resultant mixture was stirred at rt overnight. After acidic aqueous workup, isolated crude product was purified by prep. HPLC to afford the title intermediates.

### Intermediate 32

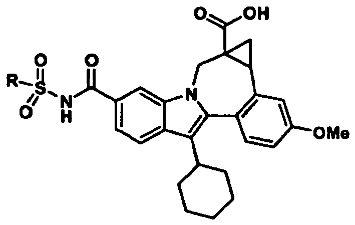

General procedure for making acids. Methyl esters hydrolyzed using 1N NaOH in THF-MeOH.

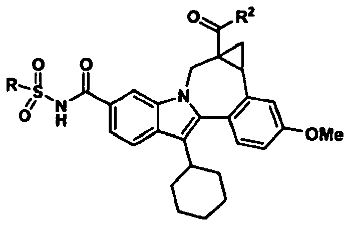

General procedure for making amides for some examples. Acid derivatives (1 equiv) were combined with corresponding amine (1.2 equiv), triethylamine (2-3 equiv) and TBTU (1.3 equiv) in anh. DMF and stirred at rt for 1-2 h until completion of the amide coupling. Isolated crude products were purified by prep. HPLC to provide the desired amides.

### Additional compounds

Additional compounds can be made using the following routes.

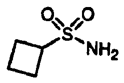

To a 250mL RBF equipped with a stir bar was added bromocyclobutane (3.49 mL, 37.0 mmol) and 70mL of diethyl ether. The flask was cooled to -78°C (acetone/dry ice bath). To this solution was then added, via syringe, 2.0eq. of a 1.7M solution of tert-butyllithium (43.6 mL, 74.1 mmol). The mixture was stirred for 60minutes, then cannulated into a 500mL flask containing sulfuryl chloride (6.00 mL, 74.1 mmol) in 30mL of diethylether at -78°C. The suspension was warmed to room temperature overnight. The white mixture was diluted with 40mL of diethylether, filtered and set aside. A 3 necked 500mL RBF equipped with a stir bar and dry THF (10 mL) was cooled to -65°C with the aid of a dry ice/isopropanol bath and gaseous ammonia was slowly sparged into the flask. Previously synthesized cyclobutanesulfonyl chloride (5.2g, 33.6 mmol) was then dripped in via syringe (crude mixture in -200mL of ether/THF). Sparging of ammonia gas was continued for an additional 5 minutes. The mixture was kept at -65°C for 4 hours then allowed to slowly warm to room temperature. The reaction mixture was filtered and washed with 100mL of THF. The solvent was evaporated to give 2.1g of the desired sulfonamide (46% yield) as a pale yellow oily solid. ¹H NMR (500 MHz, DMSO-D6): δppm: 1.81 - 1.89 (m, 2 H), 2.16 - 2.22 (m, 2 H), 2.23 - 2.31 (m, 2 H), 3.66 - 3.74 (m, 1 H), 6.68 (s, 2 H).

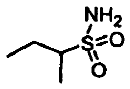

¹H NMR (500 MHz, DMSO-D6): δ ppm 0.94 (m, 3 H), 1.20 (m, 3 H), 1.30 - 1.45 (m, 1 H), 1.90 (m, 1 H), 2.76 (m, 1H), 6.59 (s, 2 H).

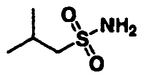

¹H NMR (500 MHz, DMSO-D6): δ ppm 1.02 (d, *J*= 6.95 Hz, 6 H), 2.11 (m, 1 H), 2.86 (d, *J*= 6.22 Hz, 2 H), 6.71 (s, 2 H).

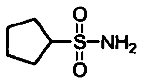

¹H NMR (500 MHz, DMSO-D6): δ ppm 1.51 - 1.66 (m, 4 H), 1.86 (m, 4 H), 3.37 (m, 1 H), 6.65 (s, 2 H).

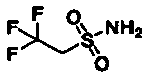

¹H NMR (500 MHz, DMSO-D6): δ ppm 4.24 (m, 2 H), 7.46 (s, 2 H).

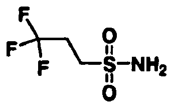

¹H NMR (500 MHz, DMSO-D6): δ ppm 2.70 (m, 2H), 3.20 (m, 2 H), 7.01 (s, 2 H).

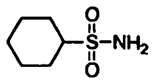

¹H NMR (500 MHz, DMSO-D6): δ ppm 1.07 - 1.17 (m, 1H), 1.22 - 1.38 (m, 4H), 1.62 (m, 1H), 1.78 (m, 2H), 2.05 (m, 2H), 2.68 - 2.77 (m, 1 H), 6.57 (s, 2 H).

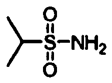

**¹H** NMR (300 MHz, DMSO-D6): δ ppm 1.22 (d, *J*=6.59 Hz, 6 H), 3.00 (m, 1 H), 6.59 (s, 2 H).

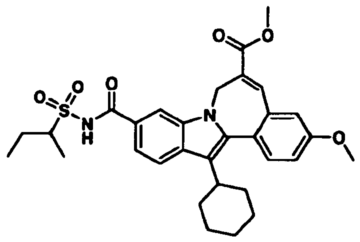

*Methyl 10-((sec-butylsulfonyl)carbamoyl)-13-cyclohexyl-3-methoxy-7H-indolo[2,1-a][2]benzazepine-6-carboxylate*. In a 100 mL round-bottomed flask (RBF) was added carboxylic acid 1 (575 mg, 1.291 mmol) and 1,1'-carbonyldiimidazole (460 mg, 2.84 mmol) in THF (15 mL) to give a yellow solution. The mixture was stirred at room temperature under nitrogen for 1 hour then heated to 70°C, in an oil bath, for 90 minutes. The mixture was cooled and sec-butyl sulfonamide (921 mg, 6.71 mmol) in 4 mL of THF was added along with neat DBU (0.389 mL, 2.58 mmol). The RBF was returned to the oil bath and heated overnight at 70°C. The reaction mixture was transferred to a separatory funnel, diluted with 100mL of DCM, washed x3 with 100 mL of 0.5 M HCl, then with 100 mL of H₂O, and finally saturated NaCl. The organic mixture was dried over MgSO4, filtered and concentrated to give 713mgs of the desired acylsulfonamidc 2 as a yellow solid (96% yield) which was placed under vacuum overnight. LC data was recorded on a Shimadzu LC-10AS liquid chromatograph equipped with a Phenomenex-Luna 10µ, C18, 4.6x30mm column, using a SPD-10AV UV-Vis detector at a detector wave length of 220nM. The elution conditions employed a flow rate of 5 ml/min., a gradient of 100% solvent A / 0% solvent B to 0% solvent A / 100% solvent B, a gradient time of 2 min., a hold time of 1 min., and an analysis time of 3 min. where solvent A was 10% MeOH / 90% H2O / 0.1% trifluoroacetic acid and solvent B was 10% H2O / 90% MeOH / 0.1% trifluoroacetic acid. MS data was determined using a Micromass Platform for LC in electrospray mode.1H NMR (500 MHz, CD3OD): δ ppm 0.84 - 0.92 (m, 3 H), 1.03 (t, J =7.32 Hz, 3 H), 1.23 (m, 1 H), 1.28 - 1.44 (m, 7 H), 1.58 (m, 1 H), 1.72 (m, 2 H), 1.85 (m, 1 H), 1.95 - 2.07 (m, 3 H), 2.17 (m, 1 H), 2.78 (m, 1 H), 3.69 (m, 2 H), 3.83 - 3.91 (m, 3 H), 7.02 (s, 1 H), 7.11 (m, 1 H), 7.47 (d, J = 7.63 Hz, 1 H), 7.74 (m, 3 H), 8.25 (s, 1 H). LC/MS: m/z 565.22, Rf 2.192 min., 97.5 % purity.

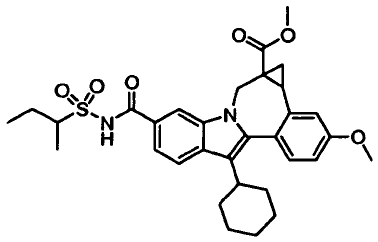

*Methyl 5-((sec-butylsulfonyl)carbamoyl)-8-cyclohexyl-11-methoxy-1,12b-dihydro cyclopropa[d]indolo[2,1-a][2]benzazepine-1a(2H)-carboxylate.* To 63.1 mgs of 95% NaH in 5 mL of dry DMF in a 100 mL RBF was added 629 mgs of trimethylsulfoxonium iodide at room temperature. The mixture was stirred at room temperature under nitrogen for 30minutes. A solution of Intermediate 9 (in 7 mL of DMF) was added via syringe and the reaction was stirred for 15 - 20 minutes. The reaction mixture was quickly cooled to 0°C with an ice bath, I mL of I M HCl was added followed by 60 mL of ice water. The heterogeneous mixture was stirred for 30 minutes. The mixture was filtered and the yellow solid was washed with ice water. The solid was taken up in 2% methanol/DCM and was purified using a Biotage Horizon MPLC employing a 40+M column with a solvent gradient of 2% methanol/DCM to 10% methanol/DCM. 450mgs (62%yield) of the compound was obtained as a yellow solid after solvent evaporation. LC data was recorded on a Shimadzu LC-10AS liquid chromatograph equipped with a Phenomenex-Luna 10µ, C18, 4.6x30mm column, using a SPD-10AV UV-Vis detector at a detector wave length of 220nM. The elution conditions employed a flow rate of 5 ml/min., a gradient of 100% solvent A / 0% solvent B to 0% solvent A / 100% solvent B, a gradient time of 2 min., a hold time of 1 min., and an analysis time of 3 min. where solvent A was 10% MeOH / 90% H2O / 0.1% trifluoroacetic acid and solvent B was 10% H2O / 90% MeOH / 0.1% trifluoroacetic acid. MS data was determined using a Micromass Platform for LC in electrospray mode. 1H NMR (300 MHz, CD3OD): δ ppm 0.19 (m, 0.35 H), 1.03 - 1.14 (m, 3 H), 1.19 - 1.34 (m, 2.65 H), 1.43 (m, 5 H), 1.55 - 1.66 (m, 2 H), 1.74 (m, 2 H), 1.89 - 1.94 (m, 2 H), 1.99 - 2.14 (m, 3 H), 2.64 - 2.95 (m, 2 H), 3.35 (d, J=15.00 Hz, 0.65 H), 3.48 (m, 2 H), 3.67 - 3.81 (m, 2 H), 3.85 (s, 3 H), 3.90 - 3.98 (m, 0.35 H), 5.17 (m, 0.35 H), 5.36 (m, 0.65 H), 6.91 - 6.98 (m, 1 H), 7.09 (m, 0.35 H), 7.16 (m, 0.65 H), 7.19 - 7.27 (m, 1 H), 7.52 - 7.65 (m, 1 H), 7.83 (m, 1 H), 8.09 (s, 0.35 H), 8.29 (s, 0.65 H). LC/MS: m/z 579.31, Rf 2.167 min., 95.2 % purity.

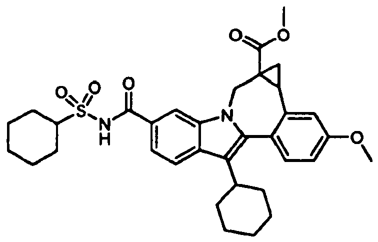

*Methyl 8-cyclohexyl-5-((cyclohexylsulfonyl)carbamoyl)-11-methoxy-1,12b-dihydrocyclo propa[d]indolo[2,1-a][2]benzazepine-1a(2H)-carboxylate*. 1H NMR (300 MHz, CD3OD): δ ppm 0.23 (m, 0.35 H), 1.14 - 1.53 (m, 10 H), 1.60 - 1.79 (m, 3 H), 1.91 (m, 3 H), 2.09 (m, 1.65 H), 2.18 (m, 3 H), 2.81 - 2.98 (m, 3 H), 3.41-3.46 (m, 0.65 H), 3.50 (m, 2 H), 3.71 - 3.79 (m, 2 H), 3.88 (s, 3 H), 3.99 - 4.04 (m, 0.35 H), 5.25 (m, 0.35 H), 5.45 (m, 0.65 H), 6.97 - 7.02 (m, 1 H), 7.13 (m, 0.35 H), 7.21 (m, 0.65 H), 7.26 - 7.32 (m, 1 H), 7.55 - 7.65 (m, 1 H), 7.85 - 7.92 (m, 1 H), 8.11 (s, 0.35 H), 8.32 (s, 0.65 H). LC/MS: m/z 605.42, Rf 2.223 min., 99.2 % purity.

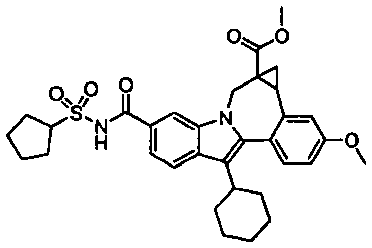

*Methyl 8-cyclohexyl-5-((cyclopentylsulfonyl)carbamoyl)-11-methoxy-1,12b-dihydrocyclo propa[d]indolo[2,1-a][2]benzazepine-1a(2H)-carboxylate*. 1H NMR (300 MHz, CD3OD): δ ppm 0.23 (m, 0.35 H), 1.27 (m, 2.65 H), 1.39 (m, 2 H), 1.60 - 1.79 (m, 7 H), 1.91 - 2.19 (m, 8 H), 2.67 - 2.97 (m, 2 H), 3.47 (m, 0.65 H), 3.50 (m, 3 H), 3.78 - 3.87 (m, 3 H), 4.10 (m, 0.35 H), 4.29 (m, 1 H), 5.22 (m, 0.35 H), 5.43 (m, 0.65 H), 6.98 - 7.02 (m, 1 H), 7.14 (m, 0.35 H), 7.21 (m, 0.65 H), 7.26 - 7.32 (m, 1 H), 7.55 - 7.65 (m, 1 H), 7.85 - 7.91 (m, 1 H), 8.10 (s, 0.35 H), 8.32 (s, 0.65 H). LC/MS: m/z 591.33, Rf 2.200 min., 100 % purity.

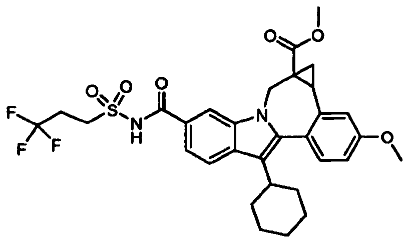

*methyl 8-cyclohexyl-11-methoxy-5-(((3,3,3-trifluoropropyl)sulfonyl)carbamoyl)-1,12b-dihydrocyclopropa[d]indolo[2,1-a][2]benzazepine-1a(2H)-carboxylate.* 1H NMR (300 MHz, CD3OD): δ ppm 0.19 (m, 0.35 H), 1.25 (m, 1.65 H), 1.41 (m, 2 H), 1.65 (m, 1 H), 1.76 (m, 2 H), 1.94 (m, 2 H), 2.04 (m, 1 H), 2.61 - 2.84 (m, 6 H), 2.88 - 2.96 (m, 1 H), 3.35 - 3.40 (m, 0.65 H), 3.48 (m, 2 H), 3.80 (m, 2 H), 3.86 (m, 3 H), 3.89 - 3.98 (m, 0.35 H), 5.18 (m, 0.35 H), 5.38 (m, 0.65 H), 6.96 - 7.01 (m, 1 H), 7.13 (m, 0.35 H), 7.20 (m, 0.65 H), 7.24 - 7.30 (m, 1 H), 7.58 - 7.69 (m, 1 H), 7.84 - 7.90 (m, 1 H), 8.13 (s, 0.35 H), 8.34 (s, 0.65 H). LC/MS: m/z 619.32, Rf 2.188 min., 99.5 % purity.

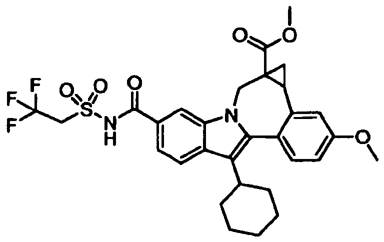

*methyl 8-cyclohexyl-11-methoxy-5-(((2,2,2-trifluoroethyl)sulfonyl)carbamoyl)-1,12b-dihydro cyclopropa[d]indolo[2,1-a][2]benzazepine-1a(2H)-carboxylate.* 1H NMR (300 MHz, CD3OD): δ ppm 0.13 (m, 0.35 H), 1.18 (m, 1.65 H), 1.38 (m, 2 H), 1.57 - 1.62 (m, 2 H), 1.73 (m, 2 H), 1.87 (m, 2 H), 1.96 - 2.05 (m, 1 H), 2.60 - 2.90 (m, 1.35 H), 3.17 - 3.22 (m, 0.65 H), 3.45 (m, 2 H), 3.74 (m, 1 H), 3.84 (m, 2 H), 4.04 - 4.10 (m, 3 H), 4.38 - 4.53 (m, 2 H), 5.06 (m, 0.35 H), 5.18 (m, 0.65 H), 6.90 - 6.96 (m, 1 H), 7.06 (m, 0.35 H), 7.13 (m, 0.65 H), 7.16 - 7.22 (m, 1 H), 7.63 (m, 0.65 H), 7.70 - 7.80 (m, 1.35 H), 8.14 (s, 0.35 H), 8.33 (s, 0.65 H). LC/MS: m/z 605.29, Rf 2.178 min., 96.5 % purity.

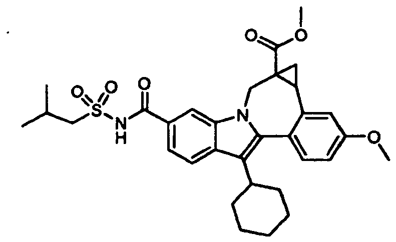

*Methyl 8-cyclohexyl-5-((isobutylsulfonyl)carbamoyl)-11-methoxy-1,12b-dihydrocyclopropa [d]indolo[2,1-a][2,1-a][2]benzazepine-1a(2H)-carboxy*/*ate*. 1H NMR (300 MHz, CD3OD): δ ppm 0.17 (m, 0.35 H), 1.09 (m, 6 H), 1.22 (m, 1.65 H), 1.38 (m, 2 H), 1.49 - 1.60 (m, 1 H), 1.73 (m, 2 H), 1.87 (m, 2 H), 1.96 - 2.05 (m, 2 H), 2.15 - 2.39 (m, 1 H), 2.61- 2.87 (m, 2 H), 2.96 (d, J = 6.22 Hz, 2 H), 3.19 (m, 2 H), 3.43 (m, 2 H), 3.70 (m, 2 H), 3.84 (m, 2 H), 5.06 - 5.11 (m, 1 H), 6.90 - 6.95 (m, 1 H), 7.05 - 7.11 (m, 1 H), 7.16 - 7.23 (m, 1 H), 7.67 - 782 (m, 2 H), 8.20 (s, 0.35 H), 8.39 (s, 0.65 H). LC/MS: m/z 579.30, Rf 2.190 min., 96.2% purity.

*General procedure for the transformation of esters of formula I to corresponding amides.* In a 100 mL round-bottomed flask was added I N sodium hydroxide (3 eq., 1.583 ml, 1.583 mmol) and bridged ester 1 (1 eq., 0.528 mmol) in methanol (4.00 ml) and THF (4.00 ml) to give a yellow solution. The mixture was stirred for 3 hours at room temperature. 3 equivalents of 1 N HCl was then added, the product diluted with ethyl acetate then extracted, washed with brine and dried over MgSO₄. Filtration and subsequent evaporation of volatiles gave the carboxylic acids 2 in near quantitative yield. To a 0.10 mmol solution of carboxylic acid 2 in 1 mL of anhydrous N,N-Dimethylformamide (DMF) in a 2 dram vial equipped with a Teflon™ lined screw cap was added 0.3 mmol (3 eq.) of 2-(1H-Benzotriazole-1-yl)-1,1,3,3,-Tetramethyluronium Tetrafluoroborate (TBTU) in 1.0 mL of anhydrous DMF followed by the addition of 0.2 mmol (2 eq.) of amine 3 in 1.0 mL of anhydrous DMF and 0.4 mmol of neat *N,N*-diisopropylethylamine. The reaction was shaken on a VWR Vortex-Genie 2 Mixer overnight at room temperature. The reaction volumes were then reduced in a Savant Speedvac and the crude products were taken up in 1.2 mL of methanol and purifed using a Shimadzu preparative HPLC employing methanol/water and 0.1% trifluoroacetic acid buffer with a Phenomenex Luna, C18, 30 mm x 100 mm, 10 µm column at a gradient of 40-100% B and a flow rate of 40 mL/min. over 10 minutes with a 5-10 minute hold, to give carboxamides 4 as yellow amorphous solids (65% - 70% yield). Post-purification LC/MS data was obtained on a Shimadzu analytical LC /Micromass Platform LC (ESI+) at 220nm using the following set of conditions: Column I (Phenomenex 10µm C18, 4.6 x 30mm), Solvent system I (gradient of 0-100%B where B = 90% HPLC grade methanol 0.1% trifluoroacetic acid/ 10% HPLC grade water), in 2 minutes with a 1 minute hold at a flow rate of 5 mL/minute.

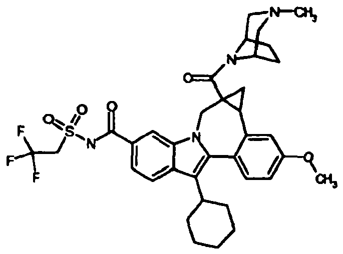

*8-Cyclohexyl-11-methoxy-1a-((3-methyl-3,8-diazabicyclo[3.2.1]oct-8-yl)carbonyl)-N-((2,2,2-trifluoroethyl)sulfonyl)-1,1a,2,12b-tetrahydrocyclopropa[d]indolo[2,1-a][2]benzazepine-5-carboxamide.* 1H NMR (300 MHz, CD3OD): δ ppm 0.16 (m, 0.20 H), 1.22 - 1.37 (m, 2.80 H), 1.45 (m, 3 H), 1.62 (m, 1 H), 1.78 (m, 3 H), 1.92 - 2.21 (m, 5 H), 2.52 - 2.69 (m, 1 H), 2.81 (m, 3 H), 2.94 (m, 2 H), 3.16 (m, 1 H), 3.39 (m, 2 H), 3.51 - 3.65 (m, 2 H), 3.85 - 3.92 (m, 3 H), 4.15 - 4.37 (m, 1 H), 4.66 (m, 3 H), 5.10 (m, 1 H), 6.97 - 7.05 (m, 1 H), 7.15 (d, J=1.83 Hz, 0.20 H), 7.19 (d, J=1.83 Hz, 0.80 H), 7.30 (d, J=8.42 Hz, 1 H), 7.53 - 7.63 (m, 1 H), 7.89 (d, J=8.42 Hz, 1 H), 7.99 - 8.08 (m, 1 H). LC/MS: m/z 699.35, Rf 1.810 min., 98.0 % purity.

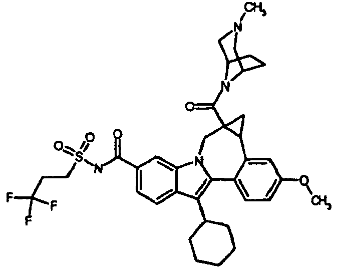

*8-Cyclohexyl-11-methoxy-1a-((3-methyl-3,8-diazabicyclo[3.2.1]oct-8-yl)carbonyl)-N-((3,3,3-trifluoropropyl)sulfonyl)-1,1a,2,12b-tetrahydrocyclopropa[d]indolo[2,1-a][2]benzazepine-5-carboxamide.* 1H NMR (300 MHz, CD3OD): δ ppm 0.14 (m, 0.20 H), 1.19 - 1.33 (m, 2.80 H), 1.42 (m, 3 H) 1.60 (m, 1 H), 1.76 (m, 3 H), 2.00 (m, 5 H), 2.54 (m, 1 H), 2.65 (m, 1 H), 2.72 - 2.86 (m, 5 H), 2.87 - 3.01 (m, 2 H), 3.30 - 3.44 (m, 2 H), 3.49 - 3.63 (m, 2 H), 3.78 - 3.89 (m, 5 H), 4.17 - 4.29 (m, 1 H), 4.64 (m, 1 H), 5.07 (m, 1 H), 6.96 - 7.03 (m, 1 H), 7.13 (d, J=2.56 Hz, 0.20 H), 7.17 (d, J=2.56 Hz, 0.80 H), 7.28 (d, J=8.42 Hz, 1 H), 7.54 (d, J=8.42 Hz, 0.80 H), 7.59 (d, J=8.42 Hz, 0.20 H), 7.83 - 7.90 (m, 1 H), 7.97 (s, 0.80 H), 8.07 (s, 0.20 H), LC/MS: m/z 713.36, Rf 1.822 min., 98.7 % purity.

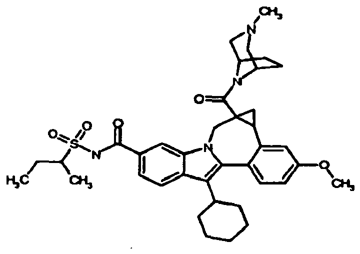

*N-(sec-butylsulfonyl)-8-cyclohexyl-11-methoxy-1a-((3-methyl-3,8-diazabicyclo [3.2.1]oct-8-yl)carbonyl)-1,1a,2,12b-tetrahydrocyclopropa[d]indolo[2,1-a][2] benzazepine-5-carboxamide.* 1 H NMR (300 MHz, CD3OD): δ ppm 0.17 (m, 0.20 H), 1.08 (t, J=7.50 Hz, 3 H), 1.19 - 1:34 (m, 1.80 H), 1.38 - 1.53 (m, 6 H), 1.70 (m, 5 H), 2.08 (m, 5 H), 2.40 (m, 1 H), 2.67 (m, 1 H), 2.82 (m, 3 H), 2.94 (m, 3 H), 3.27 (m, 1 H), 3.32 - 3.46 (m, 2 H), 3.49 - 3.65 (m, 2 H), 3.74 (m, 1 H), 3.84 - 3.92 (m, 3 H), 4.21 (m, 1 H), 4.65 (m, 1 H), 5.10 (m, 1 H), 6.97 - 7.05 (m, 1 H), 7.14 - 7.20 (m, 1 H), 7.30 (m, 1 H), 7.55 (m, 1 H), 7.87 - 7.92 (m, 1 H), 7.97 - 8.05 (m, 1 H). LC/MS: m/z 673.32, Rf 1.820 min., 98.2 % purity.

Isomer-A and Isomer B were obtained from the SFC chiral separation of (1a*R*, 12b*S*)-*N*-(*sec*-butylsulfonyl)-8-cyclohexyl-11-methoxy-1a-((3-methyl-3,8-diazabicyclo [3.2.1]oct-8-yl)carbonyl)-1,1a,2,12b-tetrahydrocyclopropa[*d*]indolo[2,1-*a*][2] benzazepine-5-carboxamide which was prepared from the chiral ethyl ester. SFC Separation conditions: Column: ChiralPak AD-H, 30 x 250mm, 5µm; Mobile Phase: 25% isopropyl alcohol/ 75% CO2; Pressure: 100 bar; Temperature: 35°C; Flow Rate: 70 mL/min.; UV: 260 nm; Injection: 2 mL (∼5 mg/mL in 9:1 IPA:ACN); Collection: Isomer-A (Peak 1) 35.0 - 41.5 min; Isomer-B (Peak 2) 43.5 - 52.5 min. Isomer-A. Analytical HPLC were performed by using Shimadzu-VP instrument with UV detection at 254 nm and 256 nm. Analytical HPLC method: Solvent A = 5% MeCN-95% H₂O-0.1 % TFA, Solvent B = 95% MeCN-5%H₂O-0.1% TFA, Start %B = 10, Final %B = 100, Gradient time = 10 min, Flow Rate = 1 ml/min. Column: Waters Sunfire C-18, 4.6 x 150 mm, 3.5 uM, Rₜ = 7.86 min. LC/MS were performed by using Shimadzu-VP instrument with UV detection at 220 nm and Waters Micromass. HPLC method: Solvent A = 10% MeOH-90% H₂O-0.1% TFA, Solvent B = 90% MeOH-10%H₂O-0.1% TFA, Start %B = 0, Final %B = 100, Gradient time = 2 min, Flow Rate = 5 ml/min, Column: Xterra MS C18 S7 3.0 x 50mm; (ES+) m/z (M+H)⁺ = 673.56, HPLC Rₜ = 1.637 min. HPLC method: Solvent A = 5% MeCN-95% H₂O-10 mM NH₄OAc, Solvent B = 95% MeCN-5%H₂O-10 mM NR₄OAc, Start %B = 0, Final %B = 100, Gradient time = 2 min, Flow Rate = 5 ml/min, Column: Phenomenex Lina C 18 5um 3.0 x 50mm; (ES+) m/z (M+H)⁺ = 673.54, HPLC Rₜ = 1.255 min. Optical rotation: -74.75°, (3.21 mg/ml MeOH, 589 nm, 50 mm cell).

Isomer-B. Analytical HPLC were performed by using Shimadzu-VP instrument with UV detection at 254 nm and 256 nm. Analytical HPLC method: Solvent A = 5% MeCN95% H2O0.1% TFA, Solvent B = 95% MeCN5%H2O0.1% TFA, Start %B = 10, Final %B = 100, Gradient time = 10 min, Flow Rate = I ml/min, Column: Waters Sunfire C-18, 4.6 x 150 mm, 3.5 uM, Rt = 8.35 min. LC/MS were performed by using Shimadzu-VP instrument with UV detection at 220 nm and Waters Micromass. HPLC method: Solvent A = 10% MeOH90% H2O0.1% TFA, Solvent B = 90% MeOH 10%H2O0.1% TFA, Start %B = 0, Final %B = 100, Gradient time = 2 min, Flow Rate = 5 ml/min. Column: Xterra MS C18 S7 3.0 x 50mm; (ES+) m/z (M+H)+ = 673.53, HPLC Rt = 1.650 min. HPLC method: Solvent A = 5% MeCN95% H2O10 mM NH4OAc, Solvent B = 95% MeCN5%H2O10 mM NH4OAc Start %B = 0, Final %B = 100, Gradient time = 2 min, Flow Rate = 5 ml/min, Column: Phenomenex Lina C18 5um 3.0 x 50mm; (ES+) m/z (M+H)+ = 673.54, HPLC Rt = 1.263 min. Optical rotation:⁻75.36°, (3.29 mg/ml MeOH. 589 nm, 50 mm cell).

### Reference example

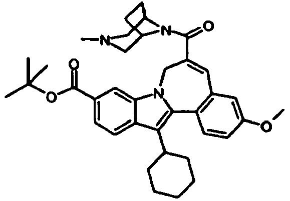

*tert-Butyl 13-cyclohexyl-3-methoxy-6-((3-methyl-3,8-diazabicyclo[3.2.1]oct-8-yl)carbonyl)-7H-indolo[2,1-a][2]benzazepine-10-carboxylate*. HATU (680 mg, 1.8 mmol) was added to a stirring solution of 10-(*tert*-butoxycarbonyl)-13-cyclohexyl-3-methoxy-7*H*-indolo[2,1-*a*][2]benzazepine-6-carboxylic acid (670mg, 1.37 mmol) and 3-methyl-3,8-diazabicyclo[3.2.1]octane di HCl salt (560 mg, 2.81 mmol) in DMF (6 mL) and TEA (1.2 mL, 8.2 mmol) and the reaction was stirred for 30 min (complete by LCMS). The reaction mixture was diluted with water (∼35 mL) (precipitate formed) and stirred ON. The precipitate was collected by filtration, flushed with water and dried under high vacuum at 55 °C to yield *tert*-butyl 13-cyclohexyl-3-methoxy-6-((3-methyl-3,8-diazabicyclo[3.2.1]oct-8-yl)carbonyl)-7*H*-indolo[2,1-*a*][2]benzazepine-10-carboxylate (775 mg, 1.30 mmol, 95% yield) as a light yellow solid. The material was used without further purification. ¹HNMR (300 MHz, CDCl₃) δ ppm 1.14 - 3.95 (m, 24H), 1.59 (s, 9H), 3.86 (s, 3H), 4.21 - 5.26 (m, 2H), 6.82 (s, 1H), 6.88 (d, *J* = 2.6 Hz, 1H), 7.01 (dd, *J* = 8.8, 2.6 Hz, 1H), 7.47 (d, *J* = 8.8 Hz, 1H), 7.66 (dd, *J* = 8.4, 1.1 Hz, 1H), 7.80 (d, *J* = 8.4 Hz, 1H), 8.02 (br s, 1H). LC-MS retention time: 3.72 min; m/z 596 (MH+). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 mL/min, a gradient of 100% solvent A / 0% solvent B to 0% solvent A / 100% solvent B, a gradient time of 4 min, a hold time of 1 min, and an analysis time of 5 min where solvent A was 10% MeOH / 90% H₂O / 0.1% trifluoroacetic acid and solvent B was 10% H₂O / 90% MeOH / 0.1% trifluoroacetic acid. MS data was determined using a Micromass Platform for LC in electrospray mode.

### Reference example

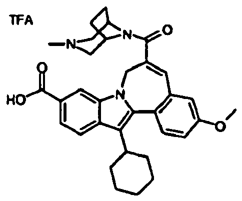

*13-Cyclohexyl-3-methoxy-6-((3-methyl-3,8-diazabicyclo[3.2.1]oct-8-yl)carbonyl)-7H-indolo[2,1-a][2]benzazepine-10-carboxylic acid trifluoroacetate*. *tert*-Butyl 13-cyclohexyl-3-methoxy-6-((3-methyl-3,8-diazabicyclo[3.2.1]oct-8-yl)carbonyl)-7*H*-indolo[2,1-*a*][2]benzazepine-10-carboxylate (300 mg, 0.504 mmol) was dissolved into DCE (5 mL) and then TFA (700 µl, 9.09 mmol) was added (reaction became green) and the reaction was stirred at rt for 1h (∼ 70% conversion by LCMS). More TFA (700 µl, 9.09 mmol) was added and the reaction was stirred 1h (complete by LCMS). The reaction mixture was concentrated on a rotary evaporator, diluted with diethyl ether and reconcentrated twice to yield 13-cyclohexyl-3-methoxy-6-((3-methyl-3,8-diazabicyclo[3.2.1]oct-8-yl)carbonyl)-7*H-*indolo[2,1-*a*][2]benzazepine-10-carboxylic acid trifluoroacetate (362 mg, 0.55 mmol, quant.) as a dark yellow solid. Used without further purification. ¹HNMR (300 MHz, DMSO-d₆) δ ppm 1.06 - 2.13 (m, 21H), 2.68 - 2.86 (m, 1H), 3.36- 3.50 (m, 2H), 3.90 (s, 3H), 4.11 - 5.35 (m, 2H), 7.14 (s, 1H), 7.18 - 7.28 (m, 2H), 7.53 (d, *J* = 8.4 Hz, 1H), 7.61 (dd, *J =* 8.4, 1.1 Hz, 1H), 7.87 (d, *J =* 8.4 Hz, 1H), 8.21 (br s, 1H), 9.55 (br s, 1H). LC-MS retention time: 3.72 min; m/z 596 (MH+). LC-MS retention time: 2.50 min; 538 m/z (MH-). LC data was recorded on a Shimadzu LC-10AS liquid chromatograph equipped with a Phenomenex-Luna 10u C18 4.6x50mm column using a SPD-10AV UV-Vis detector at a detector wave length of 220nM. The elution conditions employed a flow rate of 5 mL/min, a gradient of 100% solvent A / 0% solvent B to 0% solvent A / 100% solvent B, a gradient time of 4 min, a hold time of 1 min, and an analysis time of 5 min where solvent A was 5% acetonitrile / 95% H₂O / 10 mM ammonium acetate and solvent B was 5% H₂O / 95% acetonitrile / 10 mM ammonium acetate. MS data was determined using a Micromass Platform for LC in electrospray mode.

### Reference example

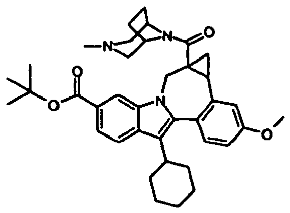

*tert-Butyl 8-cyclohexyl-11-methoxy-1a-((3-methyl-3,8-diazabicyclo[3.2.1]oct-8-yl)carbonyl)-1,1a,2,12b-tetrahydrocyclopropa[d]indolo[2,1-a][2]benzazepine-5-carboxylate.* Trimethylsulfoxonium iodide (375 mg, 1.69 mmol) was added in three portions to s stirring slurry of a 60% NaH dispersion (68 mg, 1.7 mmol) in DMSO (1.5 mL) (foaming occurred). The reaction mixture was stirred 20 min and then a solution of *tert*-butyl 13-cyclohexyl-3-methoxy-6-((3-methyl-3,8-diazabicyclo[3.2.1]oct-8-yl)carbonyl)-*7H*-indolo[2,1-a][2]benzazepine-10-carboxylate (438 mg, 0.735 mmol) in DMSO (2.5 mL) was added and the reaction was stirred for 1 h (no desired product by LCMS). The reaction mixture was heated at 90 °C for 3 h (complete by LCMS), cooled to rt, quenched with 0.25M aq. HCl (20 mL), and extracted with EtOAc (2 x 20 mL). The combined organic layers were washed with brine (20 mL), dried (MgSO₄) filtered and concentrated to yield 72009-057 as an orange oil. The oil was used without further purification as starting material in the preparation of 8-cyclohexyl-11-methoxy-1a-((3-methyl-3,8-diazabicyclo[3.2.1]oct-8-yl)carbonyl)-1,1a,2,12b-tetrahydrocyclopropa[*d*]indolo[2,1-*a*][2]benzazepine-5-carboxylic acid triflouroacetate. The compound was isolated as a mixture of enantiomers. LC-MS retention time: 3.68 min; m/z 610 (MH+). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 mL/min, a gradient of 100% solvent A / 0% solvent B to 0% solvent A / 100% solvent B, a gradient time of 4 min, a hold time of 1 min, and an analysis time of 5 min where solvent A was 10% MeOH / 90% H₂O / 0.1% trifluoroacetic acid and solvent B was 10% H₂O / 90% MeOH / 0.1% trifluoroacetic acid. MS data was determined using a Micromass Platform for LC in electrospray mode.

### Reference example

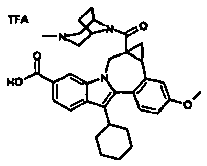

*8-Cyclohexyl-11-methoxy-1a-((3-methyl-3,8-diazabicyclo[3.2.1]oct-8-yl)carbonyl)-1,1a,2,12b-tetrahydrocyclopropa[d]indolo[2,1-a][2]benzazepine-5-carboxylic acid triflouroacetate*. Starting material (448 mg, 0.735 mmol) was dissolved into DCE (6 mL) and then TFA (1.5 mL, 19 mmol) was added (reaction became dark red) and the reaction was stirred at rt for 2h (complete by LCMS). The reaction was concentrated on a rotary evaporator, diluted twice with diethyl ether and reconcentrated to an orange oil. The residue was slurried with diethyl ether and the solids (360 mg yellow solid) were collected by filtration and rinsed with hexanes. Addition solids (52 mg of a yellow solid) were collected from the filtrate and rinsed with hexanes. The combined solids were shown to be 8-cyclohexyl-11-methoxy-1a-((3-methyl-3,8-diazabicydo[3.2.1]oct-8-yl)carbonyl)-1,1a,2,12b-tetrahydrocyclopropa[d]indolo[2,1-*a*][2]benzazepine-5-carboxylic acid triflouroacetate (412 mg, 0.62 mmol, 84%). The compound was isolated as a mixture of cnantiomcrs and prcscnts as a 1:5 mixture of rotamers or atrope isomers. For major isomer: ¹HNMR (300 MHz, CDCl₃) δ ppm 0.81 - 1.01 (m, 2H), 1.13 - 2.65 (m, 18H), 2.62 (s, 3H) 2.69 - 3.70 (m, 2H), 3.60 (d, *J*= 15.4 Hz, 1H), 3.87 (s, 3H), 4.36 - 5.30 (m, 3H), 6.95 (dd, *J =* 8.8, 2.2 Hz, 1H), 7.09 (d, *J* =2.2 Hz, 1H), 7.28 (d, *J* = 8.8 Hz, 1H), 7.75 (d, *J* = 8.4 Hz, 1H), 7.86 (d, *J* = 8.4 Hz, 1H), 8.08 (s, 1H). LC-MS retention time: 3.24 min; 554 m/z (MH+). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 mL/min, a gradient of 100% solvent A / 0% solvent B to 0% solvent A / 100% solvent B, a gradient time of 4 min, a hold time of 1 min, and an analysis time of 5 min where solvent A was 10% MeOH / 90% H₂O / 0.1 % trifluoroacetic acid and solvent B was 10% H₂O / 90% MeOH / 0.1% trifluoroacetic acid. MS data was determined using a Micromass Platform for LC in electrospray mode.

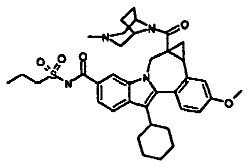

*8-Cyclohexyl-11-methoxy-1a-((3-methyl-3,8-diazabicyclo[3.2.1]oct-8-yl)carbonyl)-N-(propylsulfonyl)-1,1a,2,12b-tetrahydrocyclopropa[d]indolo[2,1-a][2]benzazepine-5-carboxamide.* CDI (21.3 mg, 0.131 mmol) was added to a stirring solution of 8-cyclohexyl-11-methoxy-1a-((3-methyl-3,8-diazabicyclo[3.2.1]oct-8-yl)carbonyl)-1,1a,2,12b-tetrahydrocyclopropa[*d*]indolo[2,1-*a*][2]benzazepine-5-carboxylic acid triflouroacetate (56 mg, 0.10 mmol)) in THF (0.5 mL) and the reaction mixture was heated at 60 °C for 1.5h. The reaction was cooled to rt and propane-1-sulfonamide (16 mg, 0.13 mmol) and then DBU (0.025 mL, 0.15 mmol, as a 20% solution in THF) were added. The reaction was stirred at rt for 1 h and more DBU (-0.025 mL) was added The reaction was stirred 2h at rt, heated at 60 °C for 1 h, and stirred ON at rt. More DBU (0.025 mL) and propane-1-sulfonamide (16.20 mg, 0.131 mmol) were added to the reaction mixture and was stirring continued at rt for 3 days. The reaction was diluted with EtOAc (2 mL) and washed with 1M HCl (2 mL). The organic layer was concentrated to dryness with a stream of nitrogen, dissolved into MeOH (1.5 mL), filtered and purified by preparative HPLC (CH₃CN /H₂O with 10 mM NH₄OAc) to yield 8-cyclohexyl-11-methoxy-1a-((3-methyl-3,8-diazabicyclo[3.2.1]oct-8-yl)carbonyl)-*N*-(propylsulfonyl)-1,1a,2,12b-tetrahydrocyclopropa[*d*]indolo[2,1-*a*][2]benzazepine-5-carboxamide (9.0 mg, 0.014 mmol, 14% yield) as a yellow solid. The compound was isolated as a mixture of enantiomers and presents as a 1:2 mixture of rotamers or atrope isomers. ¹HNMR (500 MHz, CDCl₃) δ ppm -0.37 - -0.20 (m, 0.33H), 0.24 - 0.29 (m, 0.33H), 0.89 - 3.03 (m, 31.33H), 3.54 - 3.64 (m, 2H), 3.89 (s, 2H), 3.90 (s, 1H), 4.04 - 4.67 (m, 1H), 4.74 (d, *J* = 14.7 Hz, 0.33H), 5.19 (d, *J* = 15.0 Hz, 0.67H), 6.93 (dd, *J* = 8.6, 2.4 Hz, 0.33H), 6.96 (dd, *J =* 8.6, 2.4 Hz, 0.67H), 7.00 (d, *J* = 2.4 Hz, 0.33H), 7.12 (d, *J* = 2.4 Hz, 0.67H), 7.29 (d, *J =* 8.6 Hz, 0.67H), 7.30 (d, *J* = 8.6 Hz, 0.33H), 7.55 (d, *J* = 8.2 Hz, 0.33H), 7.64 (br d, *J* = 8.2 Hz, 0.67H), 7.87 (d, *J* = 8.2 Hz, 0.67H), 7.88 (d, *J* = 8.2 Hz, 0.33H), 7.98 (s, 1H). LC-MS retention time: 3.13 min; 659 m/z (MH+). LC data was recorded on a Shimadzu LC-10AS liquid chromatograph equipped with a Phenomenex-Luna 10u C 18 3.0x50mm column using a SPD-10AV UV-Vis detector at a detector wave length of 220nM. The elution conditions employed a flow rate of 5 mL/min, a gradient of 100% solvent A / 0% solvent B to 0% solvent A / 100% solvent B, a gradient time of 4 min, a hold time of 1 min, and an analysis time of 5 min where solvent A was 10% MeOH / 90% H₂O / 0.1% trifluoroacetic acid and solvent B was 10% H₂O / 90% MeOH / 0.1% trifluoroacetic acid. MS data was determined using a Micromass Platform for LC in electrospray mode.

### Reference example

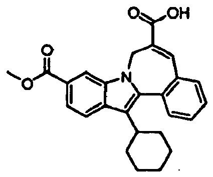

*13-cyclohexyl-10-(methoxycarbonyl)-7H-indolo[2,1-a][2]benzazepine-6-carboxylic acid* Tetrabutylammoniumhydroxide (9.1 mL, 40% solution in water) was added dropwise to a cooled solution (0° C, ice bath) containing dimethyl 13-cyclohexyl-7H-indolo[2,1-a][2]benzazepine-6,10-dicarboxylate and THF (463 mL). The solution was maintained with continued cooling for 50 min. and then concentrated to a volume of about 50 mL. The resultant solution was diluted with ethyl acetate (250 mL), washed with aq. HCl (0.5 N, 3 x 150 mL), washed with brine (150 mL), dried (magnesium sulfate), filtered, and concentrated to afford a yellow solid. LCMS: retention time: 1.698 min. LC data was recorded on a Shimadzu LC-10AS liquid chromatograph equipped with a Phenomenex-Luna 10u C18 4.6x50mm column using a SPD-10AV UV-Vis detector at a detector wave length of 220nM. The elution conditions employed a flow rate of 4 mL/min, a gradient of 100% solvent A / 0% solvent B to 0% solvent A / 100% solvent B, a gradient time of 4 min, a hold time of 1 min, and an analysis time of 5 min where solvent A was 5% CH₃CN / 95% H₂O / 10 mM ammonium acetate and solvent B was 5% H₂O / 95% CH₃CN /10 mM ammonium acetate. MS data was determined using a Micromass Platform for LC in electrospray mode: m/z 416(MH⁺). ¹H NMR (300 MHz, DMSO-D6) δ ppm: 12.97 (s, 1 H), 8.17 (m, 1 H), 7.91 (m, 2 H), 7.63 (m, 5 H), 5.56 (s, 1 H), 4.51 (m, 1 H), 3.89 (m, 3 H), 2.80 (m, 1 H), 1.99 (m, 6 H), 1.30 (m, 4 H).

### Reference example

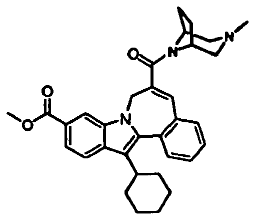

*Methyl 13-cyclohexyl-6-((3-methyl-3,8-diazabicyclo[3.2.1]oct-8-yl)carbonyl)-7H-indolo[2,1-a][2]benzazepine-10-carboxylate.* TBTU (115 mg, 0.361 mmol) was added at rt, in one portion, to a solution containing 13-cyclohexyl-10-(methoxycarbonyl)-7H-indolo[2,1-a][2]benzazepine-6-carboxylic acid (100 mg, 0.241 mmol), DIEA (0.17 mL, 0.964 mmol), 3-Methyl-3,8-diazabicyclo[3.2.1]octane dihydrochloride (115 mg, 0.289 mmol), and DMF (2.4 mL). The solution was maintained for 18 h and concentrated. The resultant residue was charged with dichloromethane (30 mL), washed with water (4 x 15 mL), washed with brine (15 mL), dried (magnesium sulfate), filtered and concentrated to afford a yellow solid which was used without further purification in the next step. LCMS: retention time: 2.272 min LC data was recorded on a Shimadzu LC-10AS liquid chromatograph equipped with a Phenomenex-Luna 10u C18 4.6x50mm column using a SPD-10AV UV-Vis detector at a detector wave length of 220nM. The elution conditions employed a flow rate of 4 mL/min, a gradient of 100% solvent A / 0% solvent B to 0% solvent A / 100% solvent B, a gradient time of 4 min, a hold time of 1 min, and an analysis time of 5 min where solvent A was 5% CH₃CN / 95% H₂O / 10 mM ammonium acetate and solvent B was 5% H₂O / 95% CH₃CN / 10 mM ammonium acetate. MS data was determined using a Micromass Platform for LC in electrospray mode: m/z 524(MH⁺). Crude ¹H NMR (300 MHz, CDCl₃) δ ppm: 8.19 (s, 1 H), 7.92 (d, J=8.4 Hz, 1 H), 7.77 (dd, J=8.78 Hz, J=1.46 Hz, 1H), 7.61 (m, 1 H), 7.49 (m, 3 H), 6.96 (s, 1 H), 5.2 (s, 1 H), 4.41 (s, 1 H), 2.81 (m, 4 H), 2.51 (s, 1 H), 1.95 (m, 15 H), 1.29 (m, 11 H), 0.76 (m, 2 H).

### Reference example

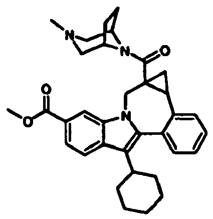

*Methyl 8-cyclohexyl-1a-((3-methyl-3,8-diazabicyclo[3.2.1]oct-8-yl)carbonyl)-1,1a,2,12b-tetrahydrocyclopropa[d]indolo[2,1-a][2]benzazepine-5-carboxylate.* Sodium hydride (60% dispersion, 573 mg, 14.3 mmol) was added at rt to a stirred suspension containing trimethylsulfoxonium iodide (3.15 g, 14.3 mmol) in anhydrous DMSO (6.9 mL) under nitrogen. The resultant mixture was stirred at rt for 1h. Methyl 13-cyclohexyl-6-((3-methyl-3,8-diazabicyclo[3.2.1]oct-8-yl)carbonyl)-7H-indolo[2,1-a][2]benzazepine-10-carboxylate (1.5 g, 2.86 mmol) was then added in small portions. The suspension was diluted with DMSO (2 mL) and then heated with stirring at 90°C for 1.5 h. The reaction mixture was allowed to cool to rt and water was added (1 mL). The mixture was poured into water (40 mL) and extracted with ethyl acetate (3 x 50 mL). The combined organic layers were washed with brine (20 mL), dried (magnesium sulfate), filtered and concentrated to afford a yellow solid which was used without further purification. LCMS: retention time: 2.238 min LC data was recorded on a Shimadzu LC-10AS liquid chromatograph equipped with a Phenomenex-Luna 10u C18 4.6x50mm column using a SPD-10AV UV-Vis detector at a detector wave length of 220nM. The elution conditions employed a flow rate of 4 mL/min, a gradient of 100% solvent A / 0% solvent B to 0% solvent A / 100% solvent B, a gradient time of 4 min, a hold time of 1 min, and an analysis time of 5 min where solvent A was 5% CH₃CN / 95% H₂O / 10 mM ammonium acetate and solvent B was 5% H₂O / 95% CH₃CN / 10 mM ammonium acetate. MS data was determined using a Micromass Platform for LC in electrospray mode:m/z 538 (MH⁺).

### Reference example

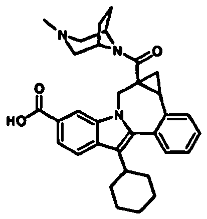

*8-cyclohexyl-1a-((3-methy*/*-3,8-diazabicyc*/*o[3.2.1]oct-8-yl)carbonyl)-1,1a,2,12b-tetrahydrocyclopropa[d]indol[2,1-a][2]benzazepine-5-carboxy*/*ic acid.* Boron tribromide (1.0 M in CH₂Cl₂, 9.6 mL) was added dropwise to a cooled solution (-20° C , dry ice/acetone) containing methyl 8-cyclohexyl-1a-((3-methyl-3,8-diazabicyclo[3.2.1]oct-8-yl)carbonyl)-1,1a,2,12b-tetrahydrocyclopropa[d]indolo[2,1-a][2]benzazepine-5-carboxylate and dichloromethane (17 mL). The mixture was stirred with continued cooling for 10 min., removed from cooling, and stirred at ambient temperature for 1.5h. The solution was cooled again (0° C, ice bath) and quenched with water (2 mL). The resultant mixture was diluted with ethyl acetate (50 mL) followed by slow addition of aqueous, saturated sodium bicarbonate (50 mL), and stirred for 30 minutes. The resulting biphasic mixture was partitioned, the organic layer washed with water (3 x10mL), washed with brine (10 mL), dried (magnesium sulfate), filtered and concentrated to afford a yellow solid.. LCMS: retention time: 2.187 min LC data was recorded on a Shimadzu LC-10AS liquid chromatograph equipped with a Phenomenex-Luna 10u C18 4.6x50mm column using a SPD-10AV UV-Vis detector at a detector wave length of 220nM. The elution conditions employed a flow rate of 4 mL/min, a gradient of 100% solvent A / 0% solvent B to 0% solvent A / 100% solvent B, a gradient time of 4 min, a hold time of I min, and an analysis time of 5 min where solvent A was 5% CH₃CN / 95% H₂O / 10 mM ammonium acetate and solvent B was 5% H₂O / 95% CH₃CN / 10 mM ammonium acetate. MS data was determined using a Micromass Platform for LC in electrospray mode: m/z 524 (MH⁺).

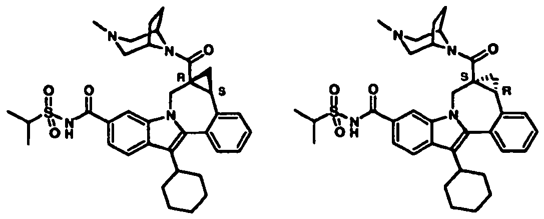

*(1aR,12bS)-8-cyclohexyl-N-(isopropylsulfonyl)-1a-((3-methyl-3,8-diazabicyclo[3.2.1]oct-8-yl)carbonyl)-1,1a,2,12b-tetrahydrocyclopropa[d]indolo[2,1-a][2]benzazepine-5-carboxamide* and *(1aS,12bR)-8-cyclohexyl-N-(isopropylsulfonyl)-1a-((3-methyl-3,8-diazabicyclo[3.2.1]oct-8-yl)carbonyl)-1,1a,2,12b-tetrahydrocyclopropa[d]indolo[2,1-a][2]benzazepine-5-carboxamide.* EDCI (275 mg, 1.43 mmol) was added at rt, in one portion, to a solution containing 8-cyclohexyl-1a-((3-methyl-3,8-diazabicyclo[3.2.1]oct-8-yl)carbonyl)-1,1a,2,12b-tetrahydrocyclopropa[d]indolo[2,1-a][2]benzazepine-5-carboxylic acid (500 mg, 0.955 mmol), DMAP (58 mg, 0.477 mmol), 2-isopropyl sulfonamide (176 mg, 1.43 mmol) and dichloromethane (9.5 mL). The solution was maintained at rt for 24 h, diluted with additional dichloromethane (50 mL), washed with aqueous saturated sodium bicarbonate (2 x 20 mL), washed with water (20 mL), dried (magnesium sulfate), filtered and concentrated. The resulting orange residue was purified by preparative reverse phase HPLC to afford a yellow solid as a TFA salt. LCMS: retention time: 2.380 min LC data was recorded on a Shimadzu LC-10AS liquid chromatograph equipped with a Phenomenex-Luna 10u C18 4.6x50mm column using a SPD-10AV UV-Vis detector at a detector wave length of 220nM. The elution conditions employed a flow rate of 4 mL/min, a gradient of 100% solvent A / 0% solvent B to 0% solvent A / 100% solvent B, a gradient time of 4 min, a hold time of 1 min, and an analysis time of 5 min where solvent A was 5% CH₃CN / 95% H₂O / 10 mM ammonium acetate and solvent B was 5% H₂O / 95% CH₃CN / 10 mM ammonium acetate. MS data was determined using a Micromass Platform for LC in electrospray mode: m/z 629 (MH⁺). Enantiomers were separated using chiral HPLC on a ChiralPak AS-H, 5 micron, 4.6 x 250 mm column (12% MeOH/88% CO₂ mobile phase). Retention times: 12.60 min. and 19.05 min.. NMR (300 MHz, MeOD) δ ppm: 8.01 (m, 2H), 7.65(m, 2H), 7.47 (m, 3H), 5.09 (m, 1H), 4.70 (s, 1H), 4.26 (m, 1H), 3.97 (m, 1H). 3.70 (m, 1H), 3.56 (m, 0.5H), 3.44 (t, 2H), 3.30 (m 0.5H), 3.20-2.55 (overlapping series of multiplets, 6H), 2.53-1.7 (overlapping series of multiplets, 11H), 1.6-0 (overlapping series of multiplets, 12H).

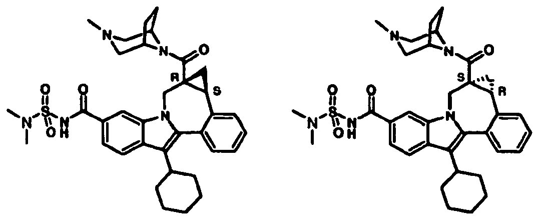

*(1aR,12bS)-8-cyclohexyl-N-(dimethylsulfamoyl)-1a-((3-methyl-3,8-diazabicyclo[3.2.1]oct-8-yl)carbonyl)-1,1a,2,12b-tetrahydrocyclopropa[d]indolo[2,1-a][2]benzazepine-5-carboxamide and (1aS,12bR)-8-cyclohexyl-N-(dimethylsulfamoyl)-1a-((3-methyl-3,8-diazabicyclo[3.2.1]oct-8-yl)carbonyl)-1,1a,2,12b-tetrahydrocyclopropa[d]indolo[2,1-a][2]benzazepine-5-carboxamide.* These compounds were prepared from 8-cyclohexyl-1a-((3-methyl-3,8-diazabicyclo[3.2.1]oct-8-yl)carbonyl)-1,1a,2,12b-tetrahydrocyclopropa[d]indolo[2,1-a][2]benzazepine-5-carboxylic acid under similar conditions to those described for (1aR,12bS)-8-cyclohexyl-N-(isopropylsulfonyl)-1a-((3-methyl-3,8-diazabicyclo[3.2.1]oct-8-yl)carbonyl)-1,1a,2,12b-tetrahydrocyclopropa[d]indolo[2,1-a][2]benzazepine-5-carboxamide and (1aS,12bR)-8-cyclohexyl-N-(isopropylsulfonyl)-1a-((3-methyl-3,8-diazabicyclo[3.2.1]oct-8-yl)carbonyl)-1,1a,2,12b-tetrahydrocyclopropa[d]indolo[2,1-a][2]benzazepine-5-carboxamide. The resulting yellow residue was purified by preparative reverse phase HPLC to afford a yellow solid as a TFA salt. LCMS: retention time: 2.746 min LC data was recorded on a Shimadzu LC-10AS liquid chromatograph equipped with a Phenomenex-Luna 10u C18 4.6x50mm column using a SPD-10AV UV-Vis detector at a detector wave length of 220nM. The elution conditions employed a flow rate of 4 mL/min, a gradient of 100% solvent A / 0% solvent B to 0% solvent A / 100% solvent B, a gradient time of 4 min, a hold time of 1 min, and an analysis time of 5 min where solvent A was 5% CH₃CN / 95% H₂O / 10 mM ammonium acetate and solvent B was 5% H₂O / 95% CH₃CN / 10 mM ammonium acetate. MS data was determined using a Micromass Platform for LC in electrospray mode: m/z 630 (MH⁺). Enantiomers were separated using chiral HPLC on a ChiralPak AS-H, 5 micron, 4.6 x 250 mm column (12% MeOH/88% CO₂ mobile phase). Retention times: 13.16 min and 20.63 min. All of the compounds and examples which follow were analyzed by the following LC method: Column: PHENOMENNEX-LUNA 3.0 x 50mm S10; Mobile Phase: (A) 10:90 methanol-water; (B) 90:10 methanol-water, Buffer 0.1% TFA; Gradient Range: 0-100% B; Gradient Time: 2 min; Flow Rate: 4 mL/min; Analysis Time: 3 min; Detection: Detector 1: UV at 220 nm; Detector 2: MS (ESI+).

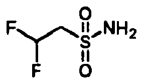

Neat potassium thioacetate (1.371 g, 12.00 mmol) was added to a stirred solution of 2-bromo-1,1-difluoroethane (1.45 g, 10.00 mmol) in DMF (6 ml) and the mixture stirred overnight. Product was extracted with ether (2X25 ml), washed with water, brine and dried (Na2SO4). Evaporation of ether gave the intermediate, HCF2CH2SCOCH3 (1.18 g, 84.3%) as a light brown oil which was dissolved in DCM (10 ml) and mixed with water (10 ml). Chlorine gas was bubbled into stirred cold (0 °C) two-phase solution of HCF2CH2SCOCH3 (1.18 g) untill permanent green color is persistent and maintained for 1-2 h. DCM layer was separated and washed with 10% NaHSO3, water, brine and dried (MgSO4). Evaporation of DCM gave the intermediate HCF2CH2SO2Cl (1.13 g) as a light yellow oil which was treated with an. NH3 (0.5 M in dioxane, 40 ml) at 0°C for 1 h. Precipitated NH4Cl was filtered through a plug of silica gel and then filtrate was evaporated to dryness to afford 2,2-difluoroethanesulfonamide as a light-yellow oil (736 mg). 1H NMR (400 MHz, CHLOROFORM-D) δ ppm ~2.04 (brd s, 2H), 3.78 (m, 2H), 5.23 (m, 1H).

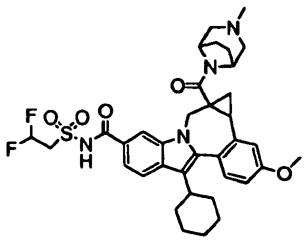

*(+*/*-)-8-cyclohexyl-N-(2,2-difluoroethylsulfonyl)-1,1a,2,12b-tetrahydro-11-methoxy-1a-(3-methyl-3,8-diazabicyclo[3.2.1]octane-8-carbonyl)cycloprop[d]indolo[2,1-a][2]benzazepine-5-carboxamide.* Neat CDI (24.60 mg, 0.152 mmol) was added to a stirred solution of acid-amide (56 mg, 0.101 mmol) in an. THF (1 ml) and the mixture was heated at 50°C for 30 min and allowed to cooled to rt. Then 2,2-difluoroethanesulfonamide (29.4 mg, 0.202 mmol) and DBU (0.046 ml, 0.303 mmol) were added consecutively and the mixture was sonicated 2-3 h. Rxn was quenched with methanol (1 ml) and acidified with few drops of TFA and purified by reverse-phase prep. HPLC to afford the product and isolated in mono TFA salt as a beige solid. LC/MS: Retention time: 2.861 min; m/e 681 (MH⁺). The product was observed to exist as inter-converting rotamers by 1H NMR (500 MHz, CHLOROFORM-D) δ ppm 1.12 - 1.31 (m, J=4.27 Hz, 3 H), 1.32 - 1.55 (m, 4 H), 1.63 - 1.87 (m, 3 H), 1.87 - 2.11 (m, 5 H), 2.14 - 2.34 (m, 1 H), 2.34 - 2.90 (m, 8 H), 2.91 - 3.06 (m, 2 H), 3.35 - 3.54 (m, 1 H), 3.56 - 3.70 (m, J=14.95 Hz, 1 H), 3.83 - 3.95 (m, 3 H), 3.93 - 4.14 (m, 1 H), 4.14 - 4.34 (m, 1 H), 4.36 - 4.64 (m, 1 H), 5.11 - 5.30 (m, 1 H), 6.11 - 6.41 (m, 1 H), 6.94 - 7.02 (m, J=8.39, 2.59 Hz, 1 H), 7.08 - 7.15 (m, J=2.44 Hz, 1 H), 7.27 - 7.33 (m, 1 H), 7.56 - 7.72 (m, 1 H), 7.89 - 7.97 (m, J=8.55 Hz, 1 H), 7.99 - 8.08 (m, 1 H).

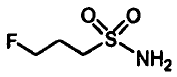

A solution of methanesulfonyl chloride (1.714 ml, 22.00 mmol) in DCM (5 ml) was added dropwise to a stirred cold (0 °C) solution of 3-fluoropropan-1-ol (1.562 g, 20 mmol) and TEA (3.35 ml, 24.00 mmol) in DCM (20 ml). The mixture was stirred at 0-5 °C for ~2 h and washed with water, satd. NaHCO3, water, brine and dried (MgSO4). Evaporation of DCM gave 3-fluoropropyl methanesulfonate as a colorless oil (2.92 g, 93.6%). Neat potassium thioacetate (2.74 g, 24.00 mmol) was added to a stirred solution of 3-fluoropropyl methanesulfonate (2.9 g) in DMSO (20 ml) and the mixture stirred overnight. Product was extracted with ether (2X25 ml), washed with water, brine and dried (MgSO4). Evaporation of ether gave S-3-fluoropropyl ethanethioate as a light brown oil. Chlorine gas was bubbled into cold (-10 °C) stirred two-phase solution of S-3-fluoropropyl ethanethioate (1.0 g, 7.34 mmol) in DCM (5 ml) and Water (5.00 ml) until green color appeared in aqueous phase and maintained for 1-2 h. Layers separated and aq. layer re-extracted with DCM (2X10 ml). Combined DCM extracts were washed with 10% NaHSO3 solution, water, brine and dried (MgSO4). Evaporation of DCM gave the intermediate, 3-fluoropropane-1-sulfonyl chloride as a light brown oil (405 mg, 34%) which was treated with an. NH3 solution (10 ml, 0.5M in dioxane) and stirred for 30-45 min. Precipitated NH4Cl was filtered off and the filtrate was evaporated to dryness to afford 3-fluoropropane-1-sulfonamide as a light brown oil (265 mg).

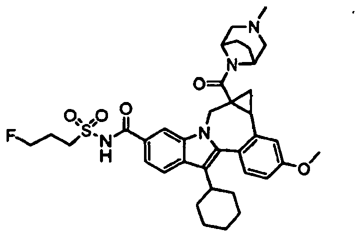

*(+*/*-)-8-cyclohexyl-N-(3-fluoropropylsulfonyl)-1,1a,2,12b-tetrahydro-11-methoxy-1a-(3-methyl-3,8-diazabicyclo[3.2.1]octane-8-carbonyl)cycloprop[d]indolo[2,1-a][2]benzazepine-5-carboxamide.* Neat CDI (24.60 mg, 0.152 mmol) was added to a stirred solution of acid-amide (56 mg, 0.10 mmol) in an. THF (1 ml) and the mixture was heated at 50 °C for 30 min and allowed to cool to rt. Then 3-fluoropropane-1-sulfonamide (28.6 mg, 0.202 mmol) and DBU (0.046 ml, 0.303 mmol) were added consecutively and the mixture was sonicated 2-3 h. Rxn was quenched with methanol (1 ml) and acidified with few drops of TFA and purified by reverse-phase prep. HPLC to afford the product and isolated in mono TFA salt form as a beige solid. LC/MS: Retention time: 2.835 min; m/e 677 (MH⁺). The product was observed to exist as inter-converting rotamers by 1H NMR (400 MHz, CHLOROFORM-D) δ ppm 0.13 - 0.37 (m, 1 H), 1.08 - 1.31 (m, 2 H), 1.31 - 1.61 (m, 4 H), 1.70 - 1.86 (m, 2 H), 1.86 - 2.11 (m, 6 H), 2.13 - 2.36 (m, 3 H), 2.38 - 2.68 (m, 1 H), 2.69 - 3.01 (m, 3 H), 3.03 - 3.34 (m, J=68.99 Hz, 1 H), 3.35 - 3.84 (m, 7 H), 3.84 - 3.95 (m, 3 H), 3.95 - 4.12 (m, 1 H), 4.44 - 4.57 (m, J=5.20, 5.20, 5.20 Hz, 2 H), 4.58 - 4.72 (m, J=5.29, 5.29 Hz, 2 H), 5.03 - 5.30 (m, 1 H), 6.91 - 7.02 (m, 1 H), 7.08 - 7.15 (m, J=2.27 Hz, 1 H), 7.27 - 7.35 (m, 1 H), 7.52 - 7.69 (m, J=31.98 Hz, 1 H), 7.85 - 7.97 (m, J=7.93, 7.93 Hz, 1 H), 8.04 - 8.13 (m, 1 H).

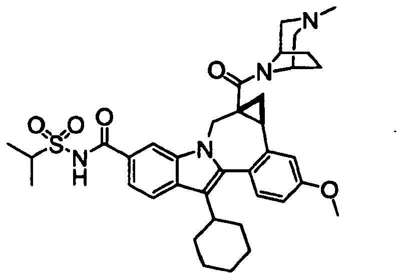

*(-)-8-cyclohexyl-N-((2S,6R)-propane-2-sulfonyl)-1,1a,2,12b-tetrahydro-11-methoxy-1a-(3-methyl-3,8-diazabicyclo[3.2.1]octane-8-carbonyl) cycloprop[d]indolo[2,1-a][2]benzazepine-5-carboxamide.* To a mixture of acid-amide (341 mg, 0.62 mmol) and 3-methyl-3,8-diazabicyclo[3.2.1]octane, 2HCl (148 mg, 0.743 mmol) in dichloromethane (5 ml) was added TEA (0.259 ml, 1.858 mmol) and HBTU (282 mg, 0.743 mmol). The mixture was stirred at room temperature overnight. Diluted with EtOAc (150 ml) and washed with aqueous 0.3 M NaHCO3 solution (2x10 mL), water brine, dried (MgSO4), removed the solvent and purified by Biotage 25M column (MeOH/DCM:0 to 25%). The collection was dissolved in 100 mL EtOAc and washed with aqueous HCl (3x20ml, 0.1M), 0.3 M NaHCO3 solution, brine, dried (MgSO4), removed the solvent to afford the product as a yellow solid (0.280 g, 68%). LC-MS retention time: 3.105; MS m/z (M+H) 659. The product was observed to exist as inter-converting rotamers by 1H NMR (400 MHz, McOD) δ ppm 1.37 (10 H, m), 2.37 (15 H, m), 3.76 (8 H, m), 3.88 (3 H, m), 4.36 (2 H, m), 5.06 (1 H, m), 6.97 (1 H, m), 7.17 (1 H, d, J=1.76 Hz), 7.27 (1 H, m), 7.55 (1 H, d, J=8.56 Hz), 7.85 (1 H, m), 7.98 (1 H, br. s.).

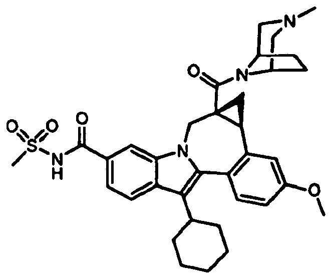

*8-cyclohexyl-N-(methylsulfonyl)-1,1a,2,12b-tetrahydro-11-methoxy-1a-(3-methyl-3,8-diazabicyclo[3.2.1]octane-8-carbonyl) cycloprop[d]indolo[2,1-a][2]benzazepine-5-carboxamide.* Step 1: The racemate was separated by using ChiralCel OJ-H column (15% EtOH/85% CO2) to afford optically pure enantiomers. Step 2: An enantiomer was hydrolyzed to afford the corresponding acid: LC-MS retention time: 3.426; MS m/z (M+H) 523. Step 3: Amide derivative was purified by prep. HPLC and isolated as a TFA salt. LC-MS retention time: 2.913; MS m/z (M+H) 631. The product was observed to exist as inter-converting rotamers. 1H NMR (400 MHz, *CHLOROFORM-d*) δ ppm 1.36 (6 H, m), 1.7-3.2 (16 H, m), 3.48 (2 H, m), 3.43 (3 H, s), 3.67 (2 H, m); 3.91 (3 H, m), 4.59 (2 H, m), 5.24 (1 H, m), 6.96 (1 H, m), 7.12 (1 H, d, *J*=2.52 Hz), 7.27 (1 H, m), 7.62 (1 H, m), 7.90 (1 H, m), 8.22 (1 H, br. s.).

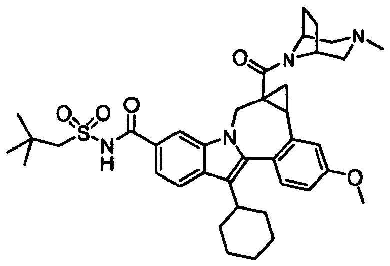

*(+*/*-)-8-cyclohenyl-N-(2,2-dimethylpropane-1-sulfonyl)-1,1a,2,12b-tetrahydro-11-methoxy-1a-(3-methyl-3,8-diazabicyc*/*o[3.2.1]octane-8-carbonyl) cycloprop[d]indolo[2,1-a][2]benzazepine-5-carboxamide.* The starting material was purified by Prep HPLC and isolated as a TFA salt. LC-MS retention time: 3.263 min; MS m/z (M+H) 687. The product was observed to exist as inter-converting rotamers. 1H NMR (500 MHz, CHLOROFORM-d) δ ppm 1.21 (9 H, s), 1.41 (5 H, m), 1.99 (11 H, m), 2.40-4.50 (14 H, m), 3.89 (3 H, s), 5.17 (1 H, m), 6.97 (1 H, m), 7.11 (1 H, d, J=2.44 Hz), 7.30 (1 H, m), 7.51 (1 H, br. s.), 7.89 (1 H, d, J=7.93 Hz), 8.07 (1 H, m).

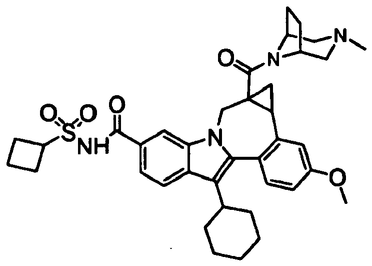

*(+*/*-)-8-cyclohexyl-N-cyclobutanesumonyl-1,1a,2,12b-tetrahydro-11-methoxy-1a-(3-methyl-3,8-diazabicyclo[3.2.1]octane-8-carbonyl) cycloprop[d]indolo[2,1-a][2]benzazepine-5-carboxamide*. The product was purified by Prep HPLC and isolated as a TFA salt. LC-MS retention time:3.138 min; MS m/z (M+H) 671. The product was observed to exist as inter-converting rotamers. 1H NMR (400 MHz, CHLOROFORM-d) δ ppm 1.37 (7 H, m), 2.01 (10 H, m), 2.63 (14 H, m), 3.46 (1 H, d), 3.62 (1 H, d, J=15.11 Hz), 3.89 (3 H, s), 4.59 (2 H, m), 5.18 (1 H, m), 6.97 (1 H, m), 7.11 (1 H, d, J=2.52 Hz), 7.29 (1 H, m), 7.62 (1 H, m), 7.89 (1 H, m), 8.05 (1 H, s).

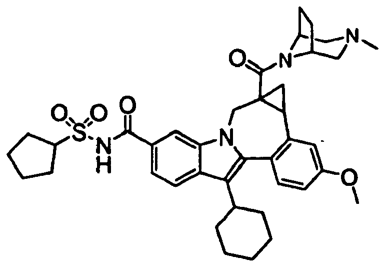

*(+*/*-)-8-cyclohexyl-N-cyclopentanesulfonyl-1,1a,2,12b-tetrahydro-11-methoxy-1a-(3-methyl-3,8-diazabicyclo[3.2.1]octane-8-carbonyl) cycloprop[d]indolo[2,1-a][2]benzazepine-5-carboxamide.* The product was purified by Prep HPLC and isolated as a TFA salt. LC-MS retention time: 3.190 min; MS m/z (M+H) 685.

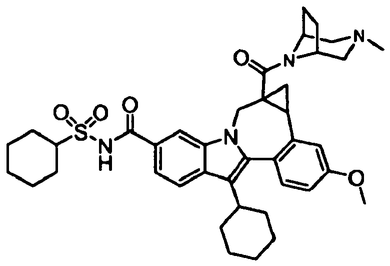

*(+*/*-)-8-cyclohexyl-N-cyclohexanesulfonyl-1,1a,2,12b-tetrahydro-11-methoxy-1a-(3-methyl-3,8-diazabicyclo[3.2.1]octane-8-carbonyl) cycloprop[d]indolo[2,1-a][2]benzazepine-5-carboxamide.* The product was purified by Prep HPLC and isolated as a TFA salt. LC-MS retention time: 3.261 min; MS m/z (M+H) 699. The product was observed to exist as inter-converting rotamers. 1H NMR (400 MHz, CHLOROFORM-d) δ ppm 1.61 (22 H, m), 2.86 (12 H, m), 3.48 (1 H, m), 3.63 (1 H, d, J=15.11 Hz); 3.74 (1 H, m), 3.89 (3 H, s), 4.56 (2 H, m), 5.24 (1 H, m), 6.98 (1 H, m), 7.11 (1 H, d, J=2.27 Hz), 7.30 (1 H, m), 7.60 (1 H, m), 7.90 (1 H, m), 8.08 (1 H, br. s.).

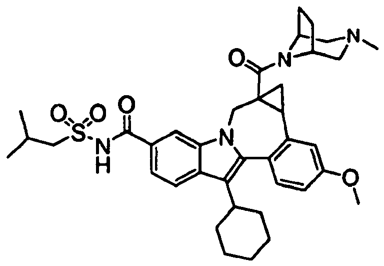

*(+*/*-)-8-cyclohexyl-N-2-methylpropane-1-sulfonyl-1,1a,2,12b-tetrahydro-11-methoxy-1a-(3-methyl-3,8-diazabicyclo[3.2.1]octane-8-carbonyl) cycloprop[d]indolo[2,1-a][2]benzazepine-5-carboxamide.* The product was purified by Prep HPLC and isolated as a TFA salt. LC-MS retention time: 3.210 min; MS m/z (M+H) 673. The product was observed to exist as inter-converting rotamers. 1H NMR (500 MHz, CHLOROFORM-d) δ ppm 1.14 (6 H, m), 1.32 (3 H, m), 1.46 (4 H, m), 1.62 (1 H, m), 1.80 (3 H, m), 2.07 (8 H, m), 2.33 (2 H, m), 2.68 (1 H, m), 2.95 (5 H, m), 3.33 (3 H, m), 3.49 (2 H, m), 3.66 (1 H, d, J=15.26 Hz), 3.89 (3 H, s), 4.21 (1 H, m), 4.65 (1 H, m), 5.15 (0 H, d, J=14.95 Hz), 7.02 (1 H, dd, J=8.55, 2.44 Hz), 7.18 (0 H, d, J=1.83 Hz), 7.32 (1 H, d, J=8.55 Hz), 7.58 (0 H, m), 7.91 (1 H, m), 7.99 (0 H, br. s.).

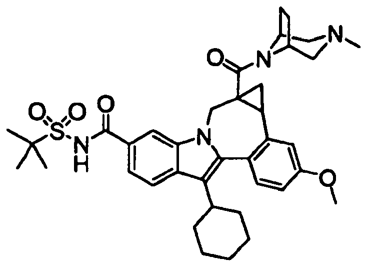

*(+*/*-)-8-cyclohexyl-N-(2-methylpropane-2-sulfonyl)-1,1a,2,12b-tetrahydro-11-methoxy-1a-(3-methyl-3,8-diazabicyclo[3.2.1]octane-8-carbonyl) cycloprop[d]indolo[2,1-a][2]benzazepine-5-carboxamide.* The product was purified by Prep HPLC and further purified by prep TLC. LC-MS retention time: 3.226 min; MS m/z (M+H) 685.

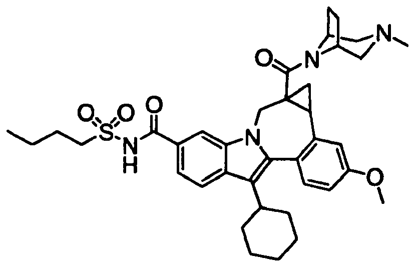

*(+*/*-)-8-cyclohexyl-N-(butanesulfonyl)-1,1a,1,12b-tetrahydro-11-methoxy-1a-(3-methyl-3,8-diazabicyclo[3.2.1]octane-8-carbonyl) cyclyprop[d]indolo[2,1-a][2]benzazepine-5-carboxamide.* The product was purified by Prep HPLC and isolated as a TFA salt LC-MS retention time:3.245 min; MS m/z (M+H) 673. The product was observed to exist as inter-converting rotamers. 1H NMR (400 MHz, CHLOROFORM-d) δ ppm 0.94 (3 H, t, J=7.30 Hz), 1.24 (2 H, m), 1.46 (6 H, m), 1.90 (14 H, m), 2.72 (4 H, m), 3.02 (2 H, m), 3.41 (1 H, m), 3.62 (3 H, m), 3.88 (3 H, s), 4.39 (2 H, m), 5.18 (1 H, m), 6.98 (1 H, m), 7.11 (1 H, d, J=2.52 Hz), 7.30 (1 H, m), 7.62 (1 H, m), 7.90 (1 H, m), 8.05 (1 H, s).

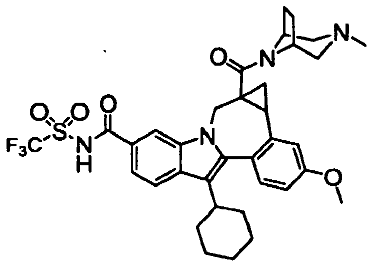

*(+*/*-)-8-cyclohexyl-N-[(+*/*-)-(trifluoromethanesulfonyl)]-1,1a,2,12b-tetrahydro-11-methoxy-1a-(3-methyl-3,8-diazabicyclo[3.2.1]octane-8-carbonyl) cycloprop[d]indolo[2,1-a][2]benzazepine-5-carboxamide.* The product was purified by Prep HPLC and isolated as a TFA salt. LC-MS retention time: 3.203min; MS m/z (M+H) 685. The product was observed to exist as inter-converting rotamers. 1H NMR (400 MHz, CHLOROFORM-d) δ ppm 1.34 (5 H, m), 2.05 (8 H, m), 3.02 (12 H, m), 3.59 (1 H, d, J=15.11 Hz), 3.89 (3 H, s), 4.51 (2 H, m), 5.16 (1 H, m), 6.98 (1 H, m), 7.11 (1 H, d, J=2.27 Hz), 7.29 (1 H, d, J=8.56 Hz), 7.58 (1 H, m), 7.86 (1 H, m), 8.05 (1 H, br. s.).

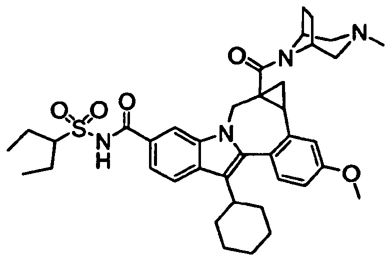

*(+*/*-)-8-cyclohexyl-N-(pentane-3-sulfonyl)-1,1a,2,12b-tetrahydro-11-methoxy-1a-(3-methyl-3,8-diazabicyclo[3.2.1]octane-8-carbonyl) cycloprop[d]indolo[2,1-a][2]benzazepine-5-carboxamide.* The product was purified by Prep HPLC and isolated as a TFA salt. LC-MS retention time: 3.250min; MS m/z (M+H) 687. The product was observed to exist as inter-converting rotamers. 1H NMR (400 MHz, CHLOROFORM-d) δ ppm 1.39 (37 H, m), 2.84 (2 H, m), 3.28 (0 H, br. s.), 3.59 (0 H, m), 3.73 (0 H, m), 3.89 (3 H, m), 4.23 (2 H, m), 5.18 (0 H, br. s.), 6.99 (1 H, m), 7.11 (1 H, br. s.), 7.25 (1 H, m), 7.69 (0 H, br. s.), 7.86 (2 H, br. s.).

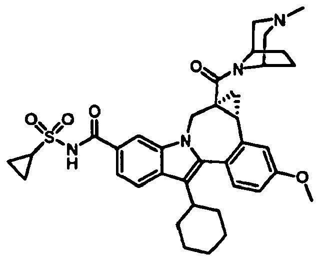

*8-cyclohexyl-N-(cyclopropylsulfonyl)-1,1a,2,12b-tetrahydro-11-methoxy-1a-(3-methyl-3,8-diazabicyclo[3.2.1]octane-8-carbonyl)cycloprop[d]indolo[2,1-a][2]benzazepine-5-carboxamide.* Step 1: The racemic starting material was separated into optically pure enantiomers by using ChiralPak AS-H, 30 x 250mm, 5µm column (15% EtOH / 85% CO2) to afford (-)-enantiomer (first peak) and (+)-enantiomer (second peak). Step 2: To a solution of one isomer (0.397 g, 0.706 mmol) in THF (4 ml) and MeOH (4ml) was added 1N NaOH (2 ml, 2.000 mmol). The mixture was stirred at room for 2 h. Diluted with EtOAc, washed with cold HCl (1N), brine, dried (MgSO₄), and removed the solvent in vacuo to afford compound acid-amide as a yellow solid (0.387 g, 100%). LC-MS retention time: 3.473 min; MS m/z (M+H) 549. Step 3: The diamide was purified and isolated as TFA salt. LC-MS retention time: 2.991 min; MS m/z (M+H) 657.

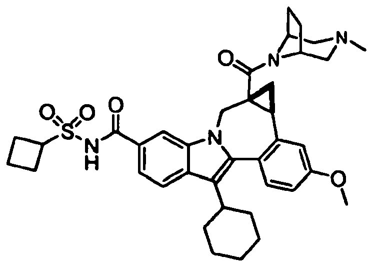

8-cyclohexyl-N-cyclobutanesulfonyl-1,1a,2,12b-tetrahydro-11-methoxy-1a-(3-methyl-3,8-diazabicyclo[3.2.1]octane-8-carbonyl) cycloprop[d]indolo[2,1-a][2]benzazepine-5-carboxamide. Step 1: To a suspension of compound diester (0.63 g, 1.189 mmol) in DCM (1 mL) was added TFA (1 mL, 12.98 mmol), stirred at r.t. for 2.5h, removed the solvents in vacuo to afford compound mono acid as a dark brown solid. LC-MS retention time: 3.686; MS m/z (M+H) 474. Step 2: To a mixture of compound mono acid (100mg, 0.211 mmol), cyclobutanesulfonamide (57.1 mg, 0.422 mmol), DMAP (103 mg, 0.845 mmol), and 1-(3-Dimethylaminopropyl)-3-ethylcarbodiimide hydrochloride (60.7 mg, 0.317 mmol) in a vial was added DCM (1 ml). The mixture was stirred at r.t. overnight and purified by Biotage 25S column (MeOH/DCM: 0 to 25%) to afford compound amide-ester as a yellow solid (0.086 g, 68%). LC-MS retention time: 3.528; MS m/z (M+H) 591. Step 3: To a solution of this compound (86 mg, 0.146 mmol) in THF (2ml) and MeOH (1ml) was added 1N NaOH (1 ml, 1.0 mmol). The mixture was stirred at room for 2 h. Diluted with EtOAc, washed with cold HCl (1N), brine, dried (MgSO₄), and removed the solvent in vacuo to afford mono acid as a brown solid. LC-MS retention time: 3.463; MS m/z (M+H) 563. Step 4: The diamide was purified by prep HPLC and isolated as TFA salt. LC-MS retention time: 3.128; MS m/z (M+H) 671.

## Claims

1. A compound selected from the group consisting of 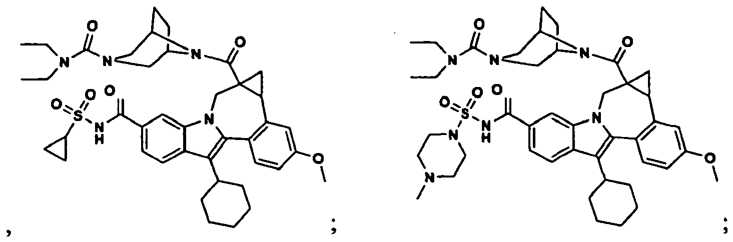 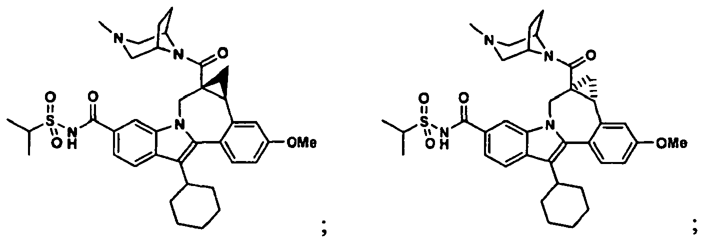 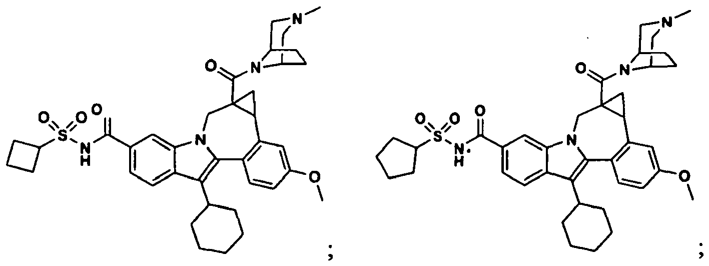 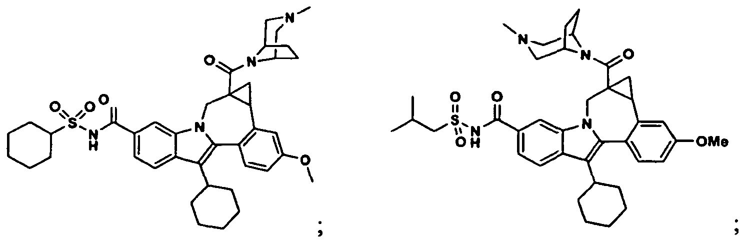 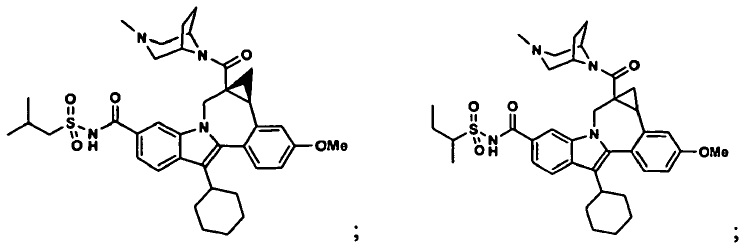 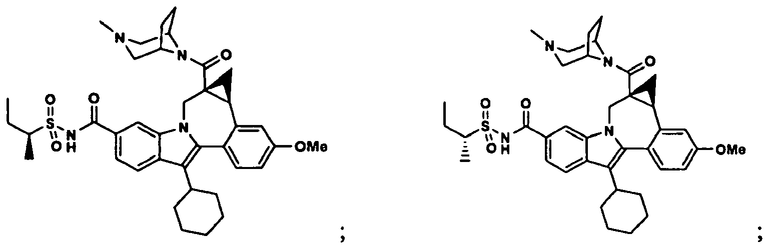 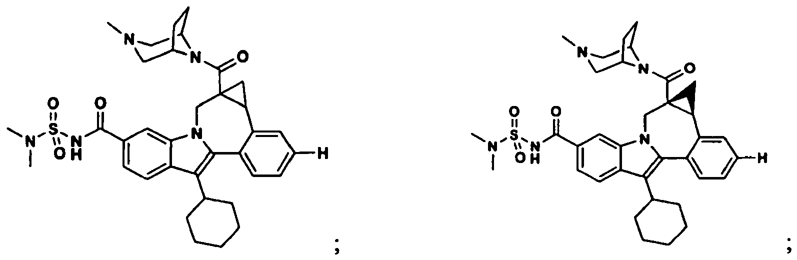 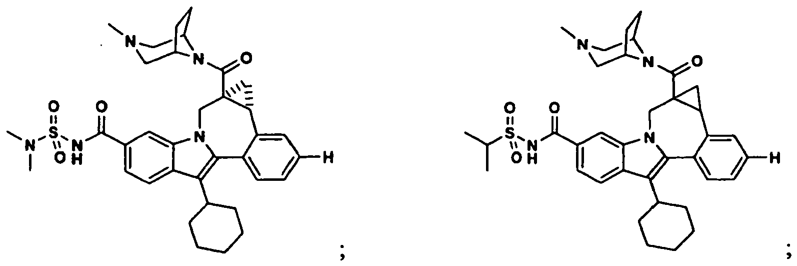 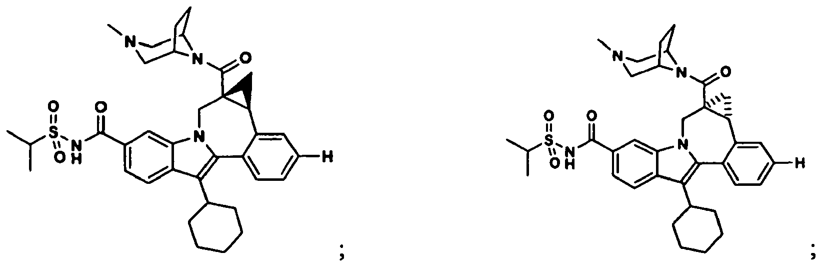 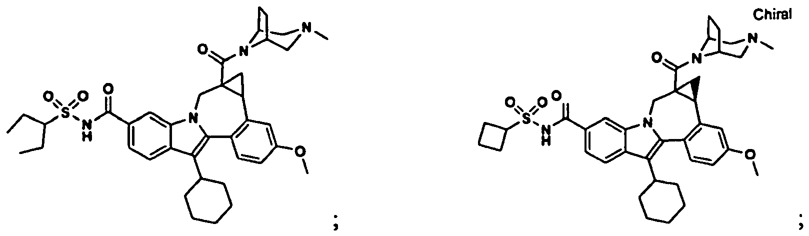 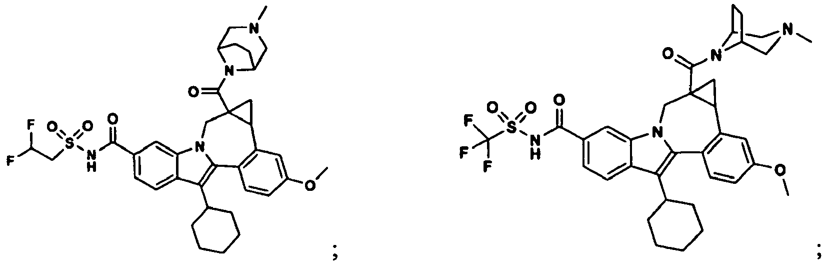 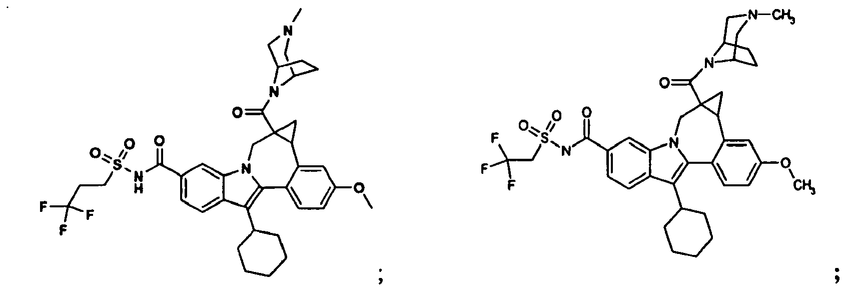 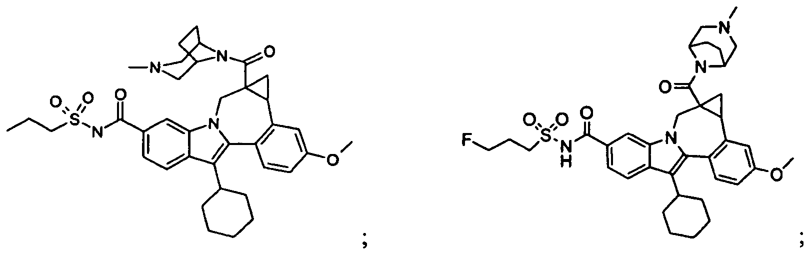 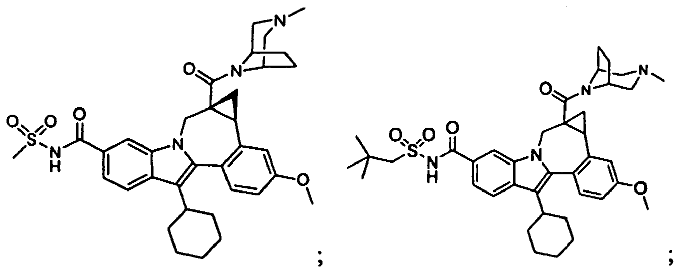 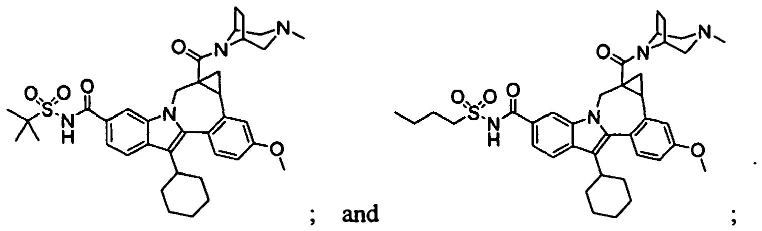 or a pharmaceutically acceptable salt thereof.

2. A compound of claim 1 selected from the group consisting of 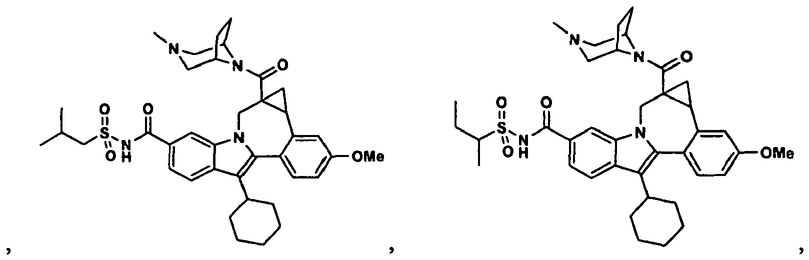 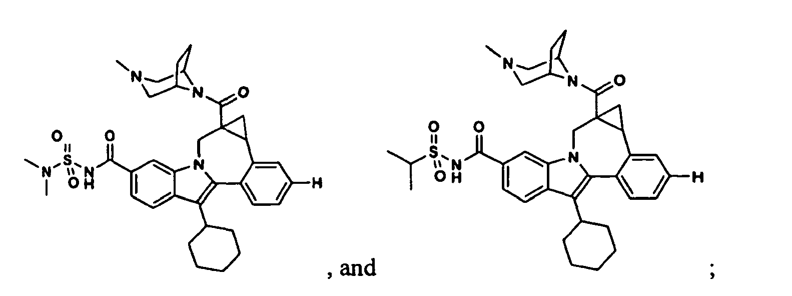 or a pharmaceutically acceptable salt thereof.

3. A compound of claim 1 selected from the group consisting of 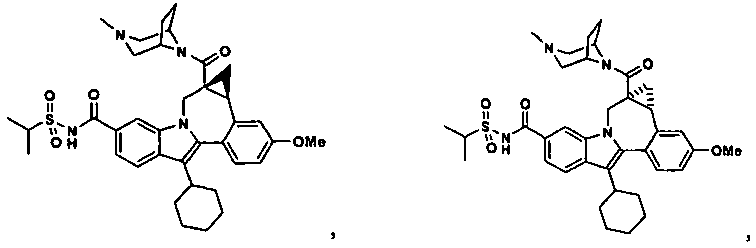 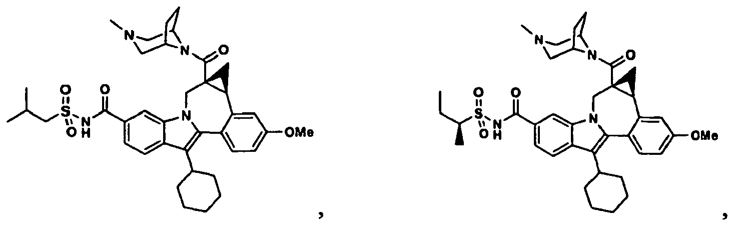 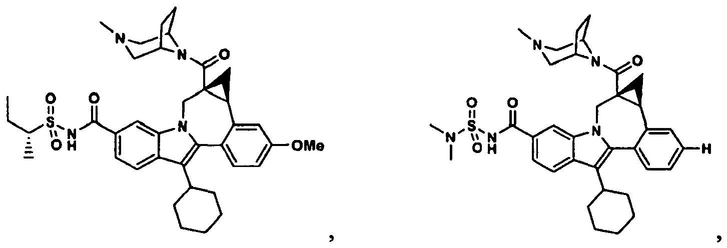 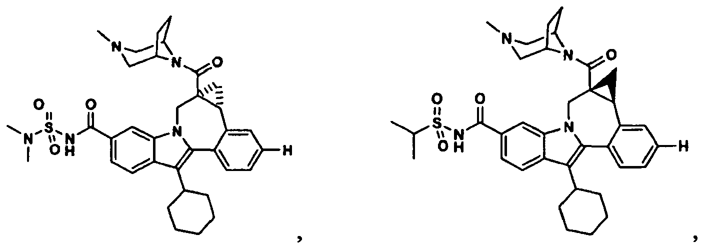 and 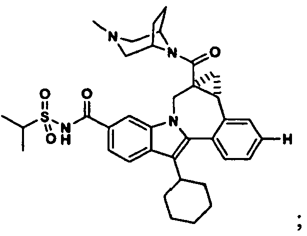 or a pharmaceutically acceptable salt thereof.

4. A composition comprising a compound of any one of claims 1 to 3, or a pharmaceutically acceptable salt thereof, and a pharmaceutically acceptable carrier.

5. The composition of claim 4 further comprising at least one additional compound having therapeutic benefits for HCV wherein the compound is selected from the group consisting of interferons, cyclosporins, interleukins, HCV metalloprotease inhibitors, HCV serine protease inhibitors, HCV polymerase inhibitors, HCV helicase inhibitors, HCV NS4B protein inhibitors, HCV entry inhibitors, HCV assembly inhibitors, HCV egress inhibitors, HCV NS5A protein inhibitors, HCV NS5B protein inhibitors, and HCV replicon inhibitors.

6. A compound of any one of claims 1 to 3 for use in treating hepatitis C infection.

7. The compound of claim 6 further comprising administering at least one additional compound having therapeutic benefits for HCV wherein the compound is selected from the group consisting of interferons, cyclosporins, interleukins, HCV metalloprotease inhibitors, HCV serine protease inhibitors, HCV polymerase inhibitors, HCV helicase inhibitors, HCV NS4B protein inhibitors, HCV entry inhibitors, HCV assembly inhibitors, HCV egress inhibitors, HCV NS5A protein inhibitors, HCV NS5B protein inhibitors, and HCV replicon inhibitors.

## Patentansprüche

1. Verbindung, ausgewählt aus der Gruppe, bestehend aus 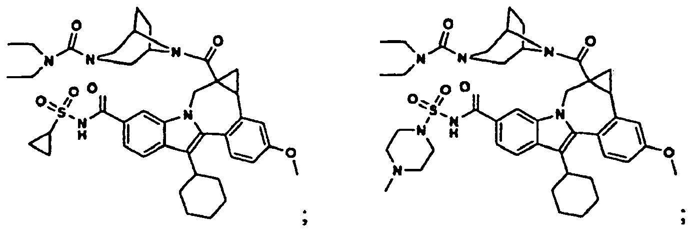 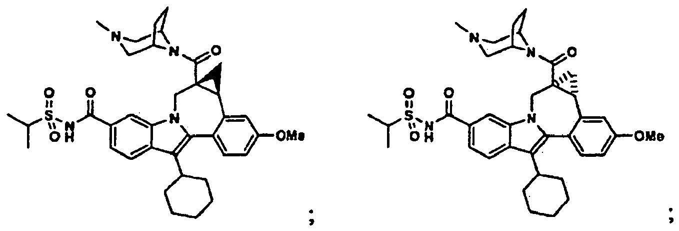 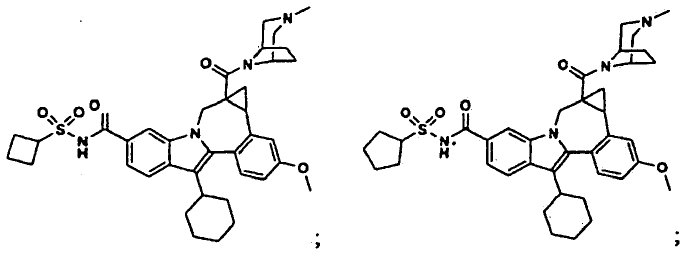 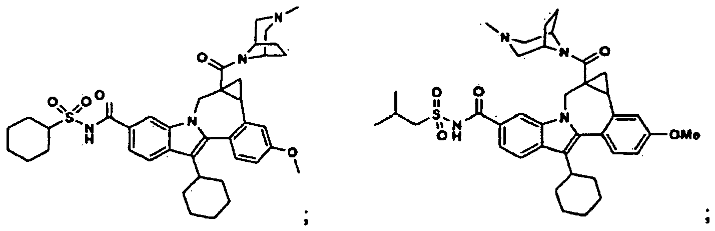 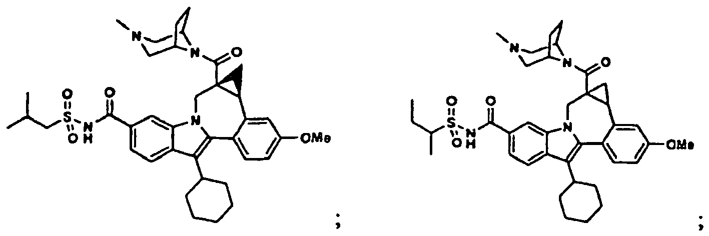 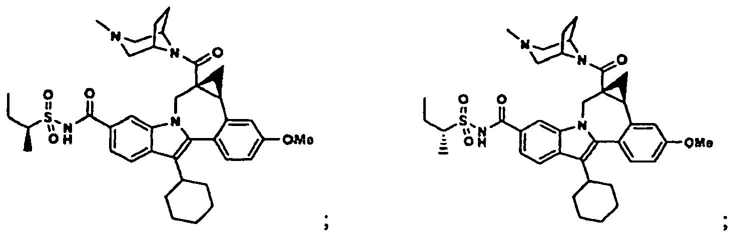 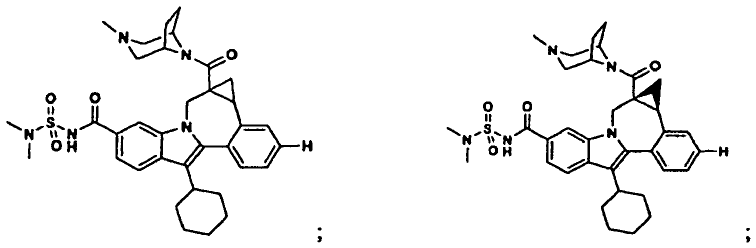 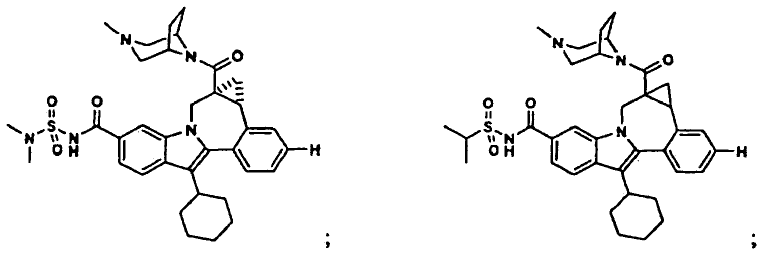 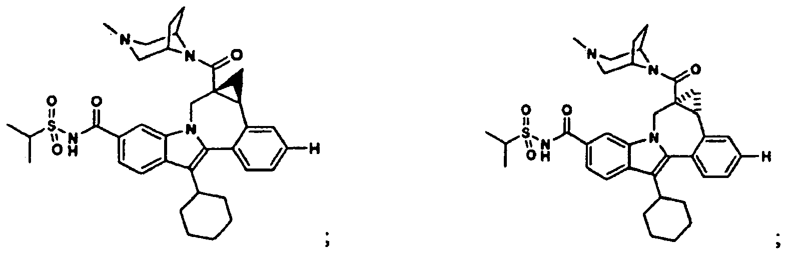 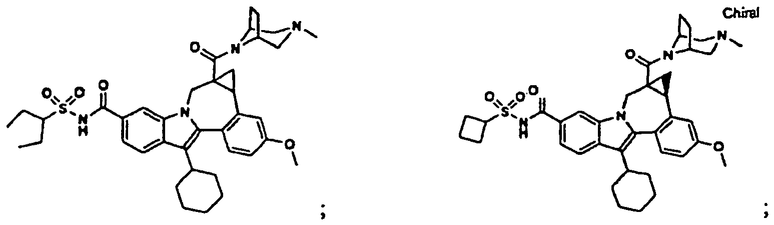 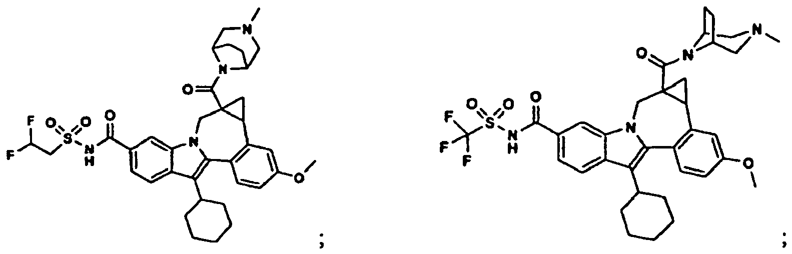 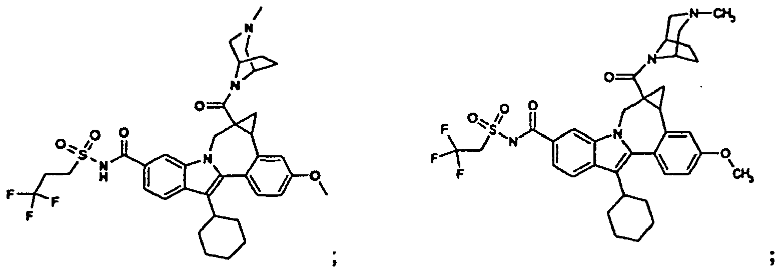 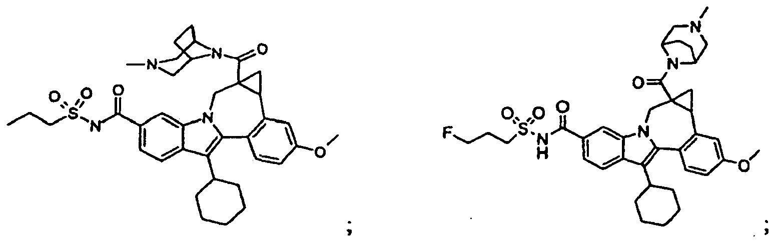 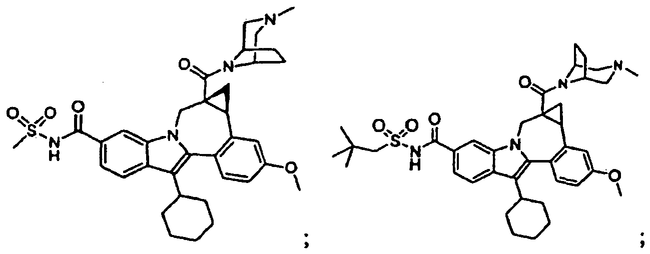 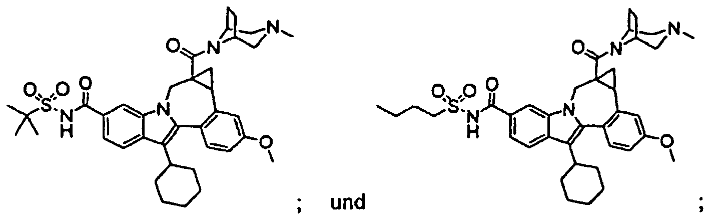 oder ein pharmazeutisch verträgliches Salz davon.

2. Verbindung nach Anspruch 1, ausgewählt aus der Gruppe, bestehend aus 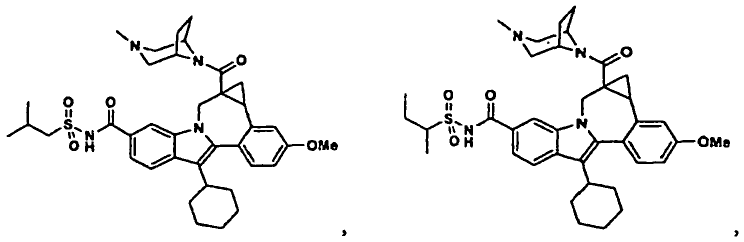 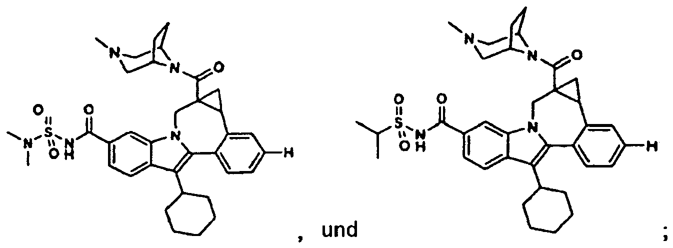 oder ein pharmazeutisch verträgliches Salz davon.

3. Verbindung nach Anspruch 1, ausgewählt aus der Gruppe, bestehend aus 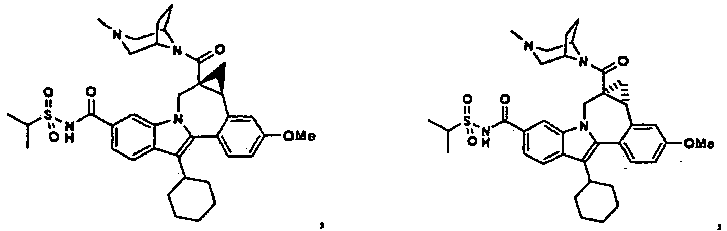 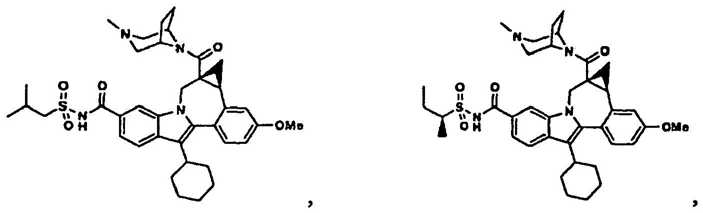 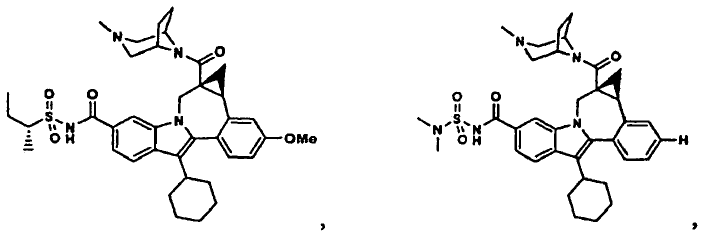 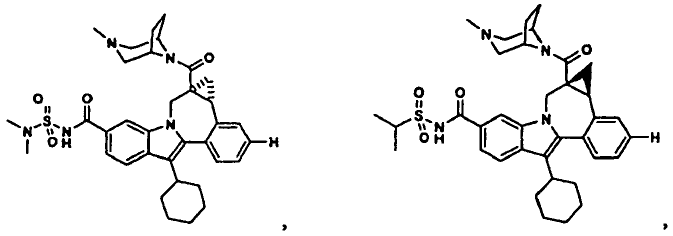 und 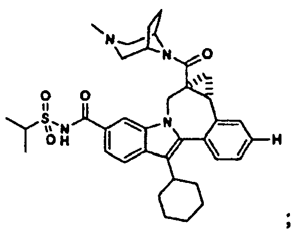 oder ein pharmazeutisch verträgliches Salz davon.

4. Zusammensetzung, umfassend eine Verbindung nach einem der Ansprüche 1 bis 3 oder ein pharmazeutisch verträgliches Salz davon und einen pharmazeutisch verträglichen Träger.

5. Zusammensetzung nach Anspruch 4, umfassend mindestens eine zusätzliche Verbindung, die therapeutischen Nutzen für HCV hat, wobei die Verbindung aus der Gruppe ausgewählt wird, bestehend aus Interferonen, Cyclosporinen, Interleukinen, HCV-Metalloprotease-Inhibitoren, HCV-Serinprotease-Inhibitoren, HCV-Polymerase-Inhibitoren, HCV-Helicase-Inhibitoren, HCV-NS4B-Protein-Inhibitoren, HCV-Entry-Inhibitoren, HCV-Assembly-Inhibitoren, HCV-Egress-Inhibitoren, HCV-NS5A-Protein-Inhibitoren, HCV-NS5B-Protein-Inhibitoren und HCV-Replikoninhibitoren.

6. Verbindung nach einem der Ansprüche 1 bis 3 zur Verwendung bei der Behandlung von Hepatitis-C-Infektion.

7. Verbindung nach Anspruch 6, weiter umfassend, mindestens eine zusätzliche Verbindung zu verabreichen, die therapeutischen Nutzen für HCV hat, wobei die Verbindung aus der Gruppe ausgewählt wird, bestehend aus Interferonen, Cyclosporinen, Interleukinen, HCV-Metalloprotease-Inhibitoren, HCV-Serinprotease-Inhibitoren, HCV-Polymerase-Inhibitoren, HCV-Helicase-Inhibitoren, HCV-NS4B-Protein-Inhibitoren, HCV-Entry-Inhibitoren, HCV-Assembly-Inhibitoren, HCV-Egress-Inhibitoren, HCV-NS5A-Protein-Inhibitoren, HCV-NS5B-Protein-Inhibitoren und HCV-Replikoninhibitoren.

## Revendications

1. Composé sélectionné parmi le groupe consistant en 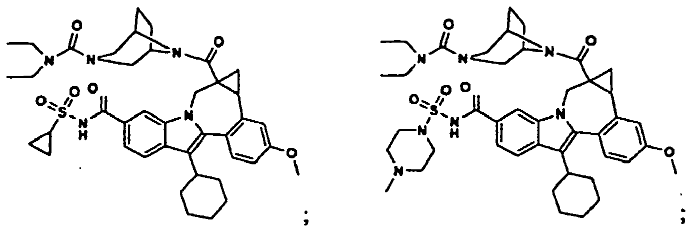 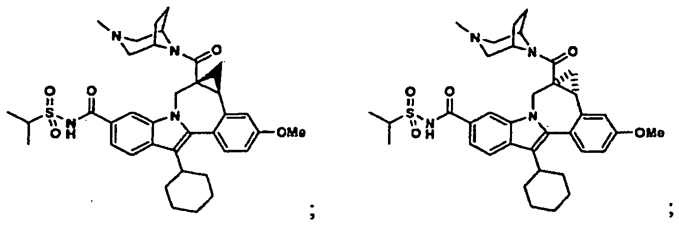 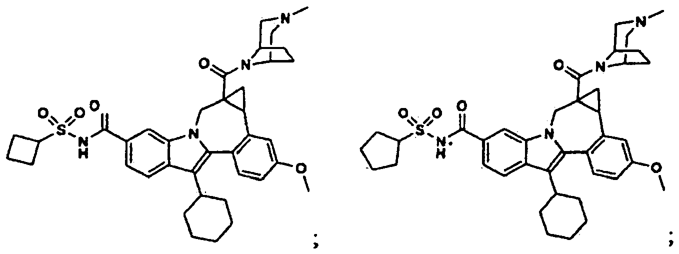 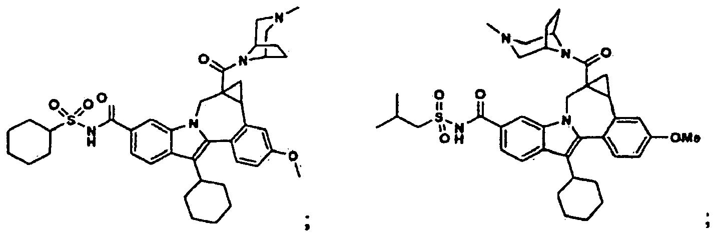 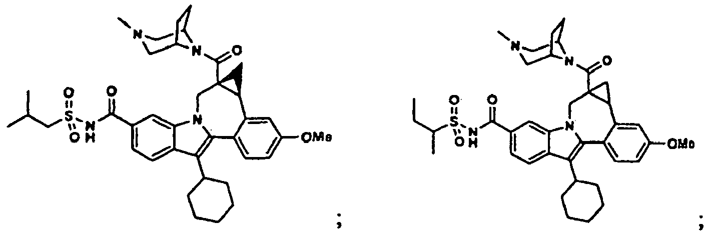 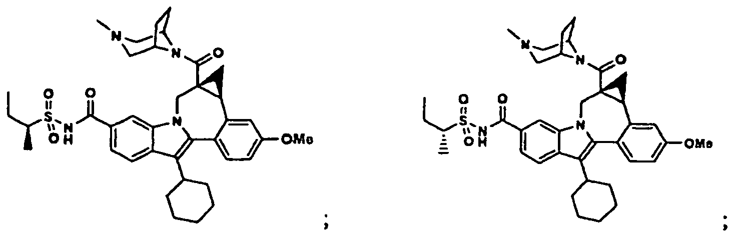 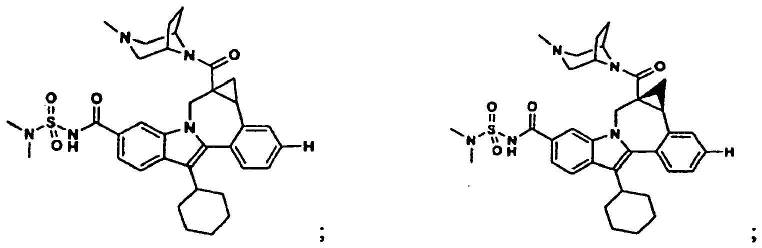 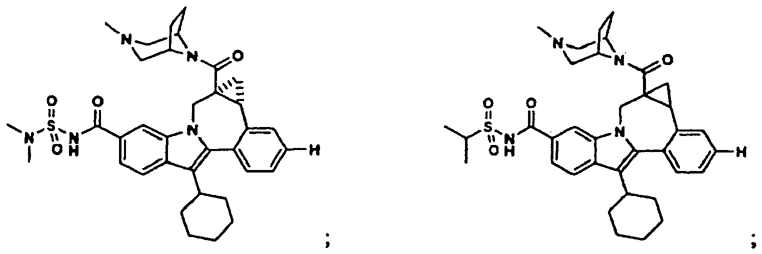 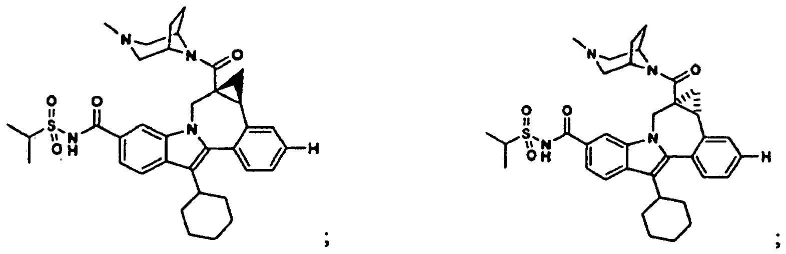 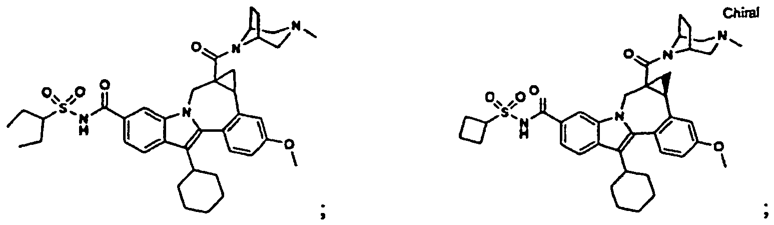 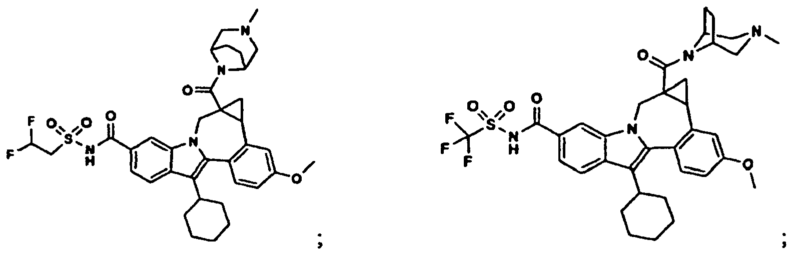 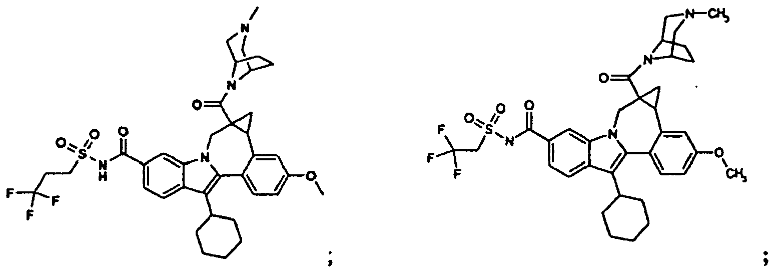 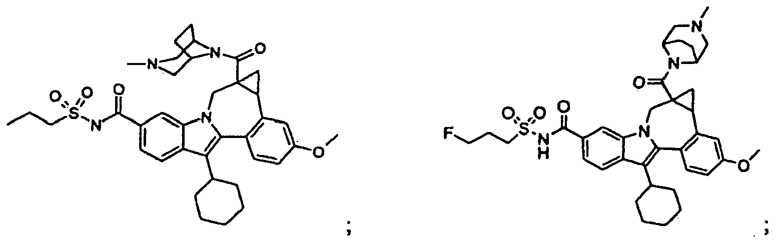 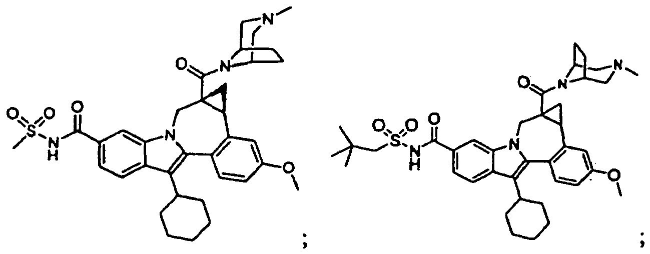 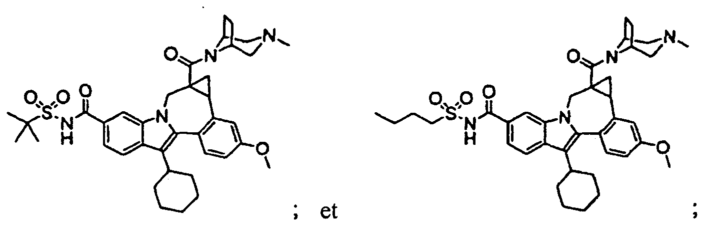 ou un sel pharmaceutiquement acceptable de celui-ci.

2. Composé selon la revendication 1, sélectionné parmi le groupe consistant en 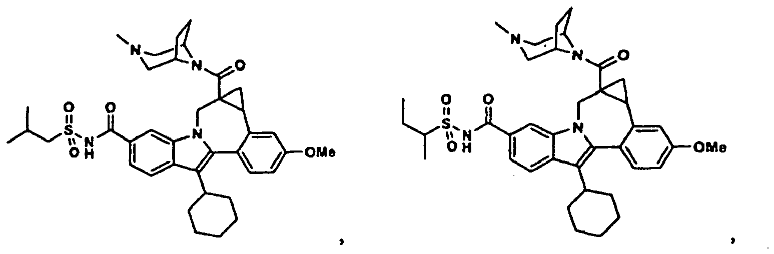 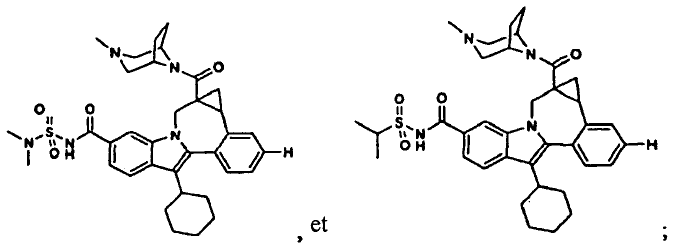 ou un sel pharmaceutiquement acceptable de celui-ci.

3. Composé selon la revendication 1, sélectionné parmi le groupe consistant en 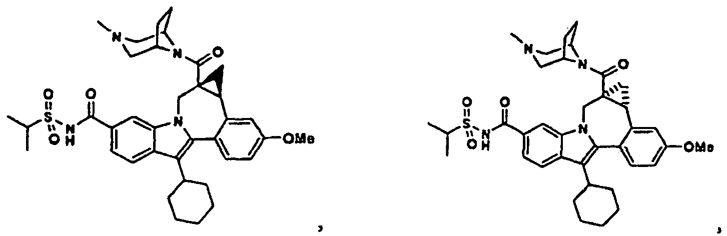 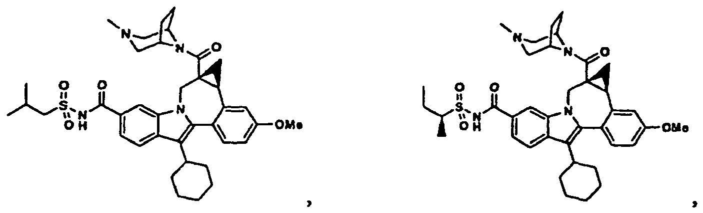 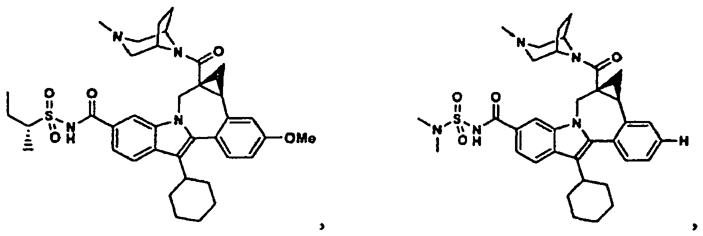 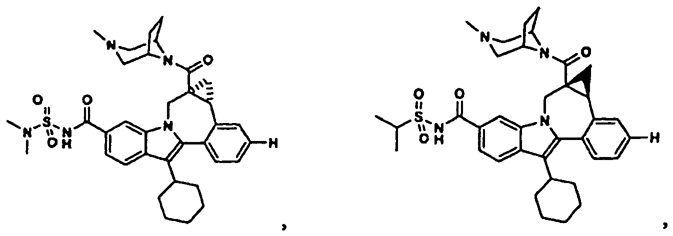 et 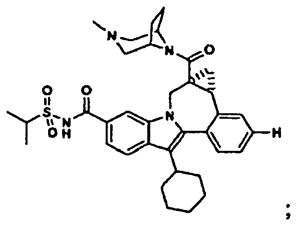 ou un sel pharmaceutiquement acceptable de celui-ci.

4. Composition comprenant un composé selon l'une quelconque des revendications 1 à 3 ou un sel pharmaceutiquement acceptable de celui-ci et un véhicule pharmaceutiquement acceptable.

5. Composition selon la revendication 4, comprenant en outre au moins un composé supplémentaire ayant des bénéfices thérapeutiques contre le VHC, où le composé est sélectionné parmi le groupe consistant en interférons, cyclosporines, interleukines, inhibiteurs des métalloprotéases du VHC, inhibiteurs de la sérine protéase du VHC, inhibiteurs de la polymérase du VHC, inhibiteurs de l'hélicase du VHC, inhibiteurs de la protéine NS4B du VHC, inhibiteurs d'entrée du VHC, inhibiteurs d'assemblage du VHC, inhibiteurs de sortie du VHC, inhibiteurs de la protéine NS5A du VHC, inhibiteurs de la protéine NS5B du VHC et inhibiteurs des réplicons du VHC.

6. Composé selon l'une quelconque des revendications 1 à 3, à utiliser dans le traitement d'une infection d'hépatite C.

7. Composé selon la revendication 6, comprenant en outre administrer au moins un composé supplémentaire ayant des bénéfices thérapeutiques contre le VHC, où le composé est sélectionné parmi le groupe consistant en interférons, cyclosporines, interleukines, inhibiteurs des métalloprotéases du VHC, inhibiteurs de la sérine protéase du VHC, inhibiteurs de la polymérase du VHC, inhibiteurs de l'hélicase du VHC, inhibiteurs de la protéine NS4B du VHC, inhibiteurs d'entrée du VHC, inhibiteurs d'assemblage du VHC, inhibiteurs de sortie du VHC, inhibiteurs de la protéine NS5A du VHC, inhibiteurs de la protéine NS5B du VHC et inhibiteurs des réplicons du VHC.
